# EUROPEAN PATENT SPECIFICATION

(11) **EP 1 708 996 B1**
(45) Date of publication and mention of the grant of the patent: **27.08.2008**
(21) Application number: 05702022.4
(22) Date of filing: 27.01.2005
(51) Int. Cl.: C07D 209/24, C07D 231/56, A61P 29/00, A61K 31/416

(54) **FUSED HETEROYRAL DERIVATIVES FOR USE AS P38 KINASE INHIBITORS**
KONDENSIERTE HETEROYRALDERIVATE ZUR VERWENDUNG ALS P38-KINASE-INHIBITOREN
DERIVES HETEROARYL FUSIONNES A UTILISER EN TANT QU'INHIBITEURS DE LA KINASE P38

(30) Priority: 30.01.2004 GB 0402143
(43) Date of publication of application: 11.10.2006
(73) Proprietor: SMITHKLINE BEECHAM CORPORATION, Philadelphia, PA 19101 (US)
(72) Inventor: BAMBOROUGH, Paul, GlaxoSmithKline, Stevenage Hertfordshire SG1 2NY (GB); CAMPOS, Sebastien Andre, GlaxoSmithKline, Stevenage Hertfordshire SG1 2NY (GB); PATEL, Vipulkumar Kantibhai, GlaxoSmithKline, Stevenage Hertfordshire SG1 2NY (GB); SWANSON, Stephen, GlaxoSmithKline, Stevenage Hertfordshire SG1 2NY (GB); WALKER, Ann Louise, GlaxoSmithKline, Stevenage Hertfordshire SG1 2NY (GB)
(74) Representative: Shore, Andrew David
(86) International application number: PCT/GB2005/000265
(87) International publication number: WO 2005/073189

(56) References cited:
- WO-A-02/16359
- WO-A-20/04010995
- COURTENY, S.M.; ET AL.: BIOORGANIC & MEDICINAL CHEMISTRY LETTERS, vol. 14, 2004, pages 3269-3273, XP002334547
- HENRY, J.R.; ET AL.: BIOORGANIC AND MEDICINAL CHEMISTRY LETTERS, vol. 8, 1998, pages 3335-3340, XP002334548

## Description

This invention relates to novel compounds and their use as pharmaceuticals, particularly as p38 kinase inhibitors, for the treatment of conditions or disease states mediated by p38 kinase activity or mediated by cytokines produced by the activity of p38 kinase.

We have now found a group of novel compounds that are inhibitors of p38 kinase.

According to the invention there is provided a compound of formula (I): 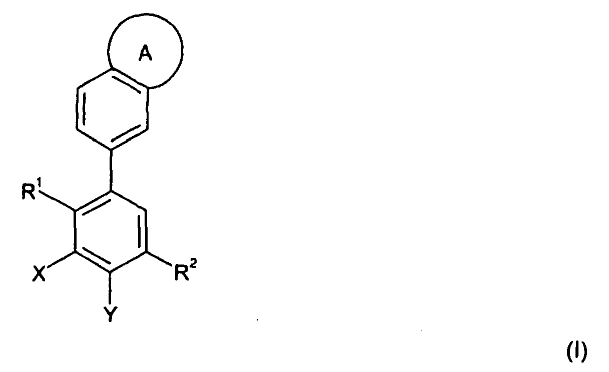 wherein
A is a fused 5-membered heteroaryl ring substituted by -(CH₂)ₘaryl or - (CH₂)ₘheteroaryl wherein the aryl or heteroaryl is optionally substituted by one or more substituents independently selected from oxo, C₁₋₆alkyl, halogen, -CN, trifluoromethyl, - OR³, -(CH₂)ₙCO₂R³, -NR³R⁴, -(CH₂)ₙCONR³R⁴, -NHCOR³, -SO₂NR³R⁴, -NHSO₂R³ and -S(O)ₚR³, and
A is optionally further substituted by one substituent selected from -OR⁵, halogen, trifluoromethyl, -CN, -CO₂R⁵ and C₁₋₆alkyl optionally substituted by hydroxy;
R¹ is selected from methyl and chloro;
R² is selected from -NH-CO-R⁶ and -CO-NH-(CH₂)_{q}-R⁷;
R3 is selected from hydrogen, -(CH₂)ᵣ-C₃₋₇cycloalkyl, -(CH₂)ᵣheterocyclyl, (CH₂)ᵣaryl, and C₁₋₆alkyl optionally substituted by up to two substituents independently selected from -OR⁸ and -NR⁸R⁹,
R⁴ is selected from hydrogen and C₁₋₆alkyl, or
R³ and R⁴, together with the nitrogen atom to which they are bound, form a 5- or 6- membered heterocyclic ring optionally containing one additional heteroatom selected from oxygen, sulfur and N-R¹⁰;
R⁵ is selected from hydrogen and C₁₋₆alkyl;
R⁶ is selected from hydrogen, C₁₋₆alkyl, -(CH₂)_{q}-C₃₋₇cycloalkyl, trifluoromethyl, - (CH₂)ₛheteroaryl optionally substituted by R¹¹ and/or R¹², and -(CH₂)ₛphenyl optionally substituted by R¹¹ and/or R¹²;
R⁷ is selected from hydrogen, C₁₋₆alkyl, C₃₋₇cycloalkyl, -CONHR¹³, phenyl optionally substituted by R¹¹ and/or R¹², and heteroaryl optionally substituted by R¹¹ and/or R¹²;
R⁸ and R⁹ are each independently selected from hydrogen and C₁₋₆alkyl;
R¹⁰ is selected from hydrogen and methyl;
R¹¹ is selected from C₁₋₆alkyl, C₁₋₆alkoxy, -(CH₂)_{q}-C₃₋₇cycloalkyl, -CONR¹³R¹⁴, - NHCOR¹⁴, halogen, -CN, -(CH₂)ₜNR¹⁵R¹⁶, trifluoromethyl, phenyl optionally substituted by one or more R¹² groups, and heteroaryl optionally substituted by one or more R¹² groups;
R¹² is selected from C₁₋₆alkyl, C₁₋₆alkoxy, halogen, trifluoromethyl, and - (CH₂)ₜNR¹⁵R¹⁶_{;}
R¹³ and R¹⁴ are each independently selected from hydrogen and C₁₋₆alkyl, or
R¹³ and R¹⁴, together with the nitrogen atom to which they are bound, form a 5- or 6-membered heterocyclic ring optionally containing one additional heteroatom selected from oxygen, sulfur and N-R¹⁰, wherein the ring may be substituted by up to two C₁₋₆alkyl groups;
R¹⁵ is selected from hydrogen, C₁₋₆alkyl and -(CH₂)_{q}-C₃₋₇cycloalkyl optionally substituted by C₁₋₆alkyl,
R¹⁶ is selected from hydrogen and C₁₋₆alkyl, or
R¹⁵ and R¹⁶, together with the nitrogen atom to which they are bound, form a 5- or 6-membered heterocyclic ring optionally containing one additional heteroatom selected from oxygen, sulfur and N-R¹⁰;
X and Y are each independently selected from hydrogen, methyl and halogen;
m, n, p and q are each independently selected from 0, 1 and 2;
r and s are each independently selected from 0 and 1; and
t is selected from 0, 1, 2 and 3;
with the proviso that when A is substituted by -(CH₂)ₘheteroaryl and m is 0, the - (CH₂)ₘheteroaryl group is not a 5-membered heteroaryl ring optionally substituted by C₁₋₂alkyl;
or a pharmaceutically acceptable derivative thereof.

In one embodiment, A includes fused 5-membered heteroaryl rings containing up to two heteroatoms independently selected from oxygen, nitrogen and sulfur. In another embodiment, A includes fused 5-membered heteroaryl rings containing up to two heteroatoms independently selected from oxygen and nitrogen. In a further embodiment, A includes 5-membered heteroaryl rings containing two heteroatoms independently selected from oxygen and nitrogen, for example rings containing a nitrogen atom and one additional heteroatom selected from oxygen and nitrogen. Examples of suitable A groups include fused isoxazolyl, pyrazolyl, pyrrolyl and thiazolyl rings such as those shown below:

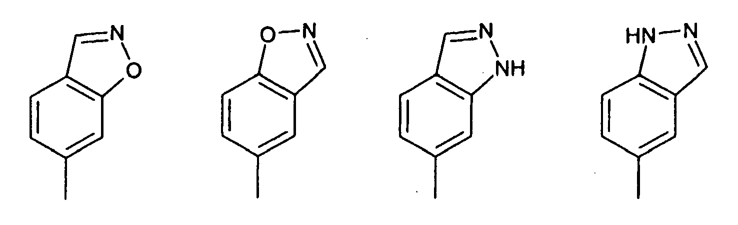

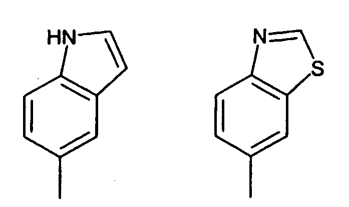

Representative examples of A groups include fused isoxazolyl, pyrazolyl and pyrrolyl rings such as those shown below:

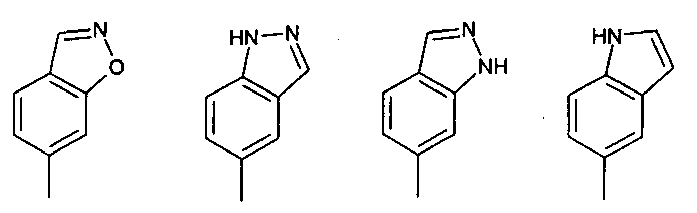

For example, A may be a fused pyrazolyl ring such as those shown below:

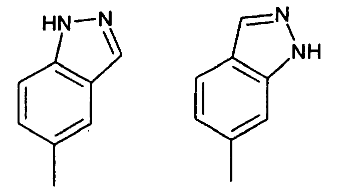

A representative example of a compound of formula (I) is wherein ring A is substituted by -(CH₂)ₘaryl or -(CH₂)ₘheteroaryl, located on any position on the ring.

For example, compounds of formula (I) include compounds wherein ring A is substituted by -(CH₂)ₘaryl or -(CH₂)ₘheteroaryl located in position (i), (ii), (iii) or (iv), for example position (ii) or (iii), as shown below:

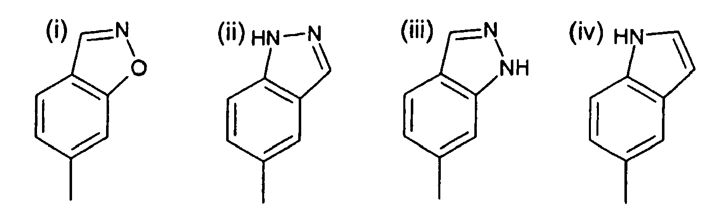

In one embodiment, the -(CH₂)ₘaryl group is -(CH₂)ₘphenyl.

In one embodiment, the -(CH₂)ₘheteroaryl group is a group wherein the heteroaryl is a 5- or 6-membered heteroaryl ring containing up to two heteroatoms independently selected from oxygen, nitrogen and sulfur. In a further embodiment, the -(CH₂)ₘheteroaryl group include groups wherein the heteroaryl is a 5- or 6-membered heteroaryl ring containing up to two heteroatoms independently selected from oxygen and nitrogen, for example pyridyl, isoxazolyl, pyrazolyl, imidazolyl, pyrimidinyl or pyrazinyl. Representative examples of the - (CH₂)ₘheteroaryl group include groups wherein the heteroaryl is a 5- or 6-membered heteroaryl ring containing up to two heteroatoms independently selected from oxygen and nitrogen, for example pyridyl, isoxazolyl or pyrimidinyl. Further representative examples of the -(CH₂)ₘheteroaryl group include groups wherein the heteroaryl is a 5- or 6-membered heteroaryl ring containing up to two heteroatoms independently selected from oxygen and nitrogen, for example pyrazolyl, imidazolyl or pyrazinyl.

The -(CH₂)ₘaryl and -(CH₂)ₘheteroaryl groups are optionally substituted and the substituents may be located on any position on the aryl or heteroaryl.

In one embodiment, the aryl or heteroaryl is optionally substituted by one or two substituents independently selected from oxo, C₁₋₆alkyl, halogen, -CN, trifluoromethyl, - OR³, -(CH₂)ₙCO₂R³, -NR³R⁴, -(CH₂)ₙCONR³R⁴, -NHCOR³, -SO₂NR³R⁴, -NHSO₂R³ and -S(O)ₚR³. In another embodiment, the aryl is optionally substituted by one or two substituents independently selected f rom C₁₋₆alkyl, halogen, - CN, trifluoromethyl, -OR³, - NR³R⁴, -(CH₂)ₙCONR³R⁴ and -S(O)ₚR³. Representative examples of substituents for the aryl include one or two substituents independently selected from C₁₋₆alkyl, in particular methyl, halogen, -CN, trifluoromethyl, -OR³, -NR³R⁴, -(CH₂)ₙCONR³R⁴ and -S(O)ₚR³. For example, aryl is optionally substituted by halogen, in particular fluorine, -OR³, in particular methoxy, or -(CH₂)ₙCONR³R⁴. In a further embodiment, the heteroaryl is optionally substituted by one or two substituents independently selected from oxo, C₁₋₆alkyl, halogen, -OR³, -NR³R⁴ and -(CH₂)ₙCONR³R⁴. Representative examples of substituents for the heteroaryl include one or two substituents independently selected from oxo and C₁₋₆alkyl, in particular methyl. Further representative examples of substituents for the heteroaryl include one or two substituents independently selected from halogen, -OR³, - NR³R⁴ and -(CH₂)ₙCONR³R⁴.

A representative example of R¹ is methyl.

A representative example of R² is -CO-NH-(CH₂)_{q}-R⁷.

Representative examples of R³ include hydrogen; -(CH₂)ᵣ-C₃₋₇cycloalkyl, in particular -(CH₂)ᵣ-cyclohexyl; -(CH₂)ᵣheterocyclyl, in particular wherein the heterocyclyl is a 5- or 6-membered heterocyclyl containing one heteroatom selected from oxygen, nitrogen and sulfur such a tetrahydrofuran or tetrahydropyran; and C₁₋₆alkyl, in particular C₁₋₄alkyl such as methyl, ethyl, or n-propyl, optionally substituted by up to two substituents independently selected from -OR⁸ and -NR⁸R⁹.

A representative example of R⁴ is hydrogen.

Alternatively, R³ and R⁴, together with the nitrogen atom to which they are bound, form a 5- or 6-membered heterocyclic ring optionally containing one additional heteroatom selected from oxygen, sulfur and N-R¹⁰, in particular morpholinyl.

In one embodiment, R⁵ is selected from hydrogen and C₁₋₄alkyl.

In one embodiment, R⁶ is a -(CH₂)ₛheteroaryl optionally substituted by R¹¹ and/or R¹²_{.}

Representative examples of R⁷ include hydrogen; C₁₋₆alkyl, in particular C₁₋₄alkyl such as methyl, ethyl, n-propyl, isopropyl and n-butyl; C₃₋₇cycloalkyl, in particular C₃₋₆cycloalkyl such as cyclopropyl, cyclobutyl and cyclopentyl; phenyl optionally substituted by R¹¹ and/or R¹²; and heteroaryl, in particular a 5- or 6-membered heteroaryl containing two heteroatoms selected from nitrogen and oxygen, for example pyrazolyl, pyridazinyl and pyrimidinyl, optionally substituted by R¹¹ and/or R¹².

Representative examples of R⁸ and R⁹ include hydrogen and C₁₋₄alkyl, in particular hydrogen and methyl.

In one embodiment, R¹¹ is selected from C₁₋₆alkyl, in particular C₁₋₄alkyl such as methyl or ethyl, or halogen, in particular fluorine.

In one embodiment, R¹² is selected from C₁₋₆alkyl, in particular C₁₋₄alkyl such as methyl or ethyl, or halogen, in particular fluorine.

A representative example of R¹¹ and R¹² is halogen, in particular fluorine. A further representative example of R¹¹ and R¹² is C₁₋₄alkyl such as methyl or ethyl.

In one embodiment, R¹³ and R¹⁴ are each independently hydrogen or C₁₋₄alkyl.

In one embodiment, R¹⁵ and R¹⁶, together with the nitrogen atom to which they are bound, form a 5- or 6-membered heterocyclic ring optionally further containing one additional oxygen atom.

In one embodiment, X and Y are each independently selected from hydrogen, chlorine and fluorine. Representative examples of X include hydrogen and fluorine. A representative example of Y is hydrogen.

In one embodiment, when A is a fused 5-membered heteroaryl ring substituted by - (CH₂)ₘaryl and m is 0, 1 or 2, or A is a fused 5-membered heteroaryl ring substituted by - (CH₂)ₘheteroaryl and m is 1 or 2, the aryl or heteroaryl is optionally substituted by one or more substituents independently selected from oxo, C₁₋₆alkyl, halogen, -CN, trifluoromethyl, -OR³, -(CH₂)ₙCO₂R³, -NR³R⁴, -(CH₂)ₙCONR³R⁴, -NHCOR³, -SO₂NR³R⁴, -NHSO₂R³ and -S(O)ₚR³, and, when A is a fused 5-membered heteroaryl ring substituted by - (CH₂)ₘheteroaryl and m is 0, the heteroaryl is a 5-membered heteroaryl ring substituted by one or more substituents independently selected from oxo, C₃₋₆alkyl, halogen, -CN, trifluoromethyl, -OR³, -(CH₂)ₙCO₂R³, -NR³R⁴, -(CH₂)ₙCONR³R⁴, -NHCOR³, - SO₂NR³R⁴, -NHSO₂R³ and -S(O)pR³, the heteroaryl is a 5-membered heteroaryl ring substituted by C₁₋₂alkyl and one or more substituents independently selected from oxo, C₁₋₆alkyl, halogen, -CN, trifluoromethyl, -OR³, -(CH₂)ₙCO₂R³, -NR³R⁴, -(CH₂)ₙCONR³R⁴, - NHCOR³, -SO₂NR³R⁴, -NHSO₂R³ and -S(O)ₚR³, or the heteroaryl is a 6-membered heteroaryl ring optionally substituted by one or more substituents independently selected from oxo, C₁₋₆alkyl, halogen, -CN, trifluoromethyl, -OR³, -(CH₂)ₙCO₂R³, -NR³R⁴, - (CH₂)ₙCONR³R⁴, -NHCOR³, -SO₂NR³R⁴, -NHSO₂R³ and -S(O)ₚR³.

Representative examples of m include 0 and 1.

In one embodiment, n is selected from 0 and 1. A representative example of n is 1. A further representative example of n is 0.

A representative example of p is 2.

Representative examples of q include 0 and 1.

Representative examples of r include 0 and 1.

In one embodiment, s is 0.

In one embodiment, t is 0.

It is to be understood that the present invention covers all combinations of the embodiments and the particular and preferred groups described hereinabove. It is also to be understood that the present invention encompasses compounds of formula (I) in which a particular group or parameter, for example R³, R⁴, R⁸, R⁹, R¹⁰, R¹², R¹⁵, R¹⁶, n, p, q, r or t, may occur more than once. In such compounds it will be appreciated that each group or parameter is independently selected from the values listed.

Particular compounds according to the invention include those mentioned in the Examples. Specific examples which may be mentioned include:
*N*-cyclopropyl-3-fluoro-4-methyl-5-(1-phenyl-1*H*-indazol-5-yl)benzamide;
*N*-cyclopropyl-3-fluoro-5-[1-(4-fluorophenyl)-1*H*-indazol-5-yl]-4-methylbenzamide;
*N*-cyclopropyl-3-fluoro-5-[1-(4-fluoro-2-methylphenyl)-1*H*-indazol-5-yl]-4-methylbenzamide;
*N*-cyclopropyl-3-fluoro-4-methyl-5-{1-[4-(4-morpholinyl)phenyl]-1*H*-indazol-5-yl}benzamide;
*N*-ethyl-3-fluoro-4-methyl-5-(1-phenyl-1*H*-indazol-5-yl)benzamide;
*N*-(cyclopropylmethyl)-3-fluoro-4-methyl-5-(1-phenyl-1*H*-indazol-5-yl)benzamide;
*N*-cyclopropyl-3-fluoro-4-methyl-5-{1-[4-(methylsulfonyl)phenyl]-1*H*-indazol-5-yl}benzamide;
*N*-cyclopropyl-3-fluoro-4-methyl-5-(1-{4-[2-(methylamino)-2-oxoethyl]phenyl}-1*H*-indazol-5-yl)benzamide;
*N*-cyclopropyl-3-[1-(4-{[2-(dimethylamino)ethyl]amino}phenyl)-1*H*-indazol-5-yl]-5-fluoro-4-methylbenzamide;
*N*-cyclopropyl-3-fluoro-4-methyl-5-{1-[4-(tetrahydro-2*H*-pyran-4-ylamino)phenyl]-1*H*-indazol-5-yl}benzamide;
*N*-cyclopropyl-3-fluoro-4-methyl-5-(1-{4-[(tetrahydro-2-furanylmethyl)amino]phenyl}-1*H-*indazol-5-yl)benzamide;
*N*-cyclopropyl-3-(1-{4-[(2,3-dihydroxypropyl)amino]phenyl}-1*H*-indazol-5-yl)-5-fluoro-4-methylbenzamide;
*N*-cyclopropyl-3-fluoro-4-methyl-5-{3-[4-(methyloxy)phenyl]-1,2-benzisoxazol-6-yl}benzamide;
*N*-cyclopropyl-3-fluoro-5-[3-(4-hydroxyphenyl)-1,2-benzisoxazol-6-yl]-4-methylbenzamide;
*N*-cyclopropyl-3-fluoro-4-methyl-5-{1-[(1-oxido-2-pyridinyl)methyl]-1*H*-indazol-5-yl}benzamide;
*N*-ethyl-3-[3-(4-fluorophenyl)-1*H*-indazol-6-yl]-4-methylbenzamide;
*N*-cyclopropyl-3-[3-(4-fluorophenyl)-1*H*-indazol-6-yl]-4-methylbenzamide;
*N*-ethyl-4-methyl-3-{3-[4-(methyloxy)phenyl]-1*H*-indazol-6-yl}benzamide; and
*N*-cyclopropyl-4-methyl-3-{3-[4-(methyloxy)phenyl]-1*H*-indazol-6-yl}benzamide;
and pharmaceutically acceptable derivatives thereof.

Further specific examples which may be mentioned include:
N-(1-ethyl-1H-pyrazol-5-yl)-3-fluoro-5-[3-(4-fluorophenyl)-1H-indazol-6-yl]-4-methylbenzamide;
3-fluoro-5-[3-(4-fluorophenyl)-1H-indazol-6-yl]-4-methyl-N-(1-methyl-1H-pyrazol-5-yl)benzamide;
N-ethyl-3-fluoro-5-{3-[4-fluoro-2-(methyloxy)phenyl]-1H-indazol-6-yl}-4-methylbenzamide;
*N*-(1,4-dimethyl-1*H*-pyrazol-5-yl)-3-fluoro-5-[3-(4-fluorophenyl)-1*H*-indazol-6-yl]-4-methylbenzamide; and
*N*-(1,4-dimethyl-1*H*-pyrazol-5-yl)-3-[3-(4-fluorophenyl)-1*H*-indazol-6-yl]-4-methylbenzamide;
and pharmaceutically acceptable derivatives thereof.

As used herein, the term "pharmaceutically acceptable" means a compound which is suitable for pharmaceutical use. Salts and solvates of compounds of the invention which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

As used herein, the term "pharmaceutically acceptable derivative", means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters, in particular salts.

The compounds of the present invention may be in the form of and/or may be administered as a pharmaceutically acceptable salt. For a review on suitable salts see Berge et al., J. Pharm. Sci., 1977, 66, 1-19.

Typically, a pharmaceutical acceptable salt may be readily prepared by using a desired acid or base as appropriate. The salt may precipitate from solution and be collected by filtration or may be recovered by evaporation of the solvent.

Salts of the compounds of the present invention may, for example, comprise acid addition salts resulting from reaction of an acid with a nitrogen atom present in a compound of formula (I). Salts encompassed within the term "pharmaceutically acceptable salts" refer to non-toxic s alts of the compounds of this invention. Suitable addition s alts a re formed from acids which form non-toxic salts and examples are acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, ethanesulfonate, formate, fumarate, gluceptate, gluconate, glutamate, glycollylarsanilate, hexylresorcinate, hydrabamine, hydrobromide, hydrochloride, hydrogen phosphate, hydroiodide, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, oxaloacetate, pamoate (embonate), palmitate, pantothenate, phosphate/diphosphate, piruvate, polygalacturonate, saccharate, salicylate, stearate, subacetate, succinate, sulfate, tannate, tartrate, teoclate, tosylate, triethiodide, trifluoroacetate and valerate. Representative salts include formate salts such as the mono- and di-formate salts.

Pharmaceutically acceptable base salts include ammonium salts such as a trimethylammonium salt, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Those skilled in the art of organic chemistry will appreciate that many organic compounds can form complexes with solvents in which they are reacted or from which they are precipitated or crystallized. These complexes are known as "solvates". As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water. A complex with water is known as a "hydrate". Solvates of the compounds of the invention are within the scope of the invention.

As used herein, the term "prodrug" means a compound which is converted within the body, e.g. by hydrolysis in the blood, into its active form that has medical effects. Pharmaceutically acceptable prodrugs are described in T. Higuchi and V. Stella, Prodrugs as Novel Delivery Systems, Vol. 14 of the A.C.S. Symposium Series; Edward B. Roche, ed., Bioreversible Carriers in Drug Design, American Pharmaceutical Association and Pergamon Press, 1987; and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Prodrugs are any covalently bonded carriers that release a compound of formula (I) *in vivo* w hen such prodrug is administered to a patient. Prodrugs a re generally prepared by modifying functional groups in a way such that the modification is cleaved, either by routine manipulation or *in vivo*, yielding the parent compound. Prodrugs include, for example, compounds of this invention wherein hydroxy or amine groups are bonded to any group that, when administered to a patient, cleaves to form the hydroxy or amine groups. Thus, representative examples of prodrugs include (but are not limited to) acetate, formate and benzoate derivatives of alcohol and amine functional groups of the compounds of formula (I). Further, in the case of a carboxylic acid (-COOH), esters may be employed, such as methyl esters, ethyl esters, and the like. Esters may be active in their own right and /or be hydrolysable under *in vivo* conditions in the human body. Suitable pharmaceutically acceptable *in vivo* hydrolysable ester groups include those which break down readily in the human body to leave the parent acid or its salt.

As used herein, the term " alkyl" refers to straight or branched hydrocarbon chains containing the specified number of carbon atoms. For example, C₁₋₆alkyl means a straight or branched alkyl containing at least 1, and at most 6, carbon atoms. Examples of "alkyl" as used herein include, but are not limited to, methyl, ethyl, n-propyl, n-butyl, n-pentyl, isobutyl, isopropyl and t-butyl. A C₁₋₄alkyl group is preferred, for example methyl, ethyl, isopropyl or t-butyl. The said alkyl groups may be optionally substituted with one or more fluorine atoms for example, trifluoromethyl.

As used herein, the term "alkoxy" refers to a straight or branched chain alkoxy groups containing the specified number of carbon atoms. For example, C₁₋₆alkoxy means a straight or branched alkoxy containing at least 1, and at most 6, carbon atoms. Examples of "alkoxy" as used herein include, but are not limited to methoxy, ethoxy, propoxy, prop-2-oxy, butoxy, but-2-oxy, 2-methylprop-1-oxy, 2-methylprop-2-oxy, pentoxy, or hexyloxy. A C₁₋₄alkoxy group is preferred, for example methoxy or ethoxy.

As used herein, the term "cycloalkyl" refers to a non-aromatic hydrocarbon ring containing the specified number of carbon atoms which may optionally contain up to one double bond. For example, C₃₋₇cycloalkyl means a non-aromatic ring containing at least three, and at most seven, ring carbon atoms. Examples of "cycloalkyl" as used herein include, but are not limited to, cyclopropyl, cyclobutyl, cyclopentyl, cyclohexyl and cycloheptyl. A C ₃₋₆cycloalkyl g roup i s p referred, for example, cyclopropyl, cyclopentyl or cyclohexyl.

As used herein, the term "aryl" refers to an aromatic carbocyclic ring such as phenyl, biphenyl or naphthyl. Preferably the aryl is phenyl.

As used herein, the terms "heteroaryl ring" and "heteroaryl", unless otherwise defined, refer to a monocyclic 5- to 7-membered unsaturated hydrocarbon ring containing at least one heteroatom independently selected from oxygen, nitrogen and sulfur. Preferably, the heteroaryl ring has five or six ring atoms. Examples of heteroaryl rings include, but are not limited to, furyl, thienyl, pyrrolyl, oxazolyl, thiazolyl, isoxazolyl, isothiazolyl, imidazolyl, pyrazolyl, oxadiazolyl, triazolyl, tetrazolyl, thiadiazolyl, pyridyl, pyridazinyl, pyrimidinyl, pyrazinyl and triazinyl. The said ring may be optionally substituted by one or more substituents independently selected from C₁₋₆alkyl and oxy.

As used herein, the terms "heterocyclic ring" or "heterocyclyl", unless otherwise defined refer to a monocyclic 3 - to 7 -membered saturated hydrocarbon ring containing at least one heteroatom independently selected from oxygen, nitrogen and sulfur. Preferably, the heterocyclyl ring has five or six ring atoms. Examples of heterocyclyl groups include, but are not limited to, pyrrolidinyl, imidazolidinyl, pyrazolidinyl, piperidyl, piperazinyl, morpholino, tetrahydropyranyl, tetrahydrofuranyl, and thiomorpholino. The said ring may be optionally substituted by one or more substituents independently selected from C₁₋₆alkyl and oxy.

As used herein, the terms "halogen" or "halo" refer to the elements fluorine, chlorine, bromine and iodine. Preferred halogens are fluorine, chlorine and bromine. A particularly preferred halogen is fluorine or chlorine.

As used herein, the terms "halogen" or "halo" refer to the elements fluorine, chlorine, bromine and iodine. Preferred halogens are fluorine, chlorine and bromine. A particularly preferred halogen is fluorine or chlorine.

As used herein, the term "optionally" means that the subsequently described event(s) may or may not occur, and includes both event(s) which occur and events that do not occur.

As used herein, the term "substituted" refers to substitution with the named substituent or substituents, multiple degrees of substitution being allowed unless otherwise stated.

Certain compounds of formula (I) may exist in stereoisomeric forms (e.g. they may contain one or m ore asymmetric carbon atoms or may exhibit cis-trans isomerism). The individual stereoisomers (enantiomers and diastereomers) and mixtures of these are included within the scope of the present invention. The present invention also covers the individual isomers of the compounds represented by formula (I) as mixtures with isomers thereof in which one or more chiral centres are inverted. Likewise, it is understood that compounds of formula (I) may exist in tautomeric forms other than that shown in the formula and these are also included within the scope of the present invention.

Separation of diastereoisomers or cis and trans isomers may be achieved by conventional techniques, e.g. by fractional crystallisation, chromatography or H.P.L.C. A stereoisomeric mixture of the agent may also be prepared from a corresponding optically pure intermediate or by resolution, such as H.P.L.C. of the corresponding racemate using a suitable chiral support or by fractional crystallisation of the diastereoisomeric salts formed by reaction of the corresponding racemate with a suitable optically active acid or base, as appropriate.

Furthermore, some of the crystalline forms of the compounds of structure (I) may exist as polymorphs, which are included in the present invention.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the working Examples.

A compound of formula (I) may be prepared by reacting a compound of formula (II) 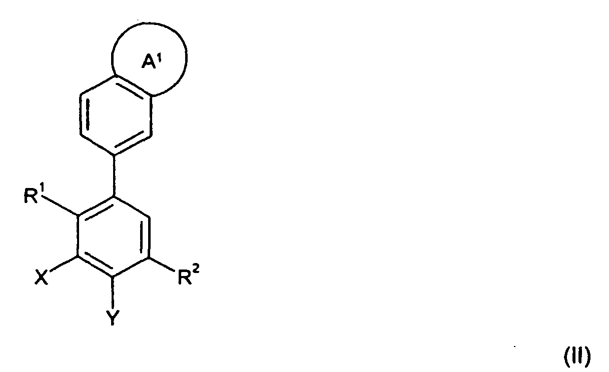 in which R¹, R², X and Y are as hereinbefore defined and A¹ is an unsubstituted fused 5- membered heteroaryl ring, with a suitable reagent, for example a halide derivative of formula (IIIA) or (IIIB)

Z-(CH₂)ₘaryl (IIIA)

Z-(CH₂)ₘheteroaryl (IIIB)

in which -(CH₂)ₘaryl and -(CH₂)ₘheteroaryl are as hereinbefore defined and Z is halogen, in particular bromine,
in, for example, the presence of a base such as sodium hydride and a solvent such as DMF.

Alternatively, when A is substituted by -(CH₂)ₘaryl wherein m is 0, the compound of formula (II) may be reacted with a boronic acid compound of formula (IV)

(HO)₂B-(CH₂)ₘaryl (IV)

in which -(CH₂)ₘaryl is as hereinbefore defined,
in the presence of copper (I) acetate and pyridine.

A compound of formula (I) or a compound of formula (II) may be prepared by reacting a compound of formula (V) 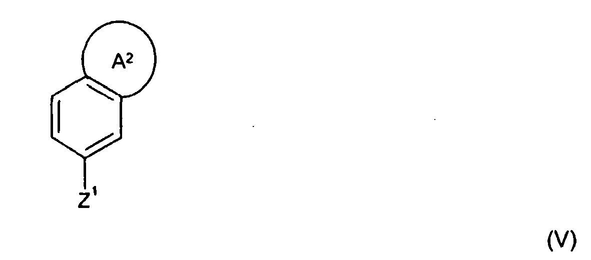 in which A² is A as hereinbefore defined, in which case the resulting product is a compound of formula (I), A² is A¹ as hereinbefore defined, in which case the resulting product is a compound of formula (II), or A² is a protected form of A or A¹, and Z¹ is halogen, in particular bromine,
with a compound of formula (VIA) or (VIB) 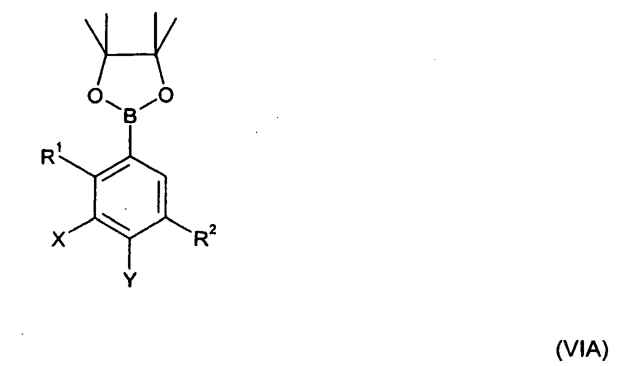 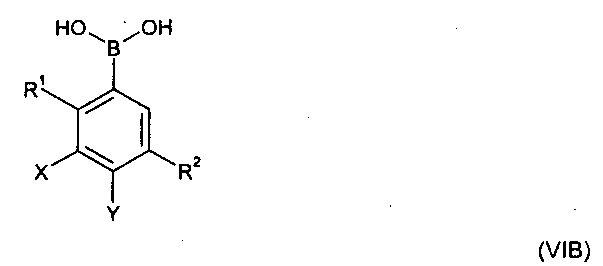 in which R¹, R², X and Y are as hereinbefore defined,
in the presence of a catalyst, for example tetrakis(triphenylphosphine)palladium,
and, if necessary, removing any protecting groups.

A compound of formula (V) wherein A² is A and A is a fused pyrazolyl ring may, for example, be prepared by reacting a compound of formula (VII) 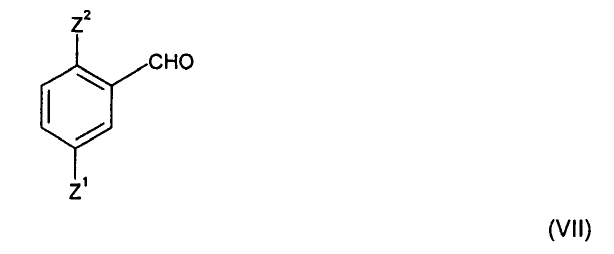 in which Z¹ is as hereinbefore defined and Z² is halogen, in particular fluorine,
with a hydrazine derivative of formula (VIIIA) or (VIIIB)

H₂NNH-(CH₂)ₘaryl (VIIIA)

H₂NNH-(CH₂)ₘheteroaryl (VIIIB)

in which -(CH₂)ₘaryl and -(CH₂)ₘheteroaryl are as hereinbefore defined,
followed by cyclisation in the presence of a base such as DBU.

Alternatively, a compound of formula (V) wherein A² is A and A is a fused pyrazolyl ring may, for example, be prepared by reacting a compound of formula (IX) 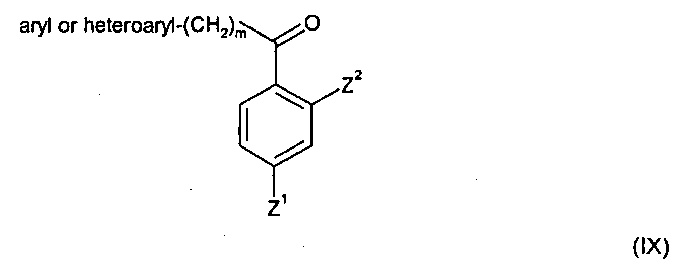 in which Z¹, Z², -(CH₂)ₘaryl and -(CH₂)ₘheteroaryl are as hereinbefore defined,
with a protected hydrazine derivative of formula (X)

H₂NNH-P (X)

in which P is a protecting group such as Boc,
followed by cyclisation in the presence of a base such as DBU.

A compound of formula (V) wherein A² is A and A is a fused isoxazolyl ring may, for example, be prepared by reacting a compound of formula (IX) as hereinbefore defined with hydroxylamine, followed by cyclisation in the presence of a base such as DBU.

A compound of formula (VIA) may be prepared by, for example, reacting a compound of formula (XI) 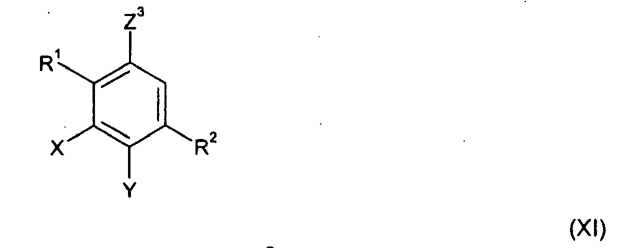 in which R¹, R², X and Y are as hereinbefore defined and Z³ is halogen, in particular iodine, with bis(pinnacolato)diboron, [1,1'-bis(diphenylphosphino)ferrocene] dichloropalladium (II) complex (PdCl₂(ppdf)) and potassium acetate in a solvent such as DMF.

A compound of formula (VIB) may be prepared by, for example, reacting a compound of formula (XI) as hereinbefore defined, with n-butyl lithium and triisopropyl borate in a solvent such as THF.

When R² is -NH-CO-R⁶, a compound of formula (XI) may be prepared by reacting an amine of formula (XII) 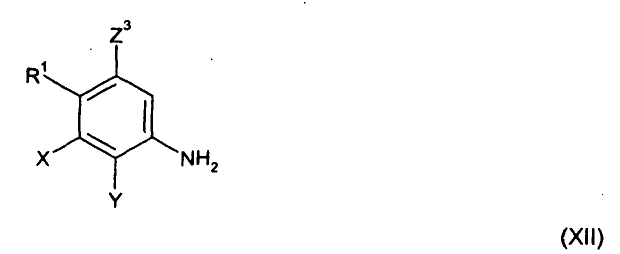 in which R¹, X, Y and Z³ are as hereinbefore defined,
with an acid compound of formula (XIII)

R⁶CO₂H (XIII)

in which R⁶ is as hereinbefore defined,
under amide forming conditions.

Suitable amide forming conditions are well known in the art and include adding a base such as DIPEA to a mixture of the amine of formula (XII), the acid of formula (XIII), and HATU in a solvent such as DMF.

Alternatively, when R² is -CO-NH-(CH₂)_{q}-R⁷, a compound of formula (XI) may readily be prepared from a corresponding acid compound of formula (XIV) 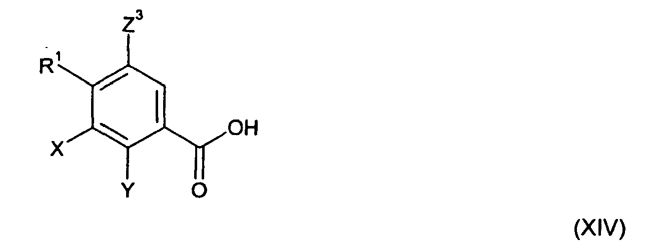 in which R¹, X, Y and Z³ are as hereinbefore defined,
by converting the acid to an activated form of the acid, for example the acid chloride, by treatment with, for example, thionyl chloride, and then reacting the activated acid thus formed with an amine compound of formula (XV)

R⁷-(CH₂)_{q}-NH₂ (XV)

in which R⁷ is as hereinbefore defined,
under amide forming conditions.

Suitable amide forming conditions are well known in the art and include treating a solution of the acid of formula (XIV), or the activated form thereof, in for example DMF, with an amine of formula (XV) in the presence of a base such as triethylamine.

Alternatively, when R² is -CO-NH-(CH₂)_{q}-R⁷, a compound of formula (I) may be prepared from a corresponding acid compound of formula (XVI) 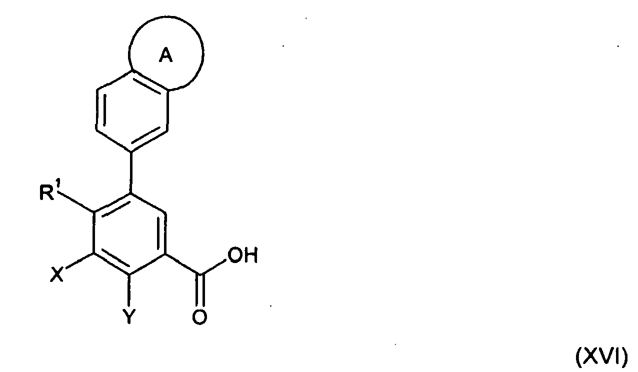 in which A, R¹, X and Y are as hereinbefore defined,
by reacting the acid with an amine compound of formula (XV) as hereinbefore defined under the conditions described above.

When A is a fused pyrazolyl, another general method for preparing compounds of formula (I) comprises reacting a compound of formula (XVII) 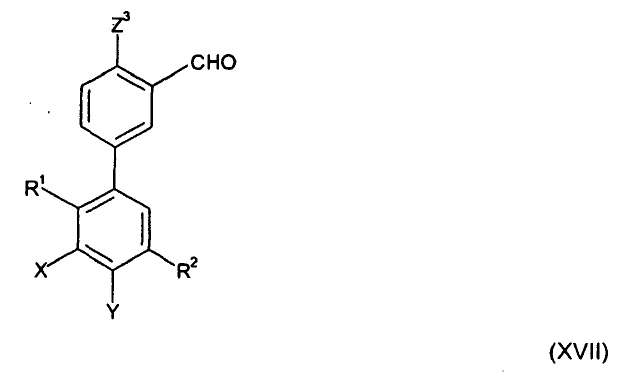 in which R¹, R², X, Y and Z³ are as hereinbefore defined, with a hydrazine derivative of formula (VIIIA) or (VIIIB) as hereinbefore defined.

A compound of formula (I) may also be prepared by reacting a compound of formula (XVIII) 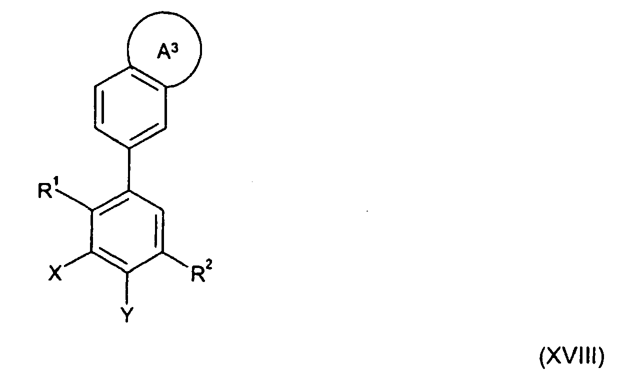 in which R¹, R², X and Y are as hereinbefore defined and A³ is a fused 5-membered heteroaryl ring substituted by halogen, with a suitable boronic acid derivative.

Alternatively, a further general method comprises final stage modification of one compound of formula (I) into another compound of formula (I). Suitable functional group transformations for converting one compound of formula (I) into another compound of formula (I) are well known in the art and are described in, for instance, Comprehensive Heterocyclic Chemistry II, eds. A. R. Katritzky, C. W. Rees and E. F. V. Scriven (Pergamon Press,1996), Comprehensive Organic Functional Group Transformations, eds. A.R. Katritzky, O. Meth-Cohn and C.W. Rees (Elsevier Science Ltd., Oxford, 1995), Comprehensive Organic Chemistry, eds. D. Barton and W.D. Ollis (Pergamon Press, Oxford, 1979), and Comprehensive Organic Transformations, R.C. Larock (VCH Publishers Inc., New York, 1989).

For example, one general method for preparing the compounds of formula (I) comprises the reaction set out in Scheme 1 below.

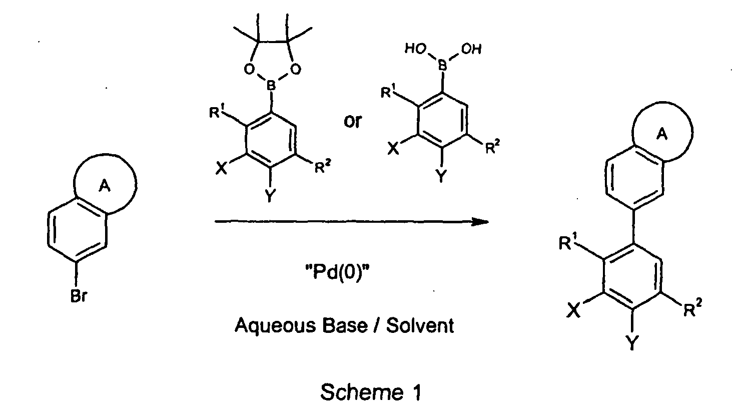

For example, another general method for preparing the compounds of formula (I) comprises the reactions set out in Scheme 2 below.

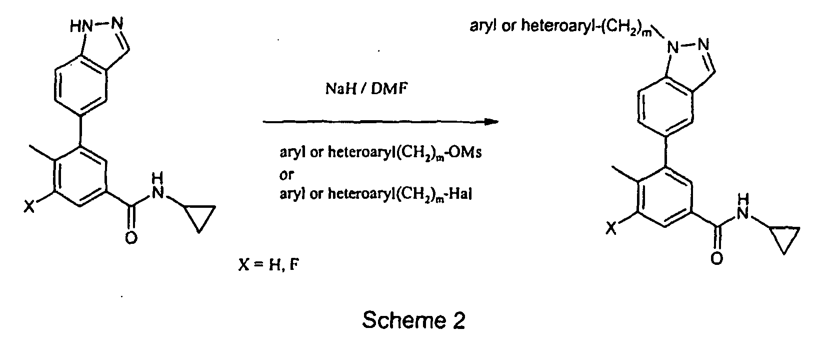

For example, another method for preparing the compounds of formula (I) comprises the reactions set out in Scheme 3 below.

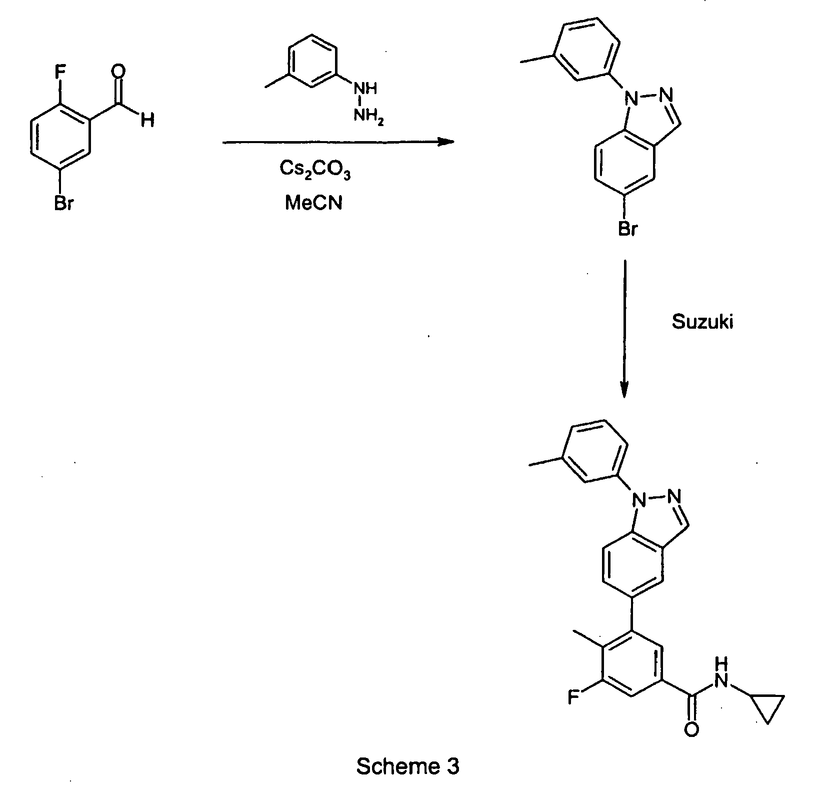

For example, another method for preparing the compounds of formula (I) comprises the reactions set out in Scheme 4 below.

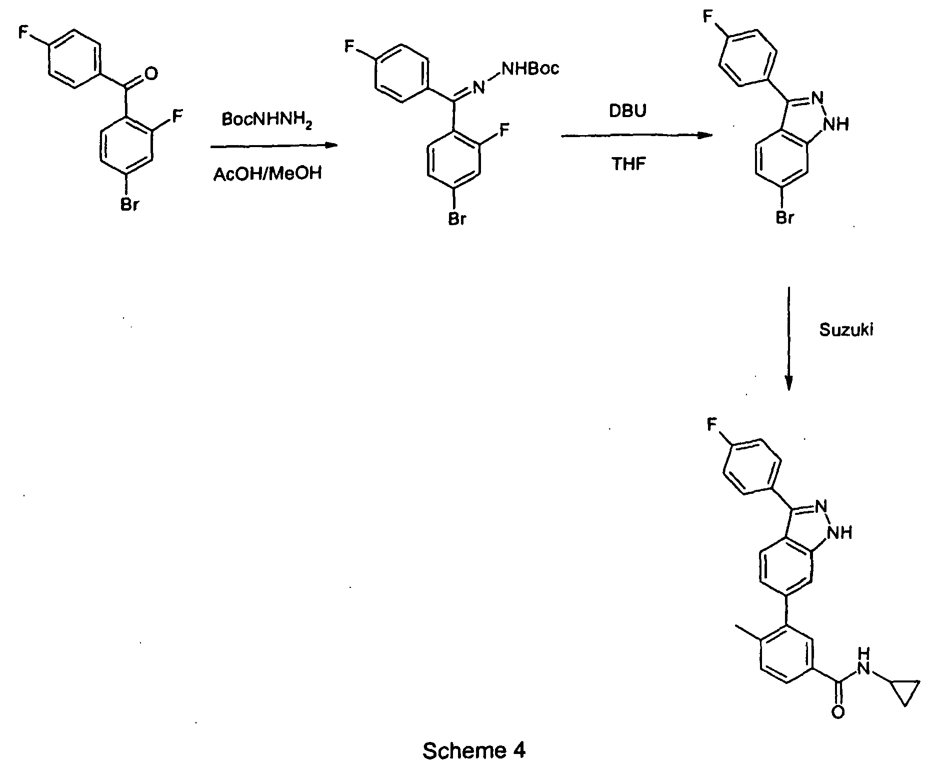

For example, another method for preparing the compounds of formula (I) comprises the reactions set out in Scheme 5 below.

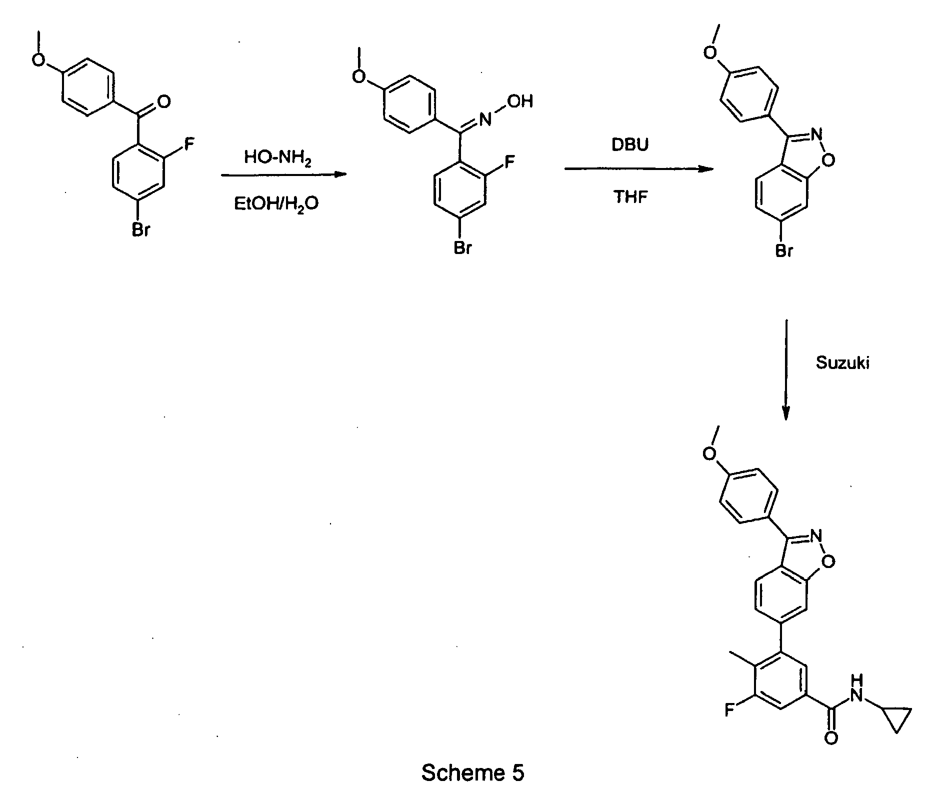

For example, another method for preparing the compounds of formula (I) comprises the reactions set out in Scheme 6 below.

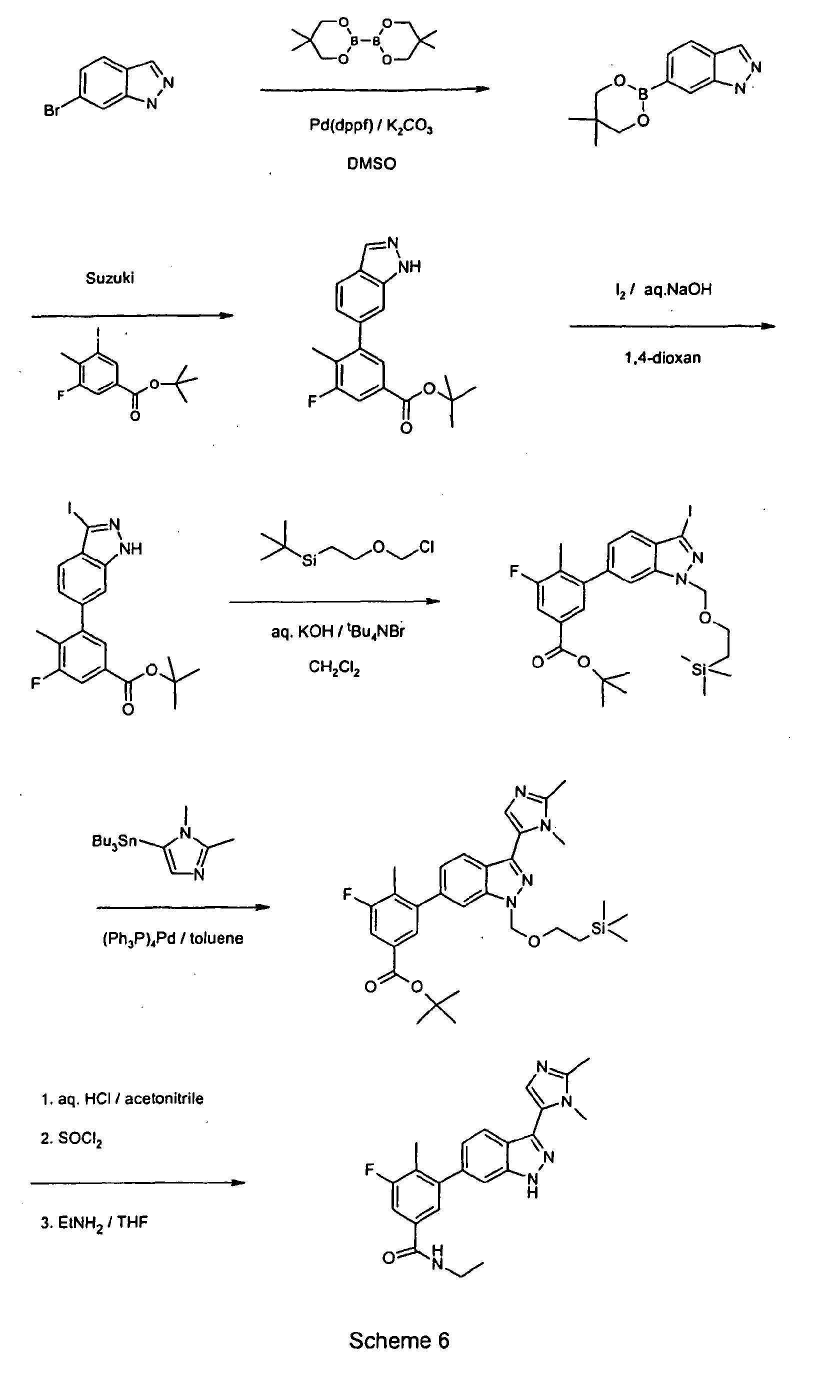

For example, a further method for preparing the compounds of formula (I) comprises the reactions set out in Scheme 7 below.

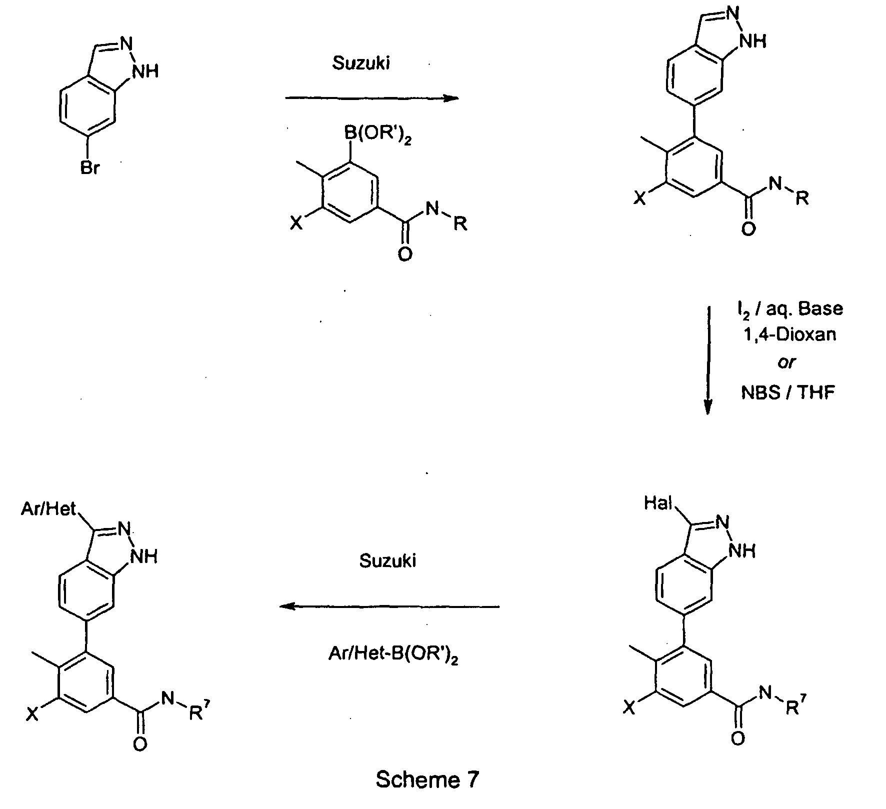

Those skilled in the art will appreciate that in the preparation of the compounds of the invention it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tert-butyldimethylsilyl; alkyl ethers such as tetrahydropyranyl or tert-butyl; or esters such as acetate.

Whilst it is possible for the compounds of the present invention to be administered as the raw chemical, the compounds of formula (I) and their pharmaceutically acceptable derivatives are conveniently administered in the form of pharmaceutical compositions eg when the agent is in admixture with a suitable pharmaceutical excipient, diluent and/or carrier selected with regard to the intended route of administration and standard pharmaceutical practice.

Thus, in another aspect of the invention, we provide a pharmaceutical composition comprising at least one compound of formula (I) or a pharmaceutically acceptable derivative thereof, in association with one or more pharmaceutically acceptable excipients, diluents and/or carriers. The excipient, diluent or carrier must be "acceptable" in the sense of being compatible with the other ingredients of the formulation and not deletrious to the recipient thereof.

According to a further aspect, the invention provides a pharmaceutical composition comprising, as active ingredient, at least one compound of the invention or a pharmaceutically acceptable derivative thereof, in association one or more pharmaceutically acceptable excipients, diluents and/or carriers for use in therapy, and in particular in the treatment of human or animal subjects suffering from a condition susceptible to amelioration by an inhibitor of p38 kinase.

The present invention also provides a pharmaceutical composition comprising a therapeutically effective amount of the compounds of the present invention and a pharmaceutically acceptable excipient, diluent and/or carrier (including combinations thereof).

There is further provided by the present invention a process of preparing a pharmaceutical composition, which process comprises mixing at least one compound of the invention or a pharmaceutically acceptable derivative thereof, together with a pharmaceutically acceptable excipient, diluent and/or carrier.

The pharmaceutical compositions may be for human or animal usage in human and veterinary medicine and will typically comprise any one or more of a pharmaceutically acceptable excipient, diluent or carrier. Acceptable carriers or diluents for therapetic use are well known in the pharmaceutical art, and are described, for example, in Remington's Pharmaceutical Sciences, Mack Publishing Co. (A. R. Gennaro edit. 1985). The choice of pharmaceutical excipient, diluent or carrier can be selected with regard to the intended route of administration and standard pharmaceutical practice. The pharmaceutical compositions may comprise as - or in addition to - the excipient, diluent or carrier any suitable binder(s), lubricant(s), suspending agent(s), coating agent(s) and solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

For some embodiments, the agents of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta- and gamma-cyclodextrins are most commonly used and suitable examples are described in WO 91/11172, WO 94/02518 and WO 98/55148.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see WO 02/00196 (SmithKline Beecham).

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The routes for administration (delivery) include, but are not limited to, one or more of: oral (e. g. as a tablet, capsule, or as an ingestable solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual. It is to be understood that not all of the compounds need be administered by the same route. Likewise, if the composition comprises more than one active component, then those components may be administered by different routes.

The compounds of formula (I) and their pharmaceutically acceptable salts and solvates may be formulated for administration in any suitable manner. They may, for example, be formulated for topical administration or administration by inhalation or, more preferably, for oral, transdermal or parenteral administration. The pharmaceutical composition may be in a form such that it can effect controlled release of the compounds of formula (I) and their pharmaceutically acceptable derivatives. In a preferred embodiment, the agents of the present invention are delivered systemically such as orally, buccally or sublingually. A particularly preferred method of administration, and corresponding formulation, is oral administration.

For oral administration, the pharmaceutical composition may take the form of, and be administered as, for example, tablets (including sub-lingual tablets) and capsules (each including timed release and sustained release formulations), ovules, pills, powders, granules, elixirs, tinctures, emulsions, solutions, syrups or suspensions prepared by conventional means with acceptable excipients for immediate-, delayed-, modified-, sustained-, pulsed- or controlled-release applications.

For instance, for oral administration in the form of a tablet or capsule, the active drug component can be combined with an oral, non-toxic pharmaceutically acceptable inert carrier such as ethanol, glycerol, water and the like. The tablets may also contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules can be made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin; natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate.

The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additives such as peppermint oil or saccharin, and the like can also be added.

Where appropriate, dosage unit formulations for oral administration can be microencapsulated. The formulation can also be prepared to prolong or sustain the release as for example by coating or embedding particulate material in polymers, wax or the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of the present invention can also be administered in the form of liposome emulsion delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

The present invention includes pharmaceutical compositions containing 0.1 to 99.5%, more particularly, 0.5 to 90% of a compound of the formula (I) in combination with a pharmaceutically acceptable carrier.

Likewise, the composition may also be administered in nasal, ophthalmic, otic, rectal, topical, intravenous (both bolus and infusion), intraperitoneal, intraarticular, subcutaneous or intramuscular, inhalation or insufflation form, all using forms well known to those of ordinary skill in the pharmaceutical arts.

For transdermal administration, the pharmaceutical composition may be given in the form of a transdermal patch, such as a transdermal iontophoretic patch.

If the compound of the present invention is administered parenterally, then examples of such administration include one or more of: intravenously, intraarterially, intraperitoneally, intrathecally, intraventricularly, intraurethrally, intrasternally, intracranially, intramuscularly or subcutaneously administering the agent; and/or by using infusion techniques. For parenteral administration, the pharmaceutical composition may be given as an injection or a continuous infusion (e.g. intravenously, intravascularly or subcutaneously). The compositions may take such forms as suspensions, solutions or emulsions in oily or aqueous vehicles and may contain formulatory a gents such as suspending, stabilizing and/or dispersing agents. F or administration by injection these may take the form of a unit dose presentation or as a multidose presentation preferably with an added preservative. Alternatively for parenteral administration the active ingredient may be in powder form for reconstitution with a suitable vehicle. For parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The compositions of the present invention may be administered by direct injection.

The compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

Alternatively the composition may be formulated for topical application, for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

For administration by inhalation the compounds according to the invention are conveniently delivered in the form of an aerosol spray presentation from pressurized packs or a nebulizer, with the use of a suitable propellant, e.g. dichlorodifluoromethane, trichlorofluoromethane, dichlorotetrafluoroethane, a hydrofluoroalkane such as tetrafluoroethane or heptafluoropropane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

Alternatively, the compound of the present invention can be administered in the form of a suppository or pessary, or it may be applied topically in the form of a gel, hydrogel, lotion, solution, cream, ointment or dusting powder.

The compounds of the present invention may also be administered by the pulmonary or rectal routes. They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

The pharmaceutical compositions generally are administered in an amount effective for treatment or prophylaxis of a specific condition or conditions. Initial dosing in humans is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, dose is from about 100 µg/kg to about 5 mg/kg body weight, daily. For administration particularly to mammals, and particularly humans, it is expected that the daily dosage level of the active agent will be from 0. 1 mg/kg to 10 mg/kg and typically around 1 mg/kg. It will be appreciated that optimum dosage will be determined by standard methods for each treatment modality and indication, taking into account the indication, its severity, route of administration, complicating conditions and the like. The physician in any event will determine the actual dosage which will be most suitable for an individual and will vary with the activity of the specific compound to be employed, the metabolic stablity and length of action of that compound, age, weight, general health, sex, diet, mode and time of administration, rate of excretion, drug combination, severity of the particular condition and response of the particular individual. The effectiveness of a selected actual dose can readily be determined, for example, by measuring clinical symptoms or standard anti-inflammatory indicia after administration of the selected dose. The above dosages are exemplary of the average case. There can, of course, be individual instances where higher or lower dosage ranges are merited, and such are within the scope of this invention. For conditions or disease states as are treated by the present invention, maintaining consistent daily levels in a subject over an extended period of time, e.g., in a maintenance regime, can be particularly beneficial. For oral and parenteral administration to humans, the daily dosage level of the agent may be in single or divided doses.

In another aspect, the present invention provides a compound of formula (I) or a pharmaceutically acceptable derivative thereof, for use in therapy.

The compounds of the present invention are generally inhibitors of the serine/threonine kinase p38 and are therefore also inhibitors of cytokine production which is mediated by p38 kinase. Within the meaning of the term "inhibitors of the serine/threonine kinase p38" are included those compounds that interfere with the ability of p38 to transfer a phosphate group from ATP to a protein substrate according to the assay described below.

It will be appreciated that the compounds of the invention may be selective for one or more of the isoforms of p38, for example p38α, p38β, p38γ and/or p38δ. In one embodiment, the compounds of the invention selectively inhibit the p38α isoform. In another embodiment, the compounds of the invention selectively inhibit the p38β isoform. In a further embodiment, the compounds of the invention selectively inhibit the p38α and p38β isoforms. Assays for determining the selectivity of compounds for the p38 isoforms are described in, for example, WO 99/61426, WO 00/71535 and WO 02/46158.

It is known that p38 kinase activity can be elevated (locally or throughout the body), p38 kinase can be incorrectly temporally active or expressed, p38 kinase can be expressed or active in an inappropriate location, p38 kinase can be constitutively expressed, or p38 kinase expression can be erratic; similarly, cytokine production mediated by p38 kinase activity can be occurring at inappropriate times, inappropriate locations, or it can occur at detrimentally high levels.

Accordingly, the present invention provides a compound of formula (I) or a pharmaceutically acceptable derivative thereof for use in the treatment or prophylaxis of a condition or disease state mediated by p38 kinase activity or mediated by cytokines produced by the activity of p38 kinase.

The present application describes a method for the treatment of a condition or disease state mediated by p38 kinase activity, or mediated by cytokines produced by the activity of p38 kinase, in a subject which comprises administering to said subject a therapeutically effective amount of a compound of formula (I) or a pharmaceutically acceptable derivative thereof. The compound may be administered as a single or polymorphic crystalline form or forms, an amorphous form, a single enantiomer, a racemic mixture, a single stereoisomer, a mixture of stereoisomers, a single diastereoisomer or a mixture of diastereoisomers.

The present application describes a method of inhibiting cytokine production which is mediated by p38 kinase activity in a subject, e.g. a human, which comprises administering to said subject in need of cytokine production inhibition a therapeutic, or cytokine-inhibiting, amount of a compound of the present invention. The compound may be administered as a single or polymorphic crystalline form or forms, an amorphous form, a single enantiomer, a racemic mixture, a single stereoisomer, a mixture of stereoisomers, a single diastereoisomer or a mixture of diastereoisomers.

These conditions may be treated by providing a therapeutically effective amount of a compound of this invention. By "therapeutically effective amount" is meant a symptomalleviating or symptom-reducing amount, a cytokine-reducing amount, a cytokine-inhibiting amount, a kinase-regulating amount and/or a kinase-inhibiting amount of a compound. Such amounts can be readily determined by standard methods, such as by measuring cytokine levels or observing alleviation of clinical symptoms. For example, the clinician can monitor accepted measurement scores for anti-inflammatory treatments. It will be appreciated that reference to treatment includes acute treatment or prophylaxis as well as the alleviation of established symptoms.

The compounds of the present invention can be administered to any subject in need of inhibition or regulation of p38 kinase or in need of inhibition or regulation of p38 mediated cytokine production. In particular, the compounds may be administered to mammals. Such mammals can include, for example, horses, cows, sheep, pigs, mice, dogs, cats, primates such as chimpanzees, gorillas, rhesus monkeys, and, most preferably, humans.

Methods of treating or reducing symptoms in a human or animal subject suffering from, for example, rheumatoid arthritis, osteoarthritis, asthma, psoriasis, eczema, allergic rhinitis, allergic conjunctivitis, adult respiratory distress syndrome, chronic pulmonary inflammation, chronic obstructive pulmonary disease, chronic heart failure, silicosis, endotoxemia, toxic shock syndrome, inflammatory bowel disease, tuberculosis, atherosclerosis, neurodegenerative disease, Alzheimer's disease, Parkinson's disease, Huntington's disease, amyotrophic lateral sclerosis, epilepsy, multiple sclerosis, aneurism, stroke, irritable bowel syndrome, muscle degeneration, bone resorption diseases, osteoporosis, diabetes, reperfusion injury, graft vs. host reaction, allograft rejections, sepsis, systemic cachexia, cachexia secondary to infection or malignancy, cachexia secondary to aquired immune deficiency syndrome (AIDS), malaria, leprosy, infectious arthritis, leishmaniasis, Lyme disease, glomerulonephritis, gout, psoriatic arthritis, Reiter's syndrome, traumatic arthritis, rubella arthritis, Crohn's disease, ulcerative colitis, acute synovitis, gouty arthritis, spondylitis, and non articular inflammatory conditions, for example, herniated/ruptured/prolapsed intervertebral disk syndrome, bursitis, tendonitis, tenosynovitis, fibromyalgic syndrome and other inflammatory conditions associated with ligamentous sprain and regional musculoskeletal strain, pain, for example that associated with inflammation and/or trauma, osteopetrosis, restenosis, thrombosis, angiogenesis, cancer including breast cancer, colon cancer, lung cancer or prostatic cancer, which comprises administering to said subject a therapeutically effective amount of a compound of formula (I) or a pharmaceutically acceptable derivative thereof.

Methods of treatment of a human or animal subject suffering from rheumatoid arthritis, asthma, psoriasis, chronic pulmonary inflammation, chronic obstructive pulmonary disease, chronic heart failure, systemic cachexia, glomerulonephritis, Crohn's disease, neurodegenerative disease, Alzheimer's disease, Parkinson's disease, epilepsy and cancer including breast cancer, colon cancer, lung cancer and prostatic cancer are described, which comprises administering to said subject a therapeutically effective amount of a compound of formula (I) or a pharmaceutically acceptable derivative thereof.

Methods of treatment of a human or animal subject suffering from rheumatoid arthritis, asthma, psoriasis, chronic pulmonary inflammation, chronic obstructive pulmonary disease, chronic heart failure, systemic cachexia, glomerulonephritis, Crohn's disease and cancer including breast cancer, colon cancer, lung cancer and prostatic cancer are described, which comprises administering to said subject a therapeutically effective amount of a compound of formula (I) or a pharmaceutically acceptable derivative thereof.

Methods of treatment of a human or animal subject suffering from rheumatoid arthritis, asthma, chronic pulmonary inflammation, chronic obstructive pulmonary disease, neurodegenerative disease, Alzheimer's disease, Parkinson's disease and epilepsy are described which comprises administering to said subject a therapeutically effective amount of a compound of formula (I) or a pharmaceutically acceptable derivative thereof.

Methods of treatment of a human or animal subject suffering from any type of pain including chronic pain, rapid onset of analgesis, neuromuscular pain, headache, cancer pain, acute and chronic inflammatory pain associated with osteoarthritis and rheumatoid arthritis, post operative inflammatory pain, neuropathic pain, diabetic neuropathy, trigeminal neuralgia, post-hepatic neuralgia, inflammatory neuropathies and migraine pain are described which comprises administering to said subject a therapeutically effective amount of a compound of formula (I) or a pharmaceutically acceptable derivative thereof.

A further aspect of the invention provides the use of a compound of formula (I), or a pharmaceutically acceptable derivative thereof, in the manufacture of a medicament for use in the treatment of a condition or disease state mediated by p38 kinase activity or mediated by cytokines produced by p38 kinase activity.

The compounds of formula (I) and their derivatives may be employed alone or in combination with other therapeutic agents for the treatment of the above-mentioned conditions. The invention thus provides, in a further aspect, a combination comprising a compound of the invention or a pharmaceutically acceptable derivative thereof together with a further therapeutic agent.

In particular, in rheumatoid arthritis therapy, combination with other chemotherapeutic or antibody agents is envisaged. Combination therapies according to the present invention thus comprise the administration of at least one compound of formula (I) or a pharmaceutically acceptable salt or solvate thereof and at least one other pharmaceutically active agent. The compound(s) of formula (I) or pharmaceutically acceptable salt(s) or solvate(s) thereof and the other pharmaceutically active agent(s) may be administered together or separately and, when administered separately, this may occur separately or sequentially in any order. The amounts of the compound(s) of formula (I) or pharmaceutically acceptable salt(s) or solvate(s) thereof and the other pharmaceutically active agent(s) and the relative timings of administration will be selected in order to achieve the desired combined therapeutic effect. Appropriate doses will be readily appreciated by those skilled in the art. It will be appreciated that the amount of a compound of the invention required for treatment will vary with the nature of the condition being treated and the age and condition of the patient and will ultimately be at the discretion of the attendant physician or veterinarian. Examples of other pharmaceutically active agents which may be employed in combination with compounds of formula (I) and their salts and solvates for rheumatoid arthritis therapy include: immunosuppresants such as amtolmetin guacil, mizoribine and rimexolone; anti-TNFα agents such as etanercept, infliximab, diacerein; tyrosine kinase inhibitors such as leflunomide; kallikrein antagonists such as subreum; interleukin 11 agonists such as oprelvekin; interferon beta 1 agonists; hyaluronic acid agonists such as NRD-101 (Aventis); interleukin 1 receptor antagonists such as anakinra; CD8 antagonists such as amiprilose hydrochloride; beta amyloid precursor protein antagonists such as reumacon; matrix metalloprotease inhibitors such as c ipemastat and other disease modifying anti-rheumatic drugs (DMARDs) such as methotrexate, sulphasalazine, cyclosporin A, hydroxychoroquine, auranofin, aurothioglucose, gold sodium thiomalate and penicillamine.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention.

The individual components of such combinations may be administered either sequentially or simultaneously in separate or combined pharmaceutical formulations by any convenient route.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

### EXAMPLES

The following Examples are illustrative embodiments of the invention, not limiting the scope of the invention in any way. Reagents are commercially available or are prepared according to procedures in the literature.

{5-[(Cyclopropylamino)carbonyl]-3-fluoro-2-methylphenyl}boronic acid, *N*-cyclopropyl-4-methyl-3-(4,4,5,5-tetramethyl-1,3,2-dioxaborolan-2-yl)benzamide, 4-methyl-3-(4,4,5,5-tetramethyl-1,3,2-dioxaborolan-2-yl)benzoic acid and 4-methyl-3-(4,4,5,5-tetramethyl-1,3,2-dioxaborolan-2-yl)benzoic acid may be prepared by the procedures described in WO 03/068747.

(4-Fluoro-2-methylphenyl)boronic acid may be prepared by the procedure described by J.B. Doherty, et al. in WO 02/058695 .

5-Bromoindazole may be prepared by the procedure described by A. Arnautu et al. in Tetrahedron Letters, 2002, 43, 2695.

3-(Bromomethyl)-5-methylisoxazole and 4-hydrazino-2,6-dimethylpyrimidine may be purchased from Maybridge International.

[4-(4-Morpholinyl)phenyl]boronic acid may be purchased from AsymChem International.

5-Bromo-1-(2-pyridinyl)-1*H*-indole, 5-bromo-1-(3-pyridinyl)-1*H*-indole and 3-(5-bromo-1*H*-indol-1-yl)benzonitrile may be prepared by the procedures described by G.H. Ladouceur, et al. in WO 03/027094.

4-Methylsulfonylphenylhydrazine may be purchased from Apin Chemical Ltd.

2-(4-Aminophenyl)-*N*-methylacetamide may be prepared by the procedure described by J.A. Cipollina, et al. in EP 666 258 A1.

Tetrahydro-2*H*-pyran-4-ylamine may be prepared by the procedure described by M Allegretti, et al. in Tetrahedron Letters, 2001, 42, 57.

6-Hydrazino-4(1*H*)-pyrimidinone may be prepared by the procedure described by B.E. Christensen, et al. in Journal of Organic Chemistry, 1971, 36, 2462.

6-Bromo-3-[4-(methyloxy)phenyl]-1,2-benzisoxazole and 4-(6-bromo-1,2-benzisoxazol-3-yl)phenol may be prepared by the procedures described by J. Aebi, et al. in EP 778 271 A2.

2-Aminopyridazine may be purchased from Sigma-RBI.

(4-Bromo-2-fluorophenyl)(4-fluorophenyl)methanone a nd (4-bromo-2-fluorophenyl)[4-(methyloxy)phenyl]methanone may be prepared by the procedures described by A Levy et al. in Journal of Organic Chemistry, 2003, 68, 3990.

LCMS was conducted on a column (3.3cm x 4.6mm ID, 3µm ABZ+PLUS), at a Flow Rate of 3ml/min, Injection Volume of 5µl, at room temperature and UV Detection Range at 215 to 330nm. Solvent A: 10mM Aqueous ammonium acetate + 0.1% formic acid. Solvent B: 95% Acetonitrile + 0.05% formic acid. Gradient : 0% A/0.7min, 0-100% A/3.5min, 100% A/1.1min, 100-0% A/0.2min.

### Intermediate 1: 5-Bromo-1-phenyl-1H-indazole

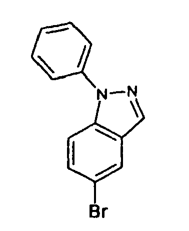

A solution of 5-bromo-2-fluorobenzaldehyde (10.0g) in boiling acetonitrile (90ml) was added to a solution of phenylhydrazine (8.3g) in boiling acetonitrile (360ml). The yellow solution was stirred for 5min, allowed to cool then concentrated under vacuum. The resulting solid was washed with hexane (3x10ml) and the residue was dissolved in DMSO (125ml). Cesium carbonate (32.9g) was added and the mixture was heated at 140°C for 24h then at 150°C for 40h. The reaction mixture was diluted with ethyl acetate (600ml) then washed with saturated sodium hydrogen carbonate (2x300ml) and brine (2x300ml). The solvent was removed under vacuum and the residue was recrystallised from methanol to give the title compound as a white solid (6.98g).
LC-MS: Rt 3.19min, MH+ 273/275.

### Intermediate 2: 5-Bromo-1-(4-fluorophenyl)-1H-indazole

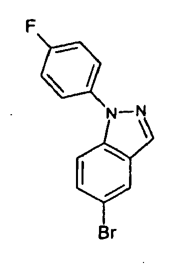

A solution of 4-fluorophenylhydrazine hydrochloride (27.4g) in ethyl acetate (50ml), was washed with saturated sodium hydrogen carbonate (3x20ml), dried and concentrated under vacuum. The resulting oil was dissolved in boiling acetonitrile (36ml) and a solution of 5-bromo-2-fluorobenzaldehyde (20g) in boiling acetonitrile (9ml) was added. The solution was allowed to cool, the solvent was evaporated and the residue was dissolved in DMSO (200ml). Cesium carbonate added and the mixture was heated by microwave in a sealed vessel at 200°C for 10min. The reaction mixture was diluted with ethyl acetate (1000ml) then washed with hydrochloric acid (1M, 3 x 200ml), saturated sodium hydrogen carbonate (2 x 200ml) and brine (200ml). The organic phase was dried and concentrated under vacuum and the residue was recrystallised from hexane to give the title compound as a white crystalline solid (16.98g).
LC-MS: Rt 3.13min, MH+ 291/293.

### Intermediate 3: tert-Butyl 5-bromo-1H-indazole-1-carboxylate

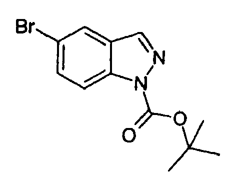

A stirred ice-cold suspension of 5-bromoindazole (2g), 4-(dimethylamino)pyridine (250mg) and triethylamine (1.55m) in acetonitrile (50ml) was treated with a solution of di-tert-butyl dicarbonate (2.8ml) in acetonitrile (20ml) over 15 min such that the temperature remained under 5°C. The reaction mixture was warmed to room temperature then stirred for 18h. The solvent was evaporated and the residue was purified by column chromatography on silica (100g) eluting with cyclohexane:ethyl acetate (15:1) to give the title compound (2.27g).
LCMS: Rt 3.55min.

### Intermediate 4: 1,1-Dimethylethyl 5-{5-[(cyclopropylamino)carbonyl]-3-fluoro-2-methylphenyl}-1H-indazole-1-carboxylate

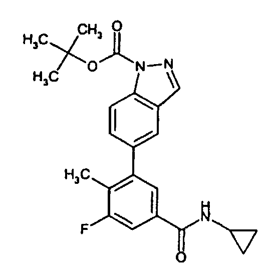

A mixture of tert-butyl 5-bromo-1H-indazole-1-carboxylate (Intermediate 3, 1.07g), {5-[(cyclopropylamino)carbonyl]-3-fluoro-2-methylphenyl}boronic acid (0.85g), sodium carbonate (1.9g) and tetrakis(triphenylphosphine)palladium(0) (0.42g) in 1,2-dimethoxyethane (70ml) was stirred at reflux under nitrogen for 20h. The solvent was removed a nd the residue was partitioned between water (50ml) a nd ethyl acetate (50ml). The aqueous layer was re-extracted with ethyl acetate (3x30ml) and the combined organic extracts were dried using a hydrophobic filter tube and concentrated under vacuum. The residue was purified by column chromatography on silica eluting with cyclohexane:ethyl acetate (75:25 to 60:40) to give the title compound (3.1g).
LCMS: Rt 3.46min, MH+ 410.

### Intermediate 5: N-Cyclopropyl-3-fluoro-5-(1H-indazol-5-yl)-4-methylbenzamide

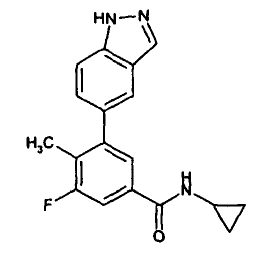

A mixture of 1,1-dimethylethyl 5-{5-[(cyclopropylamino)carbonyl]-3-fluoro-2-methylphenyl}-1H-indazole-1-carboxylate (Intermediate 4, 0.46g) in a solution of hydrogen chloride in dioxan (4M, 7ml) was stirred at room temperature under nitrogen for 4.5h. The solvent was evaporated and the residue was partitioned between dichloromethane (20ml) and aqueous sodium hydroxide (2M, 20ml). The organic layer was separated using a hydrophobic filter tube, the solvent was evaporated and the residue was purified on a Varian Bond-Elut SPE cartridge (silica, 10g) eluting with chloroform:methanol (100:0 to 98:2) to give the title compound (0.06g).
LCMS: Rt 2.96min, MH+ 310.

### Intermediate 6: (N-Cyclobutyl-4-methyl-3-(4,4,5,5-tetramethyl-1,3,2-dioxaborolan-2-yl)benzamide

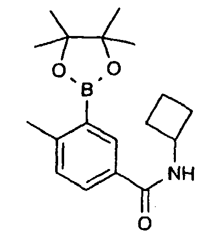

A mixture of 4-methyl-3-(4,4,5,5-tetramethyl-1,3,2-dioxaborolan-2-yl)benzoic acid (262mg) in chloroform (10ml) was stirred with 3-{[(ethylimino)methylidene]amino}-*N*,*N*,*N-*trimethyl-1-propanaminium iodide (450mg), 1-hydroxy-7-azabenzotriazole (13mg) and cyclobutylamine (102µl) for 18h. Water was added, the organic layer was separated using a hydrophobic filter tube and the solvent was removed under vacuum to give the title compound (210mg).
NMR: [δH d₆-DMSO] 8.59 (1H d, J=8Hz,), 8.08 (1H, d, J=1Hz), 7.81 (1H, dd, 8Hz J=1Hz), 7.26 (1H, d, J=8Hz), 4.47-4.37 (1H, m,), 2.50 (3H, s), 2.25-2.15 (2H, m), 2.14-2.03 (2H, m), 1.70-1.62 (2H, m), 1.32 (12H, s).

### Intermediate 7: Methyl 3-fluoro-5-iodo-4-methylbenzoate

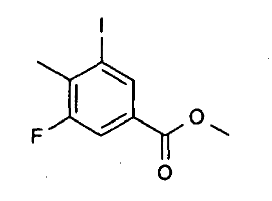

A stirred mixture of 3-fluoro-4-methylbenzoic acid (10.3g) in trifluoromethane sulfonic acid (50ml) at -20°C was treated with N-iodosuccinimide in portions over 40min. The reaction was stirred at -10°C for 44h when a further amount of N-iodosuccinimide (6.0g) was added. After 20h the reaction mixture was added to ice/water and extracted with ethyl acetate. The organic solution was washed with aqueous sodium metabisulfite and dried over sodium sulfate. The residue was dissolved in methanol (50ml), the solution was treated with concentrated sulfuric acid (91 ml) and the mixture was heated at reflux for 6h. The solvent was evaporated and the residue was dissolved in ethyl acetate. This solution was washed with aqueous sodium bicarbonate and dried with brine and over magnesium sulfate. Purification by biotage chromatography (x2), firstly using cyclohexane/ethyl acetate (100/1) and secondly cyclohexane/toluene (6/1) as eluents gave the title compound (9.31g).
LC-MS: Rt 3.55min.

### Intermediate 8: Methyl 3-fluoro-4-methyl-5-(4,4,5,5-tetramethyl-1,3,2-dioxaborolan-2-yl)benzoate

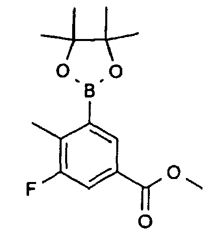

A mixture of methyl 3-fluoro-5-iodo-4-methylbenzoate (Intermediate 7, 9.04g), potassium acetate (15.06g), bis(pinacolato)diborane (11.7g) and [1,1'-bis(diphenylphosphino)ferrocene]-dichloropalladium(II) (251mg) in DMF (200ml) was deoxygenated then stirred at 90°C for 18h. The reaction mixture was evaporated to dryness, pre-absorbed on to silica and purified by Biotage chromatography using a cyclohexane/ethyl acetate gradient as eluent to give the title compound (8.39g).
LC-MS: Rt 3.78min.

### Intermediate 9: 3-Fluoro-4-methyl-5-(1-phenyl-1H-indazol-5-yl)benzoic acid

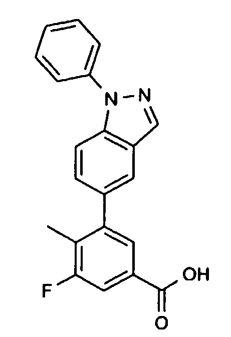

A mixture of 5-bromo-1-phenyl-1*H*-indazole (Intermediate 1, 272mg), methyl 3-fluoro-4-methyl-5-(4,4,5,5-tetramethyl-1,3,2-dioxaborolan-2-yl)benzoate (Intermediate 8, 294mg), tetrakis(triphenylphoshine)palladium (0) (30mg) and 1 M aqueous sodium bicarbonate (10ml) in isopropanol (10ml) was heated at reflux for 3h. The reaction mixture was acidified with 2M hydrochloric acid then diluted with ethyl acetate. The organic layer was separated using a hydrophobic filter tube, the solvent was evaporated and the residue was purified on an SPE cartridge (silica) eluting with a petroleum ether/ethyl acetate gradient containing 0.1 % acetic acid to give the title compound as a colourless solid (227mg).
LC-MS: Rt 3.94min MH+ 347.

### Intermediate 10: 4-(5-Bromo-1H-indol-1-yl)benzonitrile

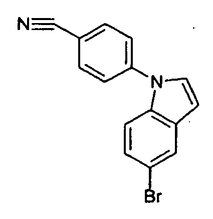

A solution of 5-bromo-1*H*-indole (196mg) and sodium hydride (60% dispersion in mineral oil, 24mg) in DMF (6ml) was stirred at room temperature under nitrogen for 1 h. A solution of 4-fluorobenzonitrile (446mg) in DMF (0.5) was added and the resulting mixture was stirred at 100°C under nitrogen for 12h. The mixture was partitioned between water and ethyl acetate and the organic layer was washed with water and brine, dried through a hydrophobic filter tube and concentrated under vacuum. The residue was partially purified on an SPE cartridge (silica, 5g) eluting with cyclohexane:ethyl acetate (99:1 to 1:1) to give impure title compound as a yellow oil.
LC-MS: Rt 3.27.

### Intermediate 11: 5-Bromo-1-(3-methylphenyl)-1H-indazole

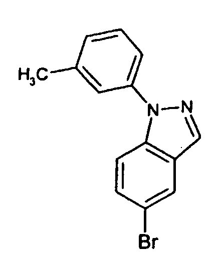

A mixture of 3-methylphenylhydrazine (240mg) and 5-bromo-2-fluorobenzaldehyde (0.2ml) in acetonitrile (1ml) was stirred at room temperature for 1 h. The solvent was removed under vacuum and the residue was dissolved in DMSO (2ml). Cesium carbonate (510mg) was added and the mixture was heated in a microwave oven at 200°C for 10min. The reaction mixture was diluted with ethyl acetate (10ml) then washed with hydrochloric acid (1M), aqueous sodium hydrogen carbonate and brine. The solvent was removed under vacuum and the residue was purified using an SPE cartridge (silica), eluting with cyclohexane to give the title compound as a yellow oil (157mg).
LC-MS: Rt 3.81 min, MH+ 287, 289.

### Intermediate 12: 5-Bromo-1-[4-(trifluoromethyl)phenyl]-1H-indazole

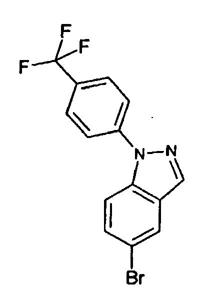

The procedure for Intermediate 11 was followed using 4-trifluoromethylphenylhydrazine (300mg), 5-bromo-2-fluorobenzaldehyde (0.2ml), acetonitrile (1ml), DMSO (2ml) and cesium carbonate (580mg) to give the title compound (202mg).
LC-MS: Rt 3.90min.

### Intermediate 13: 5-Bromo-1-[4-(methylsulfonyl)phenyl]-1H-indazole

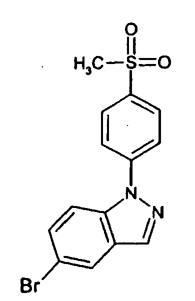

The procedure for Intermediate 11 was followed using 4-methylsulfonylphenylhydrazine (333mg), 5-bromo-2-fluorobenzaldehyde (0.21ml), acetonitrile (1ml), DMSO (2ml) and cesium carbonate (650mg) to give the title compound as a yellow solid (470mg).
LC-MS: Rt 3.24min, MH+ 351, 353.

### Intermediate 14: 2-(4-Hydrazinophenyl)-N-methylacetamide, hydrochloride

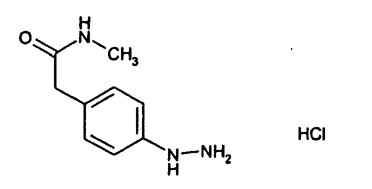

Sodium nitrite (8.5g) in water (18ml) was added over 30min to a stirred solution of 2-(4-aminophenyl)-*N*-methylacetamide (20g) in concentrated hydrochloric acid (41ml) at -7°C. The reaction mixture was stirred for 1h at -5°C then added over 30min to a solution of tin (II) chloride dihydrate (136.5g) in concentrated hydrochloric acid (160ml) maintaining a temperature below 0°C throughout. The reaction was stirred at -5°C for 2h and the white precipitate was collected by filtration and washed with ether (2x30ml). The solid was dissolved in hot methanol (110ml), filtered through Hyflo^{™} then treated with hot isopropyl acetate (325ml). The solution was allowed to cool, chilled in an ice bath and the resulting precipitate was collected by filtration, washed with isopropyl acetate (2x25ml) and dried to give the title compound as a white solid (22.2g).

| | |
|---|---|
| Microanalysis: | Found: C 49.09, H 6.53, N 18.92. |
| | Calculated for C9H13N30.HCl. 0.25H₂O: C 49.09, H 6.64, N 19.08. |

### Intermediate 15: 2-[4-(5-Bromo-1H-indazol-1-yl)phenyl]-N-methylacetamide

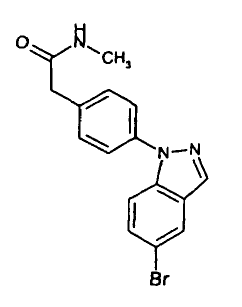

The procedure for Intermediate 11 was followed using 2-(4-hydrazinophenyl)-N-methylacetamide (Intermediate 14, 333mg), 5-bromo-2-fluorobenzaldehyde (0.21ml), acetonitrile (1ml), DMSO (2ml) and cesium carbonate (650mg) to give the title compound as a brown solid (495mg).
LC-MS: Rt 3.09min, MH+ 344, 346.

### Intermediate 16: N-Cyclopropyl-4',5-difluoro-3'-formyl-6-methyl-3-biphenylcarboxamide

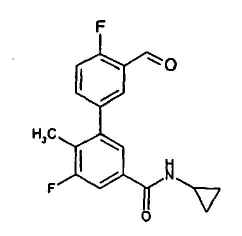

5-Bromo-2-fluorobenzaldehyde (0.48ml), {5-[(cyclopropylamino)carbonyl]-3-fluoro-2-methylphenyl}boronic acid (1.1g), tetrakis(triphenylphophine) palladium (101mg) and aqueous sodium hydrogen carbonate (1M, 8ml) were combined in isopropanol (16ml) and heated at 85°C for 18h. The cooled mixture was absorbed onto silica and applied to an SPE cartridge (silica, 50g). Elution with cyclohexane/ethyl acetate (4:1 to 1:1) gave the title compound as a yellow oil (650mg).
LC-MS: Rt 3.09min, MH+ 316.

### Intermediate 17: N-(4-Fluorophenyl)-4-methyl-3-(4,4,5,5-tetramethyl-1,3,2-dioxaborolan-2-yl)benzamide

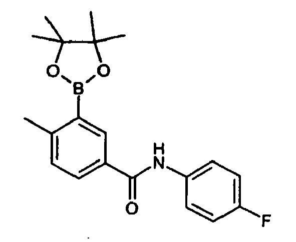

The procedure for Intermediate 6 was followed using 4-methyl-3-(4,4,5,5-tetramethyl-1,3,2-dioxaborolan-2-yl)benzoic acid (655mg), 3-{[(ethylimino)methylidene]amino}-*N*,*N*,*N* trimethyl-1-propanaminium iodide (719mg), 1-hydroxy-7-azabenzotriazole (34mg) and 4-fluoroaniline (360µl) in chloroform (10ml). The crude product, in methanol (30ml) was applied to an SCX cartridge and eluted with methanol to give the title compound as a pink foam (780mg).
LC-MS: Rt 3.62min.

### Intermediate 18: N-Ethyl-4-methyl-3-(4,4,5,5-tetramethyl-1,3,2-dioxaborolan-2-yl)benzamide

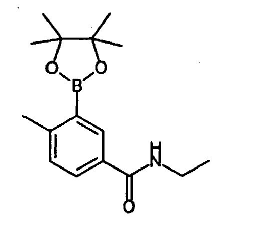

The procedure for Intermediate 6 was followed using 4-methyl-3-(4,4,5,5-tetramethyl-1,3,2-dioxaborolan-2-yl)benzoic acid (1.31g), 3-{[(ethylimino)methylidene]amino}-*N*,*N*,*N-*trimethyl-1-propanaminium iodide (2.23g), 1-hydroxy-7-azabenzotriazole (68mg) and a solution of ethylamine in THF (2M, 5ml) in chloroform (30ml).The crude product, in methanol was applied to an SCX cartridge and eluted with methanol to give the title compound as a pale yellow solid (1.36g).
LC-MS: Rt 3.20min.

### Intermediate 19: 1,1-Dimethylethyl (2Z)-2-[(4-bromo-2-fluorophenyl)(4-fluorophenyl)methylidene]hydrazinecarboxylate

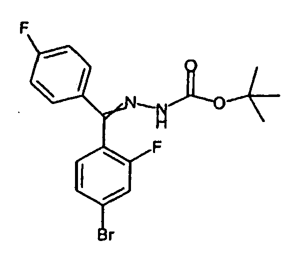

A mixture of (4-bromo-2-fluorophenyl)(4-fluorophenyl)methanone (176mg), tert-butyl carbazate (76mg) and acetic acid in methanol (4ml) was stirred at reflux for 66h. The reaction mixture was partitioned between water and ethyl acetate, the phases were separated and the organic layer was washed with water and brine. The dried (Na₂SO₄) extracts were concentrated under vacuum and the residue was purified on a SPE cartridge (silica, 5g) eluting with cyclohexane/ethyl acetate (9:1) to give the title compound as a white solid (141 mg).
LC-MS: Rt 3.71min.

### Intermediate 20: 6-Bromo-3-(4-fluorophenyl)-1H-indazole

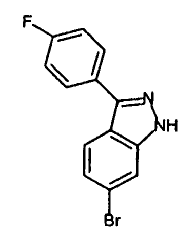

A solution of 1,1-dimethylethyl (2*Z*)-2-[(4-bromo-2-fluorophenyl)(4-fluorophenyl)methylidene]hydrazinecarboxylate (Intermediate 19, 141mg) and 1,8-diazabicyclo[5.4.0]undec-7-ene (32µl) in THF (5ml) was heated at 150°C for 30min in a microwave oven. Water and chloroform were added and the organic layer was separated using a hydrophobic filter tube. The solvent was evaporated and the residue was purified on an SPE cartridge (silica, 2g) eluting with cyclohexane/ethyl acetate (100:0 to 80:20) to give the title compound (42mg).
LC-MS: Rt 3.71min.

### Intermediate 21: 1,1-Dimethylethyl 2-{(4-bromo-2-fluorophenyl)[4-(methyloxy)phenyl]methylidene}

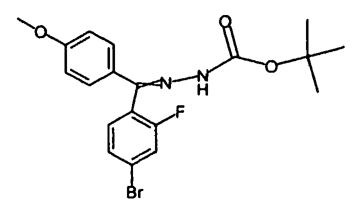

The procedure for Intermediate 19 was followed using (4-bromo-2-fluorophenyl)[4-(methyloxy)phenyl]methanone (183mg) tert-butyl carbazate (76mg) acetic acid (0.4ml) and methanol (4ml) to give the title compound as a yellow solid (110mg).
LC-MS: Rt 3.62min.

### Intermediate 22: 6-Bromo-3-[4-(methyloxy)phenyl]-1H-indazole

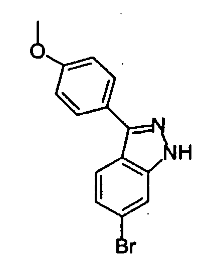

The procedure for Intermediate 20 was followed using 1,1-dimethylethyl 2-{(4-bromo-2-fluorophenyl)[4-(methyloxy)phenyl]methylidene}hydrazinecarboxylate Intermediate 21, 110mg) and 1,8-diazabicyclo[5.4.0]undec-7-ene (39µl) and THF (5ml) to give the title compound (53mg).
LC-MS: Rt 3.62min.

### Intermediate 23: N-Ethyl-3-(1H-indazol-6-yl)-4-methylbenzamide

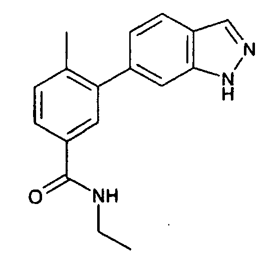

A stirred mixture of 6-iodo-1H-indazole (0.45g), N-ethyl-4-methyl-3-(4,4,5,5-tetramethyl-1,3,2-dioxaborolan-2-yl)benzamide (Intermediate 18, 0.54g) tetrakis(triphenylphosphine)palladium(0) (0.05g) and aqueous sodium hydrogen carbonate (1M, 1ml) in isopropanol (10ml) was heated at 150°C for 30min in a microwave oven. The reaction mixture was poured into water (50ml) and extracted with ethyl acetate (3x25ml). The extracts were washed with water (25ml) dried (Na₂SO₄) a nd concentrated under vacuum. The residual oil was purified by column chromatography on silica (50g) eluting with ether/ethyl acetate (7:3) to give the title compound as a pale yellow foam (0.25g). LC-MS: Rt 2.7min.

### Intermediate 24: N-Ethyl-3-(3-iodo-1H-indazol-6-yl)-4-methylbenzamide

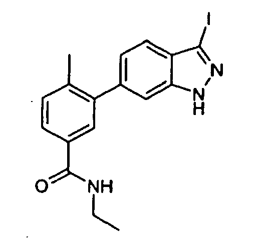

lodine (1.35g) was added to a stirred solution of N-ethyl-3-(1H-indazol-6-yl)-4-methylbenzamide(Intermediate 23, 1.1g) in 1,4-dioxan (20ml) and aqueous sodium hydroxide (2M, 20ml) then stirred at room temperature for 10min. The reaction mixture was treated with aqueous sodium bisulphite (10%, 25ml) and aqueous citric acid (10%, 25ml). The mixture was extracted with ethyl acetate (3x25ml) and the organic extracts were washed with water (30ml) dried (Na₂SO₄) and concentrated under vacuum to give the title-compound(1.54g).
LC-MS: Rt 3.29min, MH⁺406.

### Intermediate 25: N-Ethyl-3-fluoro-5-iodo-4-methylbenzamide

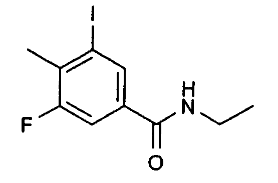

3-Fluoro-5-iodo-4-methylbenzoic acid (Intermediate 69, 20g) in thionyl chloride (20ml) was heated at 110°C for 1 h. The excess thionyl chloride was evaporated under vacuum and the residual oil was dissolved in DCM (100ml). Potassium carbonate (21g) was added to the solution followed by the slow addition of ethylamine (2M in THF, 70ml). The reaction was left at room temperature overnight, filtered and the residue was washed with ethyl acetate. The combined filtrate and washings were reduced to dryness under vacuum a nd the resulting solid was washed with ether/cyclohexane (1:1) to give the title compound as a pale beige solid (18.5g).
NMR: [δH d₆-DMSO] 8.58(1H, b), 8.15(1H, s), 7.64(1H, d), 3.26(2H, quin), 2.33 (3H, s), 1.11 (3H, t).

### Intermediate 26: {5-[(Ethylamino)carbonyl]-3-fluoro-2-methylphenyl}boronic acid

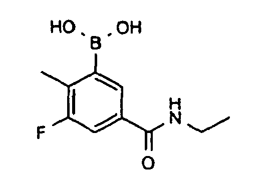

Sodium hydride (60% in mineral oil, 1.23g) was added in portions to a solution of N-ethyl-3-fluoro-5-iodo-4-methylbenzamide (Intermediate 25, 4.81g) in anhydrous THF (75ml). The resulting mixture was cooled to -75°C and n-butyllithium (1.6M in hexanes, 20ml) was added dropwise over 20min. Triisopropylborate (8ml) was added over 5min and the reaction mixture was stirred at -75°C for 6h. Water (20 ml) was added and the mixture was warmed to 15°C overnight and the mixture was partitioned between saturated aqueous ammonium chloride and ethyl acetate. The organic phase was washed with aqueous ammonium chloride and brine, dried (MgSO₄) and concentrated under vacuum. The residue was dissolved in DCM and applied to a silica column (10g) eluting with an ethyl acetate/dichloromethane gradient (0-100% ethyl acetate) followed by methanol. The methanol fractions were concentrated under vacuum to give the title compound as an off-white foam (550mg).
NMR: [δH d₄-MeOH] 7.55(1H, s), 7.48(1H, d), 3.38(2H, q), 3.30(2H, b), 2.28,(3H, s), 1.20(3H, t).

### Intermediate 27: N-Ethyl-3-fluoro-5-(3-iodo-1H-indazol-6-yl)-4-methylbenzamide

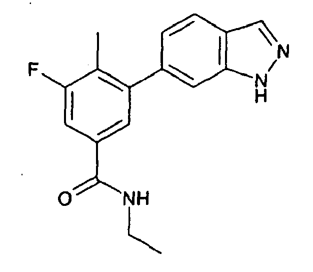

A stirred mixture of 6-iodo-1H-indazole (0.5g) {5-[(ethylamino)carbonyl]-3-fluoro-2-methylphenyl}boronic acid (Intermediate 16, 0.56g), tetrakis(triphenylphosphine)palladium(0) (0.05g) and aqueous sodium hydrogen carbonate (1M, 1ml) in isopropanol (10ml) was heated at 150°C for 30min in a microwave oven. The reaction mixture was poured into water (30ml) and extracted with ethyl acetate (3x20ml). The extracts were dried (Na₂SO₄) and concentrated under vacuum. The residual oil was purified by column chromatography on silica (50g) eluting with ether/ethyl acetate (4:1). The resultant product was triturated with a small quantity of dichloromethane to give the title compound as a pale yellow solid (0.15g).
LC-MS: Rt 2.96min, MH+ 298.

### Intermediate 28: N-Ethyl-3-fluoro-5-(3-iodo-1H-indazol-6-yl)-4-methylbenzamide

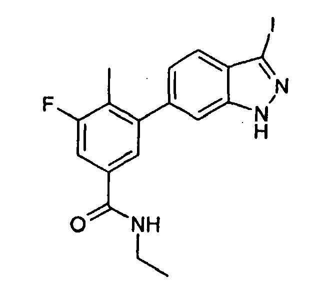

Iodine (0.153g) was added to a stirred solution of *N*-ethyl-3-fluoro-5-(3-iodo-1*H-*indazol-6-yl)-4-methylbenzamide (Intermediate 27, 0.15g) in 1,4-dioxan (4ml) and aqueous sodium hydroxide (2M, 4ml) then stirred at room temperature for 45min. The reaction mixture was treated aqueous citric a cid (20%, 20ml) followed b y s odium metabisulphite (2g). The mixture was diluted with water then extracted with ethyl acetate (2x30ml). The organic extracts were washed with water (30ml) dried (Na₂SO₄) and concentrated under vacuum to give the title-compound as a pale yellow foam (0.21g).
LC-MS: Rt 3.41min, MH⁺424.

### Intermediate 29: (4-Bromo-2-fluorophenyl)[6-(methyloxy)-3-pyridinyl]methanone

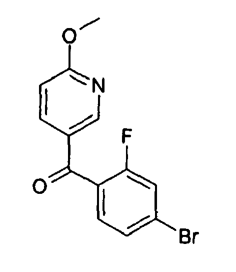

A mixture of 6-(methyloxy)-3-pyridinecarboxylic acid (0.306g) and thionyl chloride (6ml) was stirred at reflux for 2h and the solvent was removed under vacuum. The residue was co-evaporated with THF (x2) to remove residual thionyl chloride. A mixture of he residue and tetrakis(triphenylphosphine)palladium(0) (20mg) in dry T HF (5ml) was treated with a solution of (4-bromo-2-fluorophenyl)(iodo)zinc in THF(0.5M, 8.1ml) thenstirred at room temperature for 1.5h. The reaction mixture was treated with 1aqueous ammonium chloride (1M) then diluted with ethyl acetate and aqueous ammonium chloride. The organic layer was separated using a hydrophobic filter tube and concentrated under vacuum. The residue was purified on a SPE cartridge (silica, 50g) eluting with cyclohexane/ethyl acetate (100:0 to 0:100) to give impure title compound (0.331 g) as an amber oil.
LC-MS: Rt 3.3min MH+ 310/312.

### Intermediate 30: 6-Bromo-3-[6-(methyloxy)-3-pyridinyl]-1H-indazole

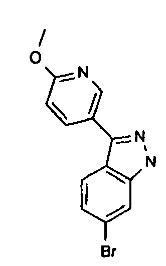

A mixture of impure (4'-bromo-2',3-difluoro-4-biphenylyl)[6-(methyloxy)-3-pyridinyl]methanone (0.33g) in methanol (5ml) was treated with 1,1-dimethylethyl hydrazinecarboxylate (93mg) and acetic acid (0.1ml) then stirred at reflux for 24h. More 1,1-dimethylethyl hydrazinecarboxylate (57mg) and acetic acid (20µl) were added and heating was continued for a further 24h. The reaction mixture was diluted with dichloromethane and stirred rapidly with saturated aqueous sodium bicarbonate. The organic layer was separated using a hydrophobic filter tube and concentrated under vacuum. The residue was purified by column chromatography on silica, e luting with cyclohexane:ethyl acetate (100:0 t o 0 : 100). The isolated product, in THF (4.5ml) was treated with 1,8-diazabicyclo[5.4.0]undec-7-ene (150µl) and heated in a sealed tube in a microwave oven at 150°C for 30min. The solvent was evaporated and the residue was purified by column chromatography on silica eluting with cyclohexane:ethyl acetate (100:0 to 0:100) to give the title compound (59mg).
LC-MS: Rt 3.5min, MH⁺ 304/306.

### Intermediate 31: N-Ethyl-3-fluoro-4-methyl-5-(4,4,5,5-tetramethyl-1,3,2-dioxaborolan-2-yl)benzamide

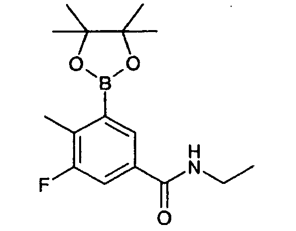

*N*-Ethyl-3-fluoro-5-iodo-4-methylbenzamide (Intermediate 25, 10.9g), bispinnacolcatodiborane (9.9g), Pd(dppf)Cl₂ (600mg) and potassium acetate (17.3g) were mixed in DMF (210ml). The mixture was degassed and then heated at 85°C under nitrogen for 18h. The cooled reaction was absorbed onto silica and applied to a silica column, eluting with an ethyl acetate/cyclohexane gradient (5-25% ethyl acetate). The resultant product was recrystallised from cyclohexane to give the title compound as a white solid (2.83g).
NMR: [δH d₆-DMSO] 8.54(1H, bt), 7.94(1H, s), 7.68(1H, d), 3.27,(2H, quin), 2.42(3H, s), 1.32(12H, s), 1.11(3H, t).

### Intermediate 32: (4-Bromo-2-fluorophenyl)(2-pyridinyl)methanone

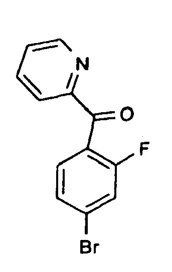

A mixture of picolinoyl chloride hydrochloride (356mg) and tetrakis(triphenylphosphine)palladium(0) (50mg) in THF (10ml) was added to (4-bromo-2-fluorophenyl)(iodo)zinc in THF (0.5M, 9ml) then stirred at room temperature for 2h. The reaction mixture was treated with aqueous ammonium chloride (1M) and diluted with ethyl acetate. The organic layer was separated using a hydrophobic filter tube and the solvent was evaporated. The residue was purified by column chromatography on silica (100g) eluting with cyclohexane/ethyl acetate (100:0 to 0:100) to give the title compound (180mg).
LC-MS: Rt 3.03min MH⁺ 280/282.

### Intermediate 33: 6-Bromo-3-(2-pyridinyl)-1H-indazole

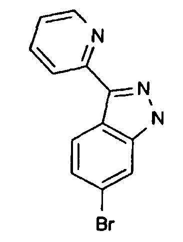

A mixture of (4-bromo-2-fluorophenyl)(2-pyridinyl)methanone (Intermediate 32, 180mg) in methanol (3ml) was treated with 1,1-dimethylethyl hydrazinecarboxylate (169mg) and acetic acid (0.1ml) then heated at reflux for 16h. The reaction mixture was partitioned between chloroform and saturated aqueous sodium hydrogen carbonate and the organic layer was concentrated under vacuum. The residue and 1,8-diazabicyclo[5.4.0]undec-7-ene (0.15ml) in THF (4.5ml) in a sealed tube was heated at 150°C in a microwave oven for 30min. The reaction mixture was evaporated and the residue was purified by column chromatography on silica eluting with cyclohexane/ethyl acetate (100:0 to 0:100) to give the title compound (74mg) as a white solid.
LC-MS: Rt 3.48min, MH⁺ 274/276.

### Intermediate 34: N-(1-Ethyl-1H-pyrazol-5-yl)-3-fluoro-5-iodo-4-methylbenzamide

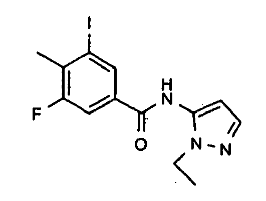

3-Fluoro-5-iodo-4-methylbenzoic acid (Intermediate 69, 6.27g) in thionyl chloride (10ml) was heated at 110°C for 2.5h. The mixture was left t o cool to room temperature overnight and the excess thionyl chloride was removed under vacuum. Potassium carbonate (1.81g) was added to a solution of the acid chloride (2.33g) in dichloromethane (10ml). A solution of 5-amino-1-ethylpyrazole (1.29g) in dichloromethane (5ml) was added and the mixture was stirred overnight at room temperature. The solvent was removed under vacuum and the residue was washed with water. The resulting solid was triturated with cyclohexane and ether to give the title compound as a cream solid (1.48g).
LCMS: Rt 3.18min, MH+ 374.

### Intermediate 35: N-(1-Ethyl-1H-pyrazol-5-yl)-3-fluoro-4-methyl-5-(4,4,5,5-tetramethyl-1,3,2-dioxaborolan-2-yl)benzamide

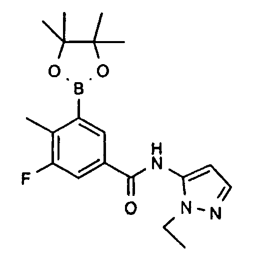

N-(1-Ethyl-1H-pyrazol-5-yl)-3-fluoro-5-iodo-4-methylbenzamide (Intermediate 34, 1.26g), bispinnacolatodiborane (2.59g), Pd(dppf)Cl₂ (50mg) and potassium acetate (1.01g) in DMF (34ml) was heated by microwave in a sealed vessel at 150°C for 15min. The reaction mixture was absorbed onto silica and applied to a silica column (100g). Elution with an ethyl acetate/cyclohexane gradient gave the title compound (850mg) as a yellow oil.
LCMS: Rt 3.36min, MH+ 374.

### Intermediate 36: (4-Bromo-2-fluorophenyl)(1,3-dimethyl-1H-pyrazol-5-yl)methanone

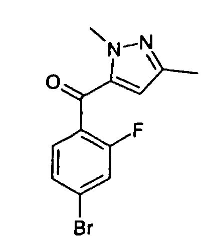

A mixture of (4-bromo-2-fluorophenyl)(iodo)zinc in tetrahydrofuran (0.5M, 3mL), 1,3-dimethyl-1H-pyrazole-5-carbonyl chloride (238mg) and tetrakis(triphenylphosphine)palladium(0) (87g) in tetrahydrofuran (3mL) was stirred at room temperature under nitrogen for 1 h. Aqueous ammonium chloride (1M, 5mL) was added to the reaction mixture which was then extracted with ethyl acetate. The organic layer was washed with water, dried using a hydrophobic filter tube and concentrated under vacuum. The residue was purified by chromatography on a silica column, eluting with a cyclohexane/ethyl acetate gradient, to give the title compound (324mg).
LC-MS: Rt 3.16min.

### Intermediate 37: 1,1-Dimethylethyl 2-[(4-bromo-2-fluorophenyl)(1,3-dimethyl-1H-pyrazol-5-yl)methylidene]hydrazinecarboxylate

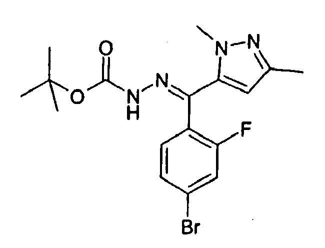

A mixture of (4-bromo-2-fluorophenyl)(1,3-dimethyl-1H-pyrazol-5-yl)methanone (Intermediate 36, 324mg) and 1,1-dimethylethyl hydrazinecarboxylate (216mg) in acetic acid (1mL) and methanol (5mL) was stirred at reflux under nitrogen for 20h. The solvent was removed under vacuum and the residue was partitioned between ethyl acetate and aqueous sodium hydrogen carbonate (1 M). The organic phase was dried using a hydrophobic filter tube and concentrated under vacuum. The residue was purified by chromatography on a silica column, eluting with a cyclohexane/ethyl acetate gradient, to give the title compound (110mg).
LC-MS: Rt 3.32min.

### Intermediate 38: 6-Bromo-3-(1,3-dimethyl-1H-pyrazol-5-yl)-1H-indazole

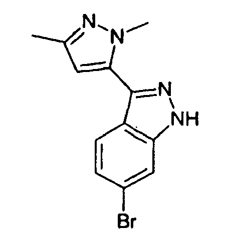

A mixture of 1,1-dimethylethyl 2-[(4-bromo-2-fluorophenyl)(1,3-dimethyl-1H-pyrazol-5-yl)methylidene]hydrazinecarboxylate (Intermediate 37, 103mg) and 1,8-diazabicyclo[5.4.0]undec-7-ene (37µL) in tetrahydrofuran (2.5mL) in a sealed vial was heated at 150°C for 25min in a microwave oven. The reaction mixture was partitioned between ethyl acetate and water. The organic layer was dried using a hydrophobic filter tube and concentrated under vacuum. The residue was purified by chromatography on a silica column, eluting with a cyclohexane/ethyl acetate gradient, to give the title compound as a colourless glass (34mg).
LC-MS: Rt 3.26min.

### Intermediate 39: 3-Fluoro-5-iodo-4-methyl-N-(1-methyl-1H-pyrazol-5-yl)benzamide

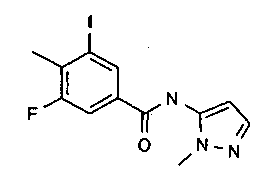

3-Fluoro-5-iodo-4-methylbenzoic acid (Intermediate 69, 6.27g) in thionyl chloride (10ml) was heated at 110°C for 2.5h. The reaction was left to cool to room temperature overnight and the excess thionyl chloride was removed under vacuum. A portion (2.18g) of the crude acid chloride in dichloromethane (10ml) was treated with potassium carbonate (1.7g) followed by a solution of 5-amino-1-methylpyrazole (0.94g) in dichloromethane (10ml). The mixture was stirred overnight then concentrated under vacuum. The residue was washed with water then triturated triturated with cyclohexane to give the title compound as a cream solid (1.44g).
LC-MS: Rt 3.08min, MH+ 360.

### Intermediate 40: 3-Fluoro-4-methyl-N-(1-methyl-1H-pyrazol-5-yl)-5-(4,4,5,5-tetramethyl-1,3,2-dioxaborolan-2-yl)benzamide

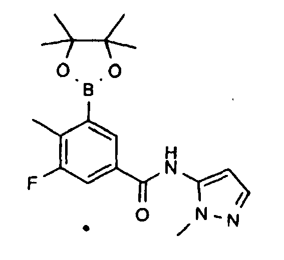

3-Fluoro-5-iodo-4-methyl-*N*-(1-methyl-1*H*-pyrazol-5-yl)benzamide (Intermediate 23, 1.44g) bispinnacolatodiborane (3.05g) Pd(dppf)Cl2 (59mg) and potassium acetate (1.19g) were combined in DMF (34ml), divided between 2 vials then heated by microwave in sealed vessels at 150°C for 15min. The mixtures were combined, absorbed onto silica and applied to silica columns (2 x 100g). The columns were eluted with an ethyl acetate/cyclohexane gradient to give the title compound as an off-white solid (217mg).
LC-MS: Rt 3.25min, MH+ 360.

### Intermediate 41: 3-Fluoro-5-iodo-4-methyl-N-1H-pyrazol-5-ylbenzamide

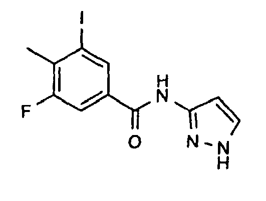

N,N-Diisopropylethlamine (3.8ml) and HATU (3.32g) were added to a solution of 3-fluoro-5-iodo-4-methylbenzoic acid (Intermediate 69, 2.07g) in DMF (80ml) and the reaction mixture was stirred for 10min at room temperature. 3-Aminopyrazole (0.9ml) was added and stirring was continued at room temperature overnight. The reaction mixture was absorbed onto silica, applied to a silica column (100g) and eluted with an ethyl acetate/cyclohexane gradient to give the title compound as a pale yellow solid (555mg).
LC-MS: Rt 3.07min, MH+ 346.

### Intermediate 42: 3-Fluoro-4-methyl-N-1H-pyrazol-5-yl-5-(4,4,5,5-tetramethyl-1,3,2-dioxaborolan-2-yl)benzamide

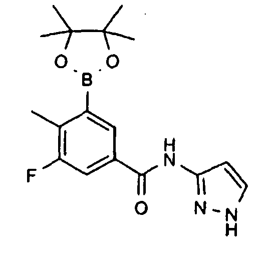

A mixture of 3-fluoro-5-iodo-4-methyl-N-1H-pyrazol-5-ylbenzamide (500mg), bispinnacolatodiborane (1.10g) Pd(dppf)Cl₂ (27.4mg) and potassium acetate (462mg) in DMF (13ml) in a sealed vessel was at 150°C in a microwave oven for 15min. The reaction mixture was absorbed onto silica and applied to a silica column (100g). Elution with an ethyl acetate/cyclohexane gradient gave the title compound as a brown foam (288mg).
LC-MS: Rt 3.21 min, MH+ 346.

### Intermediate 43: (4-Bromo-2-fluorophenyl)[6-(4-morpholinyl)-3-pyridinyl]methanone

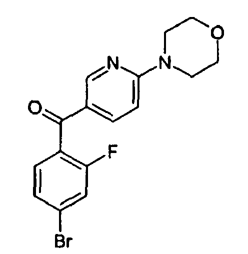

A mixture of (4-bromo-2-fluorophenyl)(iodo)zinc in tetrahydrofuran (0.5M, 3mL), 6-(4-morpholinyl)-3-pyridinecarbonyl chloride (340mg) and tetrakis(triphenylphosphine)palladium(0) (87g) in tetrahydrofuran (3mL) was stirred at room temperature under nitrogen for 1 h. Aqueous ammonium chloride (1M, 5mL) was added and the mixture w as extracted with ethyl acetate. The organic phase was washed with water, dried using a hydrophobic filter tube and concentrated under vacuum. The residue was purified by chromatography on a silica column, eluting with a cyclohexane/ethyl acetate gradient, to give the title compound (282mg).
LC-MS: Rt 3.16min.

### Intermediate 44: 1,1-Dimethylethyl 2-{(4-bromo-2-fluorophenyl)[6-(4-morpholinyl)-3-pyridinyl]methylidene}hydrazinecarboxylate

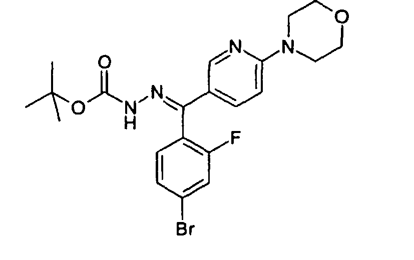

A mixture of (4-bromo-2-fluorophenyl)[6-(4-morpholinyl)-3-pyridinyl]methanone (Intermediate 43, 282mg) and 1,1-dimethylethyl hydrazinecarboxylate (152mg) in acetic acid (1mL) and methanol (5mL) was stirred at reflux under nitrogen for 20h. The solvent was removed under vacuum and the residue was partitioned between ethyl acetate and aqueous sodium hydrogen carbonate (1M). The organic phase was dried using a hydrophobic filter tube and concentrated under vacuum. The residue was purified by chromatography on a silica column, eluting with a cyclohexane/ethyl acetate gradient to give the title compound (235mg).
LC-MS: Rt 3.28min.

### Intermediate 45: 6-Bromo-3-[6-(4-morpholinyl)-3-pyridinyl]-1H-indazole

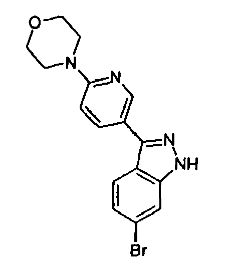

A mixture of 1,1-dimethylethyl 2-{(4-bromo-2-fluorophenyl)[6-(4-morpholinyl)-3-pyridinyl]methylidene}hydrazinecarboxylate (Intermediate 44, 235mg) and 1,8-diazabicyclo[5.4.0]undec-7-ene (73µL) in tetrahydrofuran (2.5mL) in a sealed vial was heated at 150°C for 25min in a microwave oven. The reaction mixture was partitioned between ethyl acetate and water and the organic layer was dried using a hydrophobic filter tube and concentrated under vacuum. The residue was purified by chromatography on a silica column, eluting with a cyclohexane/ethyl acetate gradient, to give the title compound as a yellow solid (70mg).
LC-MS: Rt 3.28min.

### Intermediate 46: (4-Bromo-2-fluorophenyl)(2-pyrimidinyl)methanone

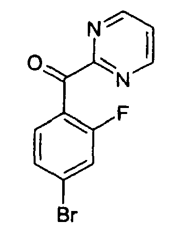

2-Pyrimidinecarboxylic acid (350mg) in thionyl chloride (3mL) was stirred at 110°C under nitrogen for 1.5h. The solvent was removed under vacuum to give the 2-pyrimidinecarbonyl chloride as a grey solid (403mg). (4-Bromo-2-fluorophenyl)(iodo)zinc in tetrahydrofuran (0.5M, 5.65mL) was added slowly to a stirred mixture of the acid chloride (403mg) and tetrakis(triphenylphosphine)palladium(0) (163mg) in tetrahydrofuran (3mL) at room temperature under nitrogen. The reaction mixture was stirred at room temperature under nitrogen for 1h and aqueous ammonium chloride (1M, 6mL) was added. The mixture was absorbed onto silica and purified by chromatography on a silica column, eluting with a cyclohexane/ethyl acetate gradient to give the title compound (257mg).
LC-MS: Rt 2.95min.

### Intermediate 47: 1,1-Dimethylethyl 2-[(4-bromo-2-fluorophenyl)(2-pyrimidinyl)methylidene]hydrazinecarboxylate

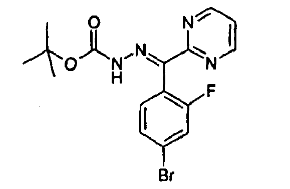

A mixture of (4-bromo-2-fluorophenyl)(2-pyrimidinyl)methanone (IIntermediate 46, 257mg) and 1,1-dimethylethyl hydrazinecarboxylate (241mg) in acetic acid (1mL) and methanol (5mL) was stirred at reflux under nitrogen for 20h. The solvent was removed under vacuum and the residue was partitioned between ethyl acetate and water. The organic phase was washed with aqueous sodium hydrogen carbonate (1 M), dried using a hydrophobic filter tube and concentrated under vacuum. The residue was purified by chromatography on a silica column, eluting with a cyclohexane/ethyl acetate gradient to give the title compound as a white solid (73mg).
LC-MS: Rt 3.08min.

### Intermediate 48: 6-Bromo-3-(2-pyrimidinyl)-1H-indazole

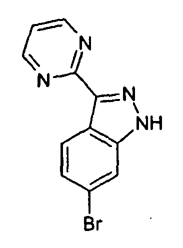

A mixture of 1,1-dimethylethyl 2-[(4-bromo-2-fluorophenyl)(2-pyrimidinyl)methylidene]hydrazinecarboxylate (Intermediate 47, 73mg) and 1,8-diazabicyclo[5.4.0]undec-7-ene (28uL) in tetrahydrofuran (2.5mL) in a sealed vial was heated at 150°C for 25min in a microwave oven. The reaction mixture was partitioned between ethyl acetate and water, the organic layer was dried using a hydrophobic filter tube and the solvent was removed under vacuum. The residue was purified by chromatography on a silica column, eluting with a cyclohexane/ethyl acetate gradient to give the title compound as a white solid (17mg).
LC-MS: Rt 3.17min.

### Intermediate 49: (4-Bromo-2-fluorophenyl)(5-pyrimidinyl)methanone

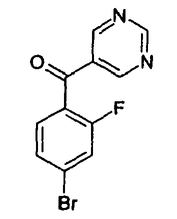

5-Pyrimidinecarboxylic acid (500mg) in thionyl chloride (5mL) was stirred at 110°C under nitrogen for 1 h. The solvent was evaporated under vacuum to give 5-pyrimidinecarbonyl chloride as a brown oil (505mg). (4-Bromo-2-fluorophenyl)(iodo)zinc in tetrahydrofuran (0.5M, 7.0mL) was added slowly to a stirred mixture of the acid chloride (500mg) and tetrakis(triphenylphosphine)palladium(0) (203mg) in tetrahydrofuran (3mL) at room temperature under nitrogen then stirred at room temperature for 1 h. Aqueous ammonium chloride (1M, 5 mL) was added and the mixture was absorbed onto silica and purified by chromatography on a silica column eluting with a cyclohexane/ethyl acetate gradient to give the title compound (712mg).
LC-MS: Rt 2.65min.

### Intermediate 50: 1,1-Dimethylethyl 2-[(4-bromo-2-fluorophenyl)(5-pyrimidinyl)methylidene]hydrazinecarboxylate

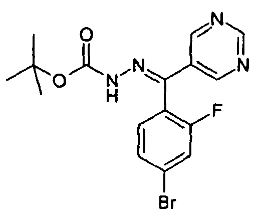

A mixture of (4-bromo-2-fluorophenyl)(5-pyrimidinyl)methanone (Intermediate 49, 712mg) and 1,1-dimethylethyl hydrazinecarboxylate (502mg) in acetic acid (1mL) and methanol (10mL) was stirred at reflux under nitrogen for 30h. More 1,1-dimethylethyl hydrazinecarboxylate (502mg) was added and the reaction mixture was stirred at reflux for a further 14h. The solvent was removed under vacuum and the residue was partitioned between ethyl acetate and sodium bicarbonate (1 M). The organic phase was dried using a hydrophobic filter tube and concentrated under vacuum. The residue was purified by chromatography on a silica column, eluting with a cyclohexane/ethyl acetate gradient, to give the title compound (531mg).
LC-MS: Rt 3.08min.

### Intermediate 51: 6-Bromo-3-(5-pyrimidinyl)-1H-indazole

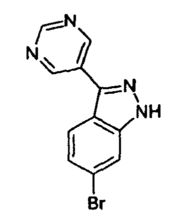

A mixture of 1,1-dimethylethyl 2-[(4-bromo-2-fluorophenyl)(5-pyrimidinyl)methylidene]hydrazinecarboxylate (Intermediate 50, 531mg) and 1,8-diazabicyclo[5.4.0]undec-7-ene (200µL) in tetrahydrofuran (4mL) in a sealed vessel was heated at 150°C for 25min in a microwave oven. The reaction mixture was partitioned between ethyl acetate and water and the organic layer was dried using a hydrophobic filter tube and concentrated under vacuum. The residue was purified by chromatography on a silica column eluting with a cyclohexane/ethyl acetate gradient, to give the title compound as a white solid (120mg).
LC-MS: Rt 2.85min.

### Intermediate 52: (4-bromo-2-fluorophenyl)(2-pyrazinyl)methanone

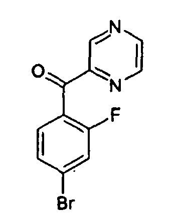

2-Pyrazinecarboxylic acid (1.5g) in thionyl chloride (10mL) was stirred at 110°C under nitrogen for 2h. The solvent was evaporated under vacuum to give 2-pyrazinecarbonyl chloride as a dark purple solid(1.5g). (4-Bromo-2-fluorophenyl)(iodo)zinc in tetrahydrofuran (0.5M, 7.02mL) was added slowly to a stirred mixture of the acid chloride (1g) and tetrakis(triphenylphosphine)palladium(0) (406mg) in tetrahydrofuran (5mL) at room temperature under nitrogen then stirred for 2h. Aqueous ammonium chloride (1M, 10mL) was added and the reaction mixture was partitioned between ethyl acetate and water. The organic phase was dried using a hydrophobic filter tube and concentrated under vacuum. The residue was purified by chromatography on a silica column eluting with a cyclohexane:ethyl acetate gradient to give the title compound as a yellow solid (215mg).
LC-MS: Rt 2.83min.

### Intermediate 53: 1,1-Dimethylethyl 2-[(4-bromo-2-fluorophenyl)(2-pyrazinyl)methylidene]hydrazinecarboxylate

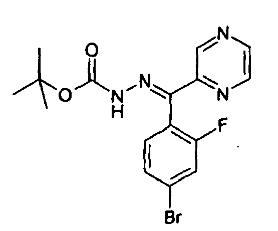

A mixture of (4-bromo-2-fluorophenyl)(2-pyrazinyl)methanone (Intermediate 52, 215mg) and 1,1-dimethylethyl hydrazinecarboxylate (152mg) in acetic acid (131µL) and methanol (10mL) was stirred at reflux under nitrogen for 20h. The solvent was removed under vacuum and the residue was partitioned between chloroform:ethyl acetate (1:1) and water. The organic phase was dried using a hydrophobic filter tube and concentrated under vacuum to give impure title compound as a brown oil (352mg).
LC-MS: Rt 3.2min.

### Intermediate 54: 6-bromo-3-(2-pyrazinyl)-1H-indazole

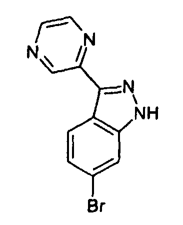

A mixture of impure 1,1-dimethylethyl 2-[(4-bromo-2-fluorophenyl)(2-pyrazinyl)methylidene]hydrazinecarboxylate (Intermediate 52, 352mg) and 1,8-diazabicyclo[5.4.0]undec-7-ene (133uL) in tetrahydrofuran (5mL) in a sealed vial was heated at 150°C for 30min in a microwave oven. The reaction mixture was partitioned between ethyl acetate and water and the organic phase was dried using a hydrophobic filter tube and concentrated under vacuum. The residue was purified by chromatography on a silica column, eluting with a cyclohexane/ethyl acetate gradient, to give the title compound as a white solid (115mg).
LC-MS: Rt 3.33min.

### Intermediate 55: 3-(3-Bromo-1H-indazol-6-yl)-N-ethyl-5-fluoro-4-methylbenzamide

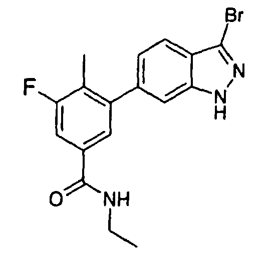

N-Bromosuccinimide (0.992g) was added to a solution of *N*-ethyl-3-fluoro-5-(1*H-*indazol-6-yl)-4-methylbenzamide (Intermediate 27, 1.4g) in THF (15ml) and stirred at 60°C under nitrogen. The reaction mixture was allowed to cool to room temperature and the solvent was evaporated under vacuum. The crude material was purified by column chromatography on silica eluting with a cyclohexane/ethyl acetate gradient to give the title compound as a yellow powder (0.92g).
LC-MS: Rt 3.33min, MH⁺ 376.

### Intermediate 56: Methyl 6-[(4-bromo-2-fluorophenyl)carbonyl]-3-pyridinecarboxylate

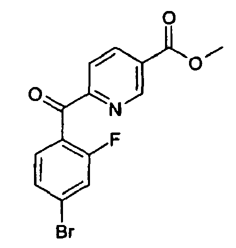

5-[(Methyloxy)carbonyl]-2-pyridinecarboxylic acid (1g) in thionyl chloride (10mL) was stirred at 110°C under nitrogen for 1 h. Excess thionyl chloride was removed under vacuum to give the methyl 6-(chlorocarbonyl)-3-pyridinecarboxylate as a white solid (1.05g). (4-Bromo-2-fluorophenyl)(iodo)zinc in tetrahydrofuran (0.5M, 10mL) was added slowly to a stirred mixture of the acid chloride (1g) and tetrakis(triphenylphosphine)palladium(0) (290mg) in tetrahydrofuran (5mL) at room temperature under nitrogen, stirred for 5h then treated with aqueous ammonium chloride (1M, 10mL). The mixture was absorbed onto silica and purified by chromatography on a silica column eluting with a cyclohexane/ethyl acetate gradient to give the title compound (635mg).
LC-MS: Rt 3.37min.

### Intermediate 57: 6-({5'-[(Ethylamino)carbonyl]-3-fluoro-2'-methyl-4-biphenylyl}carbonyl)-3-pyridinecarboxylic acid

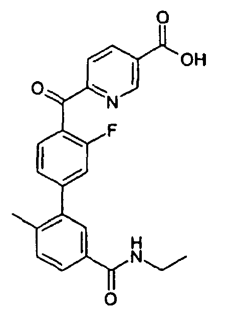

A mixture of 6-[(4-bromo-2-fluorophenyl)carbonyl]-3-pyridinecarboxylate (Intermediate 56, 500mg), N-ethyl-4-methyl-5-(4,4,5,5-tetramethyl-1,3,2-dioxaborolan-2-yl)benzamide (Intermediate 18, 428mg), aqueous sodium hydrogen carbonate (2.96mL) and tetrakis(triphenylphosphine)palladium(0) (34mg) in isopropanol (15ml) in a sealed vial was stirred at 150°C for 20 min in a microwave oven. Saturated aqueous citric acid (5mL) was added, the solvent was removed under vacuum and the residue was purified by chromatography on a silica column eluting with a cyclohexane/ethyl acetate gradient to give the title compound (460mg).
LC-MS: Rt 3.27min.

### Intermediate 58: N-Ethyl-6-({5'-[(ethylamlno)carbonyl]-3-fluoro-2'-methyl-4-biphenylyl}carbonyl)-3-pyridinecarboxamide

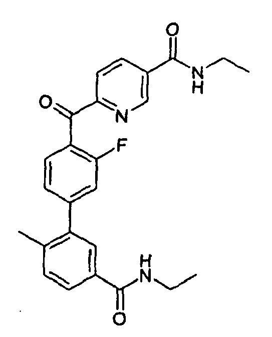

Ethylamine 2M in tetrahydrofuran (276µL) was added to a mixture of 6-({5'-[(ethylamino)carbonyl]-3-fluoro-2'-methyl-4-biphenylyl}carbonyl)-3-pyridinecarboxylic acid (Intermediate 57, 150mg), N-[3-(dimethylamino)propyl]-N'-ethylcarbodiimide hydrochloride (106mg), and 3H-[1,2,3]triazolo[4,5-b]pyridin-3-ol (5mg) in chloroform (20mL) at room temperature under nitrogen then stirred for 2h. The mixture was washed with water (20mL) dried dried using a hydrophobic filter tube and concentrated under vacuum. The residue was purified by chromatography on a silica column eluting with a cyclohexane/ethyl acetate gradient to give the title compound (98mg).
LC-MS: Rt 3.01 min.

### Intermediate 59: 1,1-Dimethylethyl 2-({5'-[(ethylamino)carbonyl]-3-fluoro-2'-methyl-4-biphenylyl}{5-[(ethylamino)carbonyl]-2-pyridinyl}methylidene)hydrazinecarboxylate

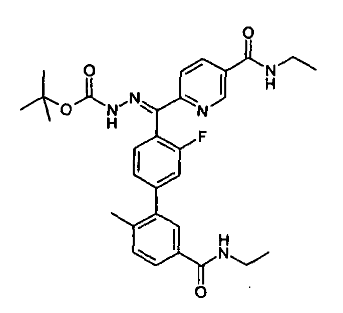

A mixture of N-ethyl-6-({5'-[(ethylamino)carbonyl]-3-fluoro-2'-methyl-4-biphenylyl}carbonyl)-3-pyridinecarboxamide (Intermediate 58, 98mg), 1,1-dimethylethyl hydrazinecarboxylate (45mg) and acetic acid (39uL) in methanol (5mL) was stirred at reflux under nitrogen for 60h. The solvent was removed under vacuum and the residue was partitioned between ethyl acetate and water. The organic phase was dried using a hydrophobic filter tube and concentrated under vacuum to give impure title compound as a yellow oil (198mg).
LC-MS: Rt 3.17min.

### Intermediate 60: 3-Fluoro-5-[3-(4-fluorophenyl)-1H-indazol-6-yl]-4-methylbenzoic acid

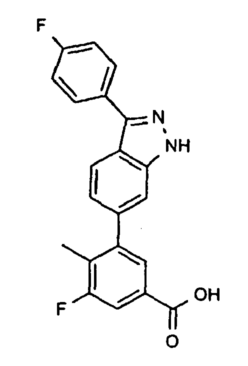

A mixture of methyl 3-fluoro-4-methyl-5-(4,4,5,5-tetramethyl-1,3,2-dioxaborolan-2-yl)benzoate (Intermediate 8, 709mg), 6-bromo-3-(4-fluorophenyl)-1*H*-indazole (Intermediate 20, 707mg), aqueous sodium hydrogen carbonate (1 M, 7.2ml) and tetrakis(triphenylphosphine)palladium (61mg) in propan-2-ol (10ml). in a sealed vessel was heated in a microwave oven at 150°C for 15min. Two further portions of aqueous sodium hydrogen carbonate (1M, 2.4ml) were added over 3d and the mixture was stirred at room temperature. The reaction was then heated at 50°C for 2h, the solvent was removed under vacuum and the residue was partitioned between ethyl acetate and water. The organic phase was concentrated under vacuum, methanol (15ml) and aqueous sodium hydroxide (2M, 10ml) were added and the mixture was stirred at room temperature overnight. The solvent was evaporated and the residue was partitioned between ethyl acetate and sodium hydroxide (2M) then filtered. The aqueous phase was acidified with hydrochloric acid (2M) and the precipitate collected by filtration and dried to give the title compound as a yellow solid (166mg).
LC-MS: Rt 3.92min, MH+ 365.

### Intermediate 61: N-(1,4-Dimethyl-1H-pyrazol-5-yl)-4-methyl-3-(4,4,5,5-tetramethyl-1,3,2-dioxaborolan-2-yl)benzamide

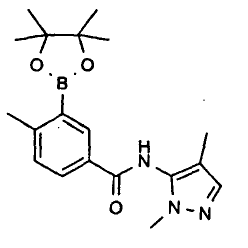

4-Methyl-3-(4,4,5,5-tetramethyl-1,3,2-dioxaborolan-2-yl)benzoic acid (507mg) was dissolved in DMF (5ml). An aliquot (1ml) was treated with DIPEA (0.2ml) and HATU (181mg) the mixture was stirred for 10min at room temperature. 1,4-Dimethyl-1*H*-pyrazol-5-amine (72mg) was added and the mixture was stirred at room temperature overnight. The solvent was evaporated under a stream of nitrogen and the residue was absorbed onto silica and purified by chromatography on a silica column eluting with cyclohexane/ethyl acetate (1:1 to give the title compound as a white solid (121mg).
LCMS: Rt 3.26min, MH+ 356.

### Intermediate 62: N-(3,5-Dimethyl-4-isoxazolyl)-4-methyl-3-(4,4,5,5-tetramethyl-1,3,2-dioxaborolan-2-yl)benzamide

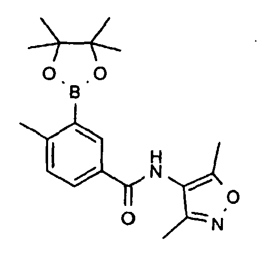

4-Methyl-3-(4,4,5,5-tetramethyl-1,3,2-dioxaborolan-2-yl)benzoic acid (507mg) was dissolved in DMF (5ml). An aliquot (1ml) was treated with DIPEA (0.2ml) and HATU (180mg) and the mixture was stirred for 10min at room temperature. 3,5-Dimethyl-4-isoxazolamine (65mg) was added and the mixture was stirred at room temperature overnight. The DMF was removed under a stream of nitrogen and the residue absorbed onto silica and purified by chromatography on a silica column eluting with cyclohexane/ethyl acetate (7:3). The resultant was tritureated with ether to give the title compound as a white solid (41 mg).
LCMS: Rt 3.31min, MH+ 357.

### Intermediate 63: 1,1-Dimethylethyl 3-fluoro-5-iodo-4-methylbenzoate

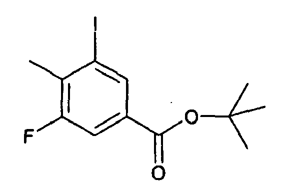

Carbonyl diimidazole (12.9g) was added to a stirred solution of 3-fluoro-5-iodo-4-methylbenzoic acid (Intermediate 69, 15g) in DMF (200ml) at 40°C. After 40 min tert-butanol (8.0ml) and 1,8-diazobicyclo[5,4,0]undec-7-ene(8.1ml) were added and the solution was stirred for 18h at 40°C. The reaction was poured into water (1000ml) a nd the precipitate collected by filtration to give the title compound as a pale brown solid (15.4g).
LC-MS: Rt 4.08min.

### Intermediate 64: 6-(5,5-Dimethyl-1,3,2-dioxaborinan-2-yl)-1H-indazole

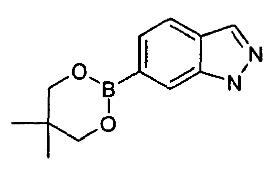

A stirred mixture of 6-bromoindazole (5g), bisneopentyl glycolato-diboron (6.9g), potassium acetate (3.0g) and Pd(dppf) (0.5g) in DMSO (500ml) was heated at 80°C for 5.5h under nitrogen. The mixture was poured into water (1500ml) and extracted into ethyl acetate (4x200ml). The combined extracts were washed with water (500ml), then concentrated under vacuum The residue was purified by column chromatography on silica eluting with an ethyl acetate/cyclohexane gradient (10:90 to 100:0) to give the title compound as a brown solid.(8.4g).
LC-MS: Rt 1.92 min.

### Intermediate 65: 1,1-Dimethylethyl 3-fluoro-5-(1H-indazol-6-yl)-4-methylbenzoate

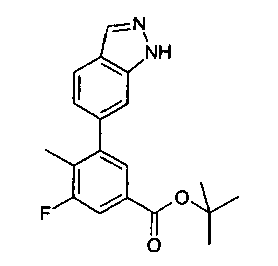

A mixture of 1,1-dimethylethyl 3-fluoro-5-iodo-4-methylbenzoate (Intermediate 63, 3.42g), 6-(5,5-Dimethyl-1,3,2-dioxaborinan-2-yl)-1*H*-indazole (Intermediate 64, 2.81g), aqueous sodium hydrogen carbonate (1M, 7.5ml) and tetrakis(triphenylphosphine)palladium(0) (120mg) in isopropanol (30ml) was stirred at relux under nitrogen for 20h. Water and ethyl acetate were added, the phases were separated and the aqueous layer was re-extracted with ethyl acetate. The solvent was evaporated and the residue was purified by column chromatography on silica eluting with a cyclohexane/ethyl acetate gradient to give the title compound as a yellow solid (1.6g).
LC-MS: Rt 3.78min.

### Intermediate 66: 1,1-Dimethylethyl 3-fluoro-5-(3-iodo-1H-indazol-6-yl)-4-methylbenzoate

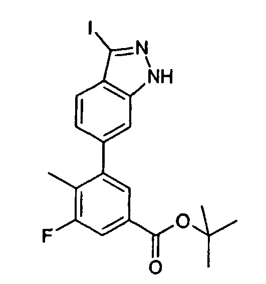

Iodine (1g) was added to a stirred solution of 1,1-dimethylethyl 3-fluoro-5-(1H-indazol-6-yl)-4-methylbenzoate (Intermediate 65, 1.1g) in 1,4-dioxan (15ml) and 2M sodium hydroxide solution (5ml). After 2h the mixture was treated with aqueous sodium bisulphite (10%, 30ml) and aqueous citric acid (10%, 30ml). The mixture was extracted with dichloromethane (3x25ml) and the combined organic extracts were washed with water (30ml), dried (Na₂SO₄) and concentrated under vacuum to give the title compound as a pale orange foam (1.4g).
LC-MS: Rt 4.22min, MH+453.

### Intermediate 67: 1,1-Dimethylethyl 3-fluoro-5-[3-iodo-1-({[2-(trimethylsilyl)ethyl]oxy}methyl)-1H-indazol-6-yl]-4-methylbenzoate

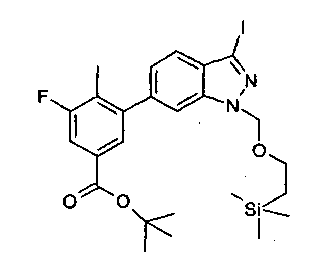

2-(Trimethylsilyl)ethoxymethyl chloride (0.67g) was added to an ice-bath cooled mixture of 1,1-dimethylethyl 3-fluoro-5-(3-iodo-1H-indazol-6-yl)-4-methylbenzoate (Intermediate 66, 1.5g), tetra-butylammonium bromide (0.2g) and aqueous potassium hydroxide (50%, 15ml) in dichloromethane (20ml). The mixture was stirred for 10min, poured into water (50ml) and extracted into dichloromethane (3x30ml). The combined extracts were washed with water (50ml), dried (Na₂SO₄) and concentrated under vacuum. The residual oil was purified by column chromatography on silica eluting with ether/cyclohexane (1:9) to give the title compound as an orange oil (1.9g).
LC-MS: Rt 4.46min MH+ 583.

### Intermediate 68: 1,1-Dimethylethyl 3-[3-(1,2-dimethyl-1H-imidazol-5-yl)-1-({[2-(trimethylsilyl)ethyl]oxy}methyl)-1H-indazol-6-yl]-5-fluoro-4-methylbenzoate

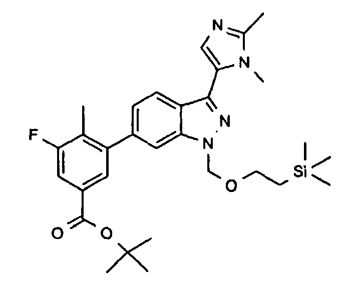

A mixture of 1,1-dimethylethyl 3-fluoro-5-[3-iodo-1-({[2-(trimethylsilyl)ethyl]oxy}methyl)-1H-indazol-6-yl]-4-methylbenzoate (Intermediate 67, 0.1g), 1,2-dimethyl-5-(tributylstannanyl)-1H-imidazole (0.15g) and tetrakis(triphenylphosphine)palladium(0) (0.03g) in toluene (1ml) was heated at 120°C for 30min in a microwave oven. The solvent was evaporated and the residue was purified by preparative HPLC to give the title compound as a pale green oil (0.09g).
LC-MS: Rt 3.47min, MH+ 551.

### Intermediate 69: 3-Fluoro-5-iodo-4-methylbenzoic acid

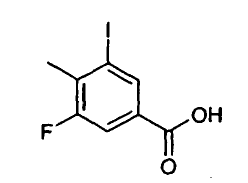

A solution of 3-fluoro-4-methylbenzoic acid (149.7g) in trifluoromethanesulphonic acid (1050ml) at -22°C under nitrogen was treated portionwise over 1.25h with iodosuccinimide (203.5g). The mixture was stirred at -20°C for and further portions of iodosuccinimide were added after 2.5h (46.5g) and 20.5h (30g). The mixture was stirred at -20°C for a further 24h then added slowly to a mixture of aqueous sodium thiosulphate (10%, 1.5L) and ice (3kg). The resultant precipitate was collected by filtration and stirred with ethyl acetate (5L) and aqueous sodium thiosulphate (10%, 1.5L). The organic phase was dried (MgSO₄) and concentrated to -1.5L then left overnight. The precipitate was collected by filtration and further material was obtained through concentration of the filtrate to give the title compound as a white solid (133.9g).
LC-MS: Rt 3.60, MH+ 281.

### Example 1: N-Cyclopropyl-3-fluoro-4-methyl-5-(1-phenyl-1H-indazol-5-yl)benzamide

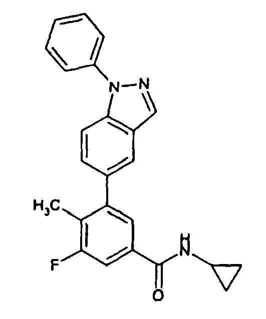

A mixture of 5-bromo-1-phenyl-1*H*-indazole (Intermediate 1, 34.6mg), {5-[(cyclopropylamino)carbonyl]-3-fluoro-2-methylphenyl}boronic acid (30mg), tetrakis(triphenylphophine) palladium (3mg) and aqueous sodium hydrogen carbonate (1M, 0.63ml) in isopropanol (2ml) in a sealed vessel was heated in a microwave oven at 150°C for 10min (70W). The reaction mixture was filtered, the solvents were removed under vacuum and the residue was purified by preparative HPLC to give the title compound (32mg).
LC-MS: Rt 3.54min, MH+ 386.

### Example 2: N-Cyclopropyl-3-fluoro-5-[1-(4-fluorophenyl)-1H-indazol-5-yl]-4-methylbenzamide

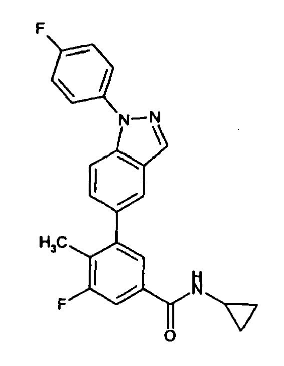

The procedure for Example 1 was followed using 5-bromo-1-(4-fluorophenyl)-1H-indazole (Intermediate 2, 36.6mg), {5-[(cyclopropylamino)carbonyl]-3-fluoro-2-methylphenyl}boronic acid (30mg), tetrakis(triphenylphophine) palladium (3mg) and aqueous sodium hydrogen carbonate (1M, 0.63ml) in isopropanol (2ml) to give the title compound (30.7mg).
LC-MS: Rt 3.56min, MH+ 404.

### Example 3: N-Cyclopropyl-3-fluoro-5-[1-(4-fluoro-2-methylphenyl)-1H-indazol-5-yl]-4-methylbenzamide

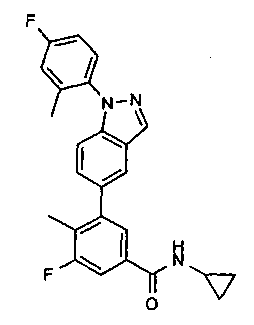

A mixture of *N*-cyclopropyl-3-fluoro-5-(1*H*-indazol-5-yl)-4-methylbenzamide (Intermediate 5, 31mg), (4-fluoro-2-methylphenyl)boronic acid (31mg), anhydrous copper (II) acetate (27mg), pyridine (16.4µl) and 4Å powdered molecular sieves (75mg) in dichloromethane (2ml) was stirred at room temperature for 24h. Further dichloromethane (1ml) was added and after 24h the reaction mixture was diluted with chloroform (3ml) and water (3ml). The organic phase was separated using a hydrophobic filter tube and the solvent was evaporated. The residue was purified by preparative HPLC to give the title compound as a yellow gum (10.5mg).
LC-MS: Rt 3.63min, MH+ 418.

### Example 4: N-Cyclopropyl-3-fluoro-5-{1-[(4-fluorophenyl)methyl]-1H-indazol-5-yl}-4-methylbenzamide

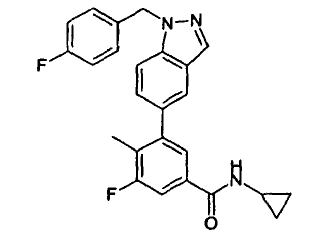

A solution of *N*-cyclopropyl-3-fluoro-5-(1*H*-indazol-5-yl)-4-methylbenzamide (Intermediate 5, 31mg) and sodium hydride (60% dispersion in mineral oil, 4mg) in DMF (1.6ml) was stirred at room temperature under nitrogen. After 1 hour, 1-(bromomethyl)-4-fluorobenzene (15µl) was added and the resulting mixture was allowed to stir under nitrogen at room temperature. After 1h, ammonia (aqueous 0.88, 200µl) was added and the resulting mixture was allowed to stir under nitrogen at room temperature. After 1 h, the mixture was concentrated. The residue was purified by preparative HPLC to give the title compound (7mg).
LC-MS: Rt 3.49, MH⁺ 418.

### Example 5: N-Cyclopropyl-3-fluoro-4-methyl-5-(1-{[4-(trifluoromethyl)phenyl]methyl}-1H-indazol-5-yl)benzamide

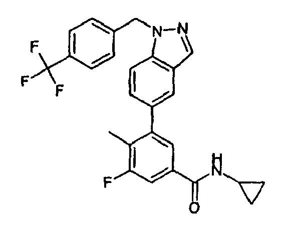

The procedure for Example 4 was followed using *N*-cyclopropyl-3-fluoro-5-(1*H-*indazol-5-yl)-4-methylbenzamide (Intermediate 5, 31 mg), sodium hydride (60% dispersion in mineral oil, 4mg), 1-(bromomethyl)-4-(trifluoromethyl)benzene (19µl) and DMF (1.6ml) to give the title compound (6mg).
LC-MS: Rt 3.65, MH⁺ 468.

### Example 6: N-Cyclopropyl-3-fluoro-4-methyl-5-[1-(phenylmethyl)-1H-indazol-5-yl]benzamide

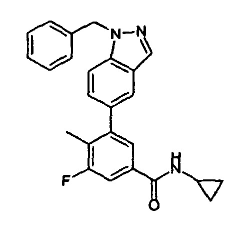

The procedure for Example 4 was followed using *N*-cyclopropyl-3-fluoro-5-(1*H-*indazol-5-yl)-4-methylbenzamide (Intermediate 5, 31 mg), sodium hydride (60% dispersion in mineral oil, 4mg), (bromomethyl)benzene (14µl) and DMF (1.6ml) to give the title compound (9mg).
LC-MS: Rt 3.48, MH⁺ 400.

### Example 7: N-Cyclopropyl-3-fluoro-4-methyl-5-{1-[(5-methyl-3-isoxazolyl)methyl]-1H-indazol-5-yl}benzamide

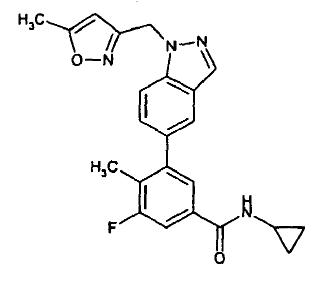

Sodium hydride (60% in mineral oil, 12mg) was added to *N*-cyclopropyl-3-fluoro-5-(1*H*-indazol-5-yl)-4-methylbenzamide (Intermediate 5, 45mg) in DMF (5ml) and the mixture was stirred at room temperature for 1 h. 3-(Bromomethyl)-5-methylisoxazole (28.2mg) was added and stirring was continued for 18h. The mixture was diluted with chloroform/ethyl acetate (1:1, 5ml) a nd washed with water (2x5ml). The organic phase was concentrated under vacuum and the residue was purified on a silica column eluting with chloroform/ethyl acetate (9:1) to give the title compound as a colourless glass (10mg).
LC-MS: Rt 3.14min, MH+ 405.

### Example 8: N-Cyclopropyl-3-fluoro-4-methyl-5-[1-(2-pyridinylmethyl)-1H-indazol-5-yl]benzamide

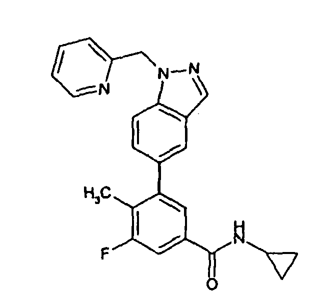

The procedure for Example 7 was followed using *N*-cyclopropyl-3-fluoro-5-(1*H-*indazol-5-yl)-4-methylbenzamide (Intermediate 5, 45mg), sodium hydride (60% in mineral oil, 18mg), 2-(bromomethyl)pyridine (40mg) and DMF (5ml) to give the title compound as a colourless glass (10mg).
LC-MS: Rt 3.07min, MH+ 401.

### Example 9: N-Cyclopropyl-3-[1-(4-fluorophenyl)-1H-indazol-5-yl]-4-methylbenzamide

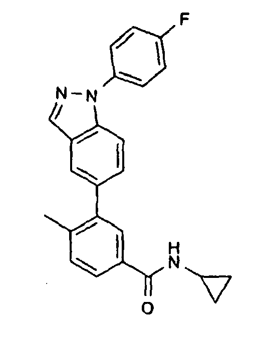

A mixture of 5-bromo-1-(4-fluorophenyl)-1*H*-indazole (Intermediate 2, 29mg), *N-*cyclopropyl-4-methyl-3-(4,4,5,5-tetramethyl-1,3,2-dioxaborolan-2-yl)benzamide (30mg), tetrakis(triphenylphoshine)palladium (0) (6mg) and 1M aqueous sodium bicarbonate (1ml) in 2-propanol (1 ml) was heated at 150°C for 15mins in a microwave oven. The reaction mixture was diluted with chloroform and the organic layer was separated using a hydrophobic filter tube. The organic layer was evaporated and the residue was purified on an SPE cartridge (silica) using a petroleum ether/ethyl acetate gradient to give the title compound as a colourless gum (34mg).
LC-MS: Rt 3.50min MH+ 386.

### Example 10: N-Cyclobutyl-3-[1-(4-fluorophenyl)-1H-indazol-5-yl]-4-methylbenzamide

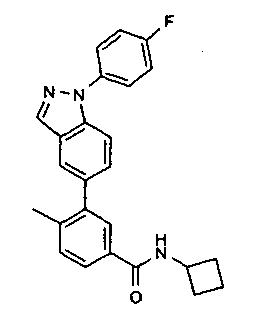

A mixture of 5-bromo-1-(4-fluorophenyl)-1*H*-indazole (Intermediate 2, 29mg), *N-*cyclobutyl-4-methyl-3-(4,4,5,5-tetramethyl-1,3,2-dioxaborolan-2-yl)benzamide (Intermediate 6, 32mg), tetrakis(triphenylphoshine)palladium (0) (6mg) ) and 1 M aqueous sodium bicarbonate in 2-propanol (1 ml) was heated at 150°C for 15min in a microwave oven. The reaction mixture was diluted with chloroform and the organic layer separated using a hydrophobic filter tube. The organic layer was evaporated and the residue was purified on an SPE cartridge (silica) eluting with a petroleum ether/ethyl acetate gradient to give the title compound as a colourless gum (34mg).
LC-MS: Rt 3.66min, MH+ 400.

### Example 11: N-Cyclopropyl-3-fluoro-4-methyl-5-{1-[4-(4-morpholinyl)phenyl]-1H-indazol-5-yl}benzamide, formate salt

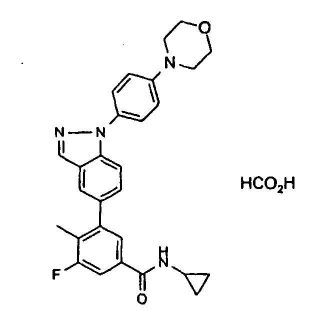

A mixture of *N*-cyclopropyl-3-fluoro-5-(1*H*-indazol-5-yl)-4-methylbenzamide (Intermediate 5, 80mg), [4-(4-morpholinyl)phenyl]boronic acid (105mg), anhydrous copper (II) acetate (71mg), pyridine (40µl) and 4Å powdered molecular sieves (200mg) in dichloromethane (5ml) was stirred at room temperature for 24h. Further dichloromethane (1ml) was added and after 24h the reaction mixture was diluted with chloroform (3ml) and water (3ml). The organic phase was separated using a hydrophobic filter tube and the solvent was evaporated. The residue was purified by preparative HPLC to give the title compound as an off-white solid (32mg).
LC-MS: Rt 3.42min, MH+ 471.

### General Method 1

A mixture of 3-fluoro-4-methyl-5-(1-phenyl-1*H*-indazol-5-yl)benzoic acid (Intermediate 9, 70mg) in thionyl chloride (3ml) was stirred at reflux for 30min. Excess thionyl chloride was removed under vacuum and the residue was a zeotroped with toluene to remove residual thionyl chloride. The crude acid chloride was dissolved in chloroform (3.1ml) and an aliquot (300µl) was treated with an amine (50µmol) in chloroform (1ml). After 1h the reaction mixture was diluted with chloroform and washed with 1 M aqueous hydrochloric acid followed by aqueous sodium hydrogen carbonate. The organic layer was separated using a hydrophobic filter tube then concentrated under vacuum to give the title compound.

### Example 12: 3-Fluoro-4-methyl-5-(1-phenyl-1H-indazol-5-yl)benzamide

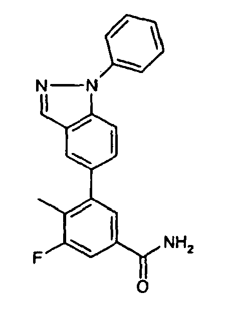

The title compound (8.3mg) was prepared by General Method 1 using a solution of ammonia in dioxan (0.5M).
LC-MS: Rt 3.43min, MH+ 346.

### Example 13: 3-Fluoro-N,4-dimethyl-5-(1-phenyl-1H-indazol-5-yl)benzamide

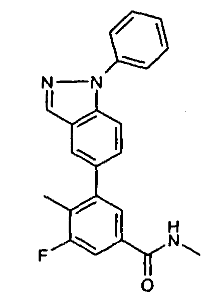

The title compound (2.0mg) was prepared by General Method 1 using a solution of methylamine in tetrahydrofuran (2M).
LC-MS: Rt 3.52min, MH+ 360.

### Example 14: N-Ethyl-3-fluoro-4-methyl-5-(1-phenyl-1H-indazol-5-yl)benzamide

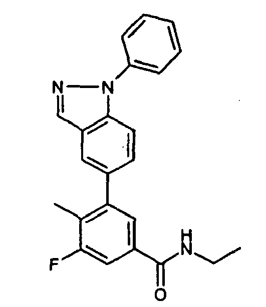

The title compound (5.9mg) was prepared by General Method 1 using a solution of ethylamine in tetrahydrofuran (2M).
LC-MS: Rt 3.62min, MH+ 374.

### Example 15: 3-Fluoro-4-methyl-5-(1-phenyl-1H-indazol-5-yl)-N-propylbenzamide

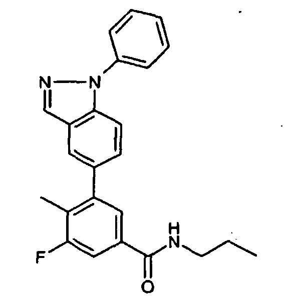

The title compound (6.4mg) was prepared by General Method 1 using propylamine.
LC-MC: 3.73min, MH+ 388.

### Example 16: N-Butyl-3-fluoro-4-methyl-5-(1-phenyl-1H-indazol-5-yl)benzamide

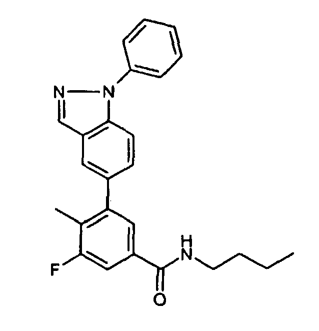

The title compound (4.6mg) was prepared by General Method 1 using butylamine.
LC-MS: Rt 3.86min, MH+ 402.

### Example 17: N-Cyclobutyl-3-fluoro-4-methyl-5-(1-phenyl-1H-indazol-5-yl)benzamide

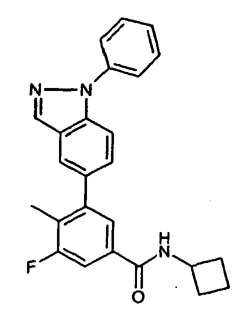

The title compound (6.6mg) was prepared by General Method 1 using cyclobutylamine.
LC-MS: Rt 3.77min, MH+ 400.

### Example 18: N-Cyclopentyl-3-fluoro-4-methyl-5-(1-phenyl-1H-indazol-5-yl)benzamide

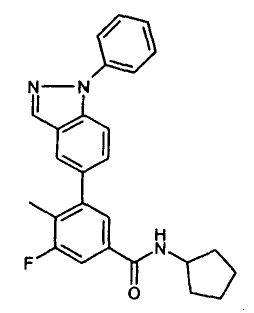

The title compound (6.5mg) was prepared by General Method 1 using cyclopentylamine.
LC-MS: Rt 3.84min, MH+ 414.

### Example 19: 3-Fluoro-4-methyl-N-(1-methylethyl)-5-(1-phenyl-1H-indazol-5-yl)benzamide

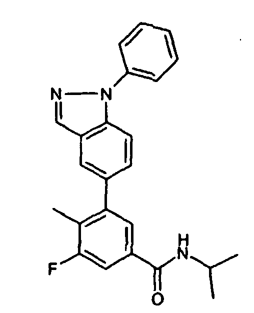

The title compound (6.6mg) was prepared by General Method 1 using isopropylamine.
LC-MS: Rt 3.68min, MH+ 388.

### Example 20: N-(Cyclopropylmethyl)-3-fluoro-4-methyl-5-(1-phenyl-1H-indazol-5-yl)benzamide

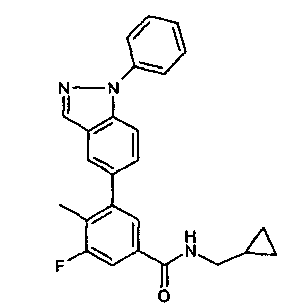

The title compound (6.5mg) was prepared by General Method 1 using cyclopropylmethylamine.
LC-MS: Rt 3.67min, MH+ 400.

### Example 21: N-Cyclopropyl-3-fluoro-4-methyl-5-[1-(2-pyridinyl)-1H-indol-5-yl]benzamide

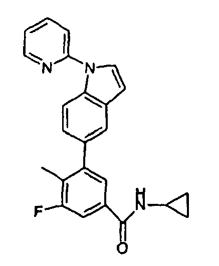

A suspension of and 5-bromo-1-(2-pyridinyl)-1*H*-indole (27mg), {5-[(cyclopropylamino)carbonyl]-3-fluoro-2-methylphenyl}boronic acid (26mg), tetrakis(triphenylphosphine)palladium(0) (1mg) and aqueous sodium hydrogen carbonate (1M, 0.3ml) in isopropanol (0.6ml) was heated at 150°C in a microwave oven for 15min. The mixture was partitioned between water and ethyl acetate and the organic layer was washed with water and brine, dried using a hydrophobic filter tube and concentrated under vacuum. The residue was purified by preparative HPLC to give the title compound (15mg).
LC-MS: Rt 3.6, MH⁺ 386.

### Example 22: N-Cyclopropyl-3-fluoro-4-methyl-5-[1-(3-pyridinyl)-1H-indol-5-yl]benzamide

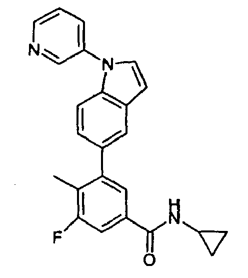

The procedure for Example 21 was followed using 5-bromo-1-(3-pyridinyl)-1*H*-indole (27mg), {5-[(cyclopropylamino)carbonyl]-3-fluoro-2-methylphenyl}boronic acid (26mg), tetrakis(triphenylphosphine)palladium (0) (1mg) and aqueous sodium hydrogen carbonate (1M, 0.3ml) in isopropanol (0.6ml) to give the title compound (18mg).
LC-MS: Rt3.31, MH⁺ 386.

### Example 23: 3-[1-(4-Cyanophenyl)-1H-indol-5-yl]-N-cyclopropyl-5-fluoro-4-methylbenzamide

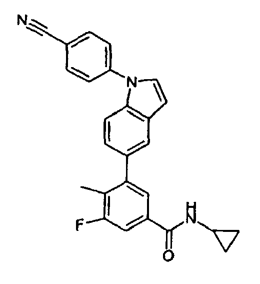

The procedure for Example 21 was followed using 4-(5-bromo-1*H*-indol-1-yl)benzonitrile (Intermediate 10, 30mg), {5-[(cyclopropylamino)carbonyl]-3-fluoro-2-methylphenyl}boronic acid (26mg), tetrakis(triphenylphosphine)palladium (0) (1mg) and aqueous sodium hydrogen carbonate (1M, 0.3ml) in isopropanol (0.6ml) to give the title compound (2.7mg).
LC-MS: Rt 3.64, MH⁺ 410.

### Example 24: 3-[1-(3-Cyanophenyl)-1H-indol-5-yl]-N-cyclopropyl-5-fluoro-4-methylbenzamide

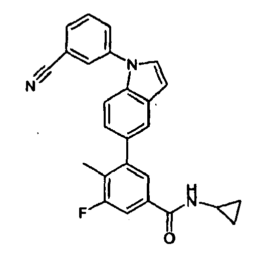

The procedure for Example 21 was followed using 3-(5-bromo-1*H*-indol-1-yl)benzonitrile (30mg), {5-[(cyclopropylamino)carbonyl]-3-fluoro-2-methylphenyl}boronic acid (26mg), tetrakis(triphenylphosphine)palladium (0) (1mg) and aqueous sodium hydrogen carbonate (1 M, 0.3ml) in isopropanol (0.6ml) to give the title compound (2.9mg).
LC-MS: Rt 3.64, MH⁺ 410.

### Example 25: N-Cyclopropyl-3-fluoro-4-methyl-5-[1-(3-methylphenyl)-1H-indazol-5-yl]benzamide

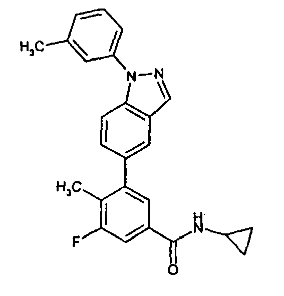

A mixture of 5-bromo-1-(3-methylphenyl)-1*H*-indazole (Intermediate 11, 179mg), {5-[(cyclopropylamino)carbonyl]-3-fluoro-2-methylphenyl}boronic acid (173mg), tetrakis(triphenylphosphine)palladium (18.7mg) and aqueous sodium hydrogen carbonate (1M, 1ml) in propan-2-ol (2.5ml) was heated in a microwave oven at 150°C for 10min. The solvent was evaporated and the residue was partitioned between chloroform and water. The organic phase was absorbed onto silica and purified on an SPE cartridge (silica) eluting with an cyclohexane/ethyl acetate gradient to give the title compound as a glass (153mg).
LC-MS: Rt 3.73min, MH+ 400.

### Example 26: N-Cyclopropyl-3-fluoro-4-methyl-5-{1-[4-(trifluoromethyl)phenyl]-1H-indazol-5-yl}benzamide

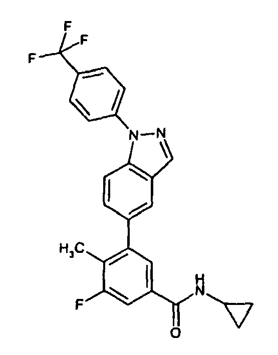

The procedure for Example 25 was followed using 5-bromo-1-[4-(trifluoromethyl)phenyl]-1*H*-indazole (Intermediate 12, 202mg), {5-[(cyclopropylamino)carbonyl]-3-fluoro-2-methylphenyl}boronic acid (167mg), tetrakis(triphenylphosphine)palladium (13.8mg) and aqueous sodium hydrogen carbonate (1M, 1.2ml) in isopropanol (2.8ml) to give the title compound (115mg).
LC-MS: Rt 3.85min, MH+ 454.

### Example 27: N-Cyclopropyl-3-fluoro-4-methyl-5-{1-[4-(methylsulfonyl)phenyl]-1H-indazol-5-yl}benzamide

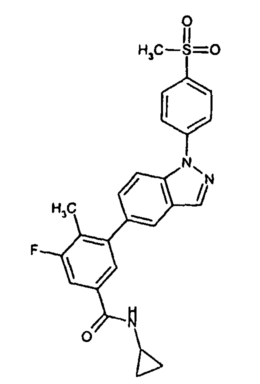

The procedure for Example 25 was followed 5-bromo-1-[4-(methylsulfonyl)phenyl]-1*H*-indazole (Intermediate 13, 210mg), {5-[(cyclopropylamino)carbonyl]-3-fluoro-2-methylphenyl}boronic acid (167mg), tetrakis(triphenylphosphine)palladium (13.8mg) and aqueous sodium hydrogen carbonate (1M, 1.2ml) in isopropanol (2.8ml) to give the title compound as a pale yellow foam (156mg).
LC-MS: Rt 3.28min, MH+ 464.

### Example 28: N-Cyclopropyl-3-fluoro-4-methyl-5-(1-{4-[2-(methylamino)-2-oxoethyl]phenyl}-1H-indazol-5-yl)benzamide

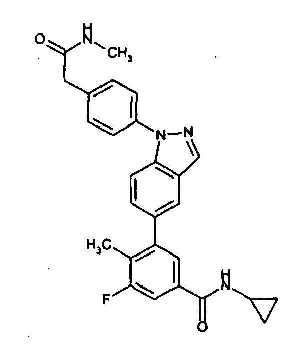

The procedure for Example 25 was followed using 2-[4-(5-bromo-1*H*-indazol-1-yl)phenyl]-*N*-methylacetamide (Intermediate 15, 206mg), {5-[(cyclopropylamino)carbonyl]-3-fluoro-2-methylphenyl}boronic acid (167mg), tetrakis(triphenylphosphine)palladium (13.8mg) and aqueous sodium hydrogen carbonate (1M, 1.2ml) in isopropanol (2.8ml). The silica SPE cartridge w as e luted with a c hloroform/methanol g radient t o give the title compound a s a yellow oil (147mg).
LC-MS: Rt 3.10min, MH+ 457.

### Example 29: 3-[1-(4-Bromophenyl)-1H-indazol-5-yl]-N-cyclopropyl-5-fluoro-4-methylbenzamide

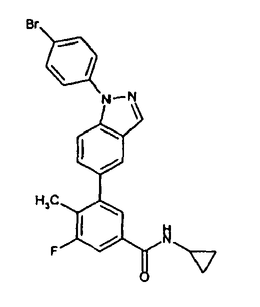

*N*-Cyclopropyl-4',5-difluoro-3'-formyl-6-methyl-3-biphenylcarboxamide (Intermediate 16, 158mg) and 4-bromophenylhydrazine (94mg) were combined in acetonitrile (3.1ml) and the mixture was warmed until a precipitate formed. The acetonitrile was removed under vacuum, the residue was dissolved in DMSO (6.2ml) and cesium carbonate (210mg) was added. The mixture was heated at 150°C in a microwave oven for 10min. The reaction mixture was diluted with ethyl acetate (10ml), washed with hydrochloric acid (1M, 5ml), saturated sodium carbonate (5ml) and brine (5ml). The organic phase was dried (magnesium sulfate) and concentrated under vacuum. The residue was purified using an SPE cartridge (silica) eluting with a cyclohexane/ethyl acetate gradient to give the title compound as a pale yellow solid (135mg).
LC-MS: Rt 3.87min, MH+ 464, 466.

### General Method 2

A mixture of 3-[1-(4-bromophenyl)-1*H*-indazol-5-yl]-*N*-cyclopropyl-5-fluoro-4-methylbenzamide (Example 29, 1eq), amine (1.2eq) and sodium tert-butoxide (4.5eq) was treated with a solution of tris(dibenzylideneacetone)dipalladium (0.015eq) and tri-t-butylphosphoniumtetrafluoroborate (0.3eq) in dry DME (10ml/eq) then heated at 125°C for 18h. The solvent was evaporated and the residue was partitioned between ethyl acetate and water. Isolation of the crude product from the organic layer was followed by purification on an acidic ion-exchange column (SCX) and/or preparative HPLC.

### Example 30: 3-(1-{4-[(Cyclohexylmethyl)amino]phenyl}-1H-indazol-5-yl}-N-cyclopropyl-5-fluoro-4-methylbenzamide, formate salt

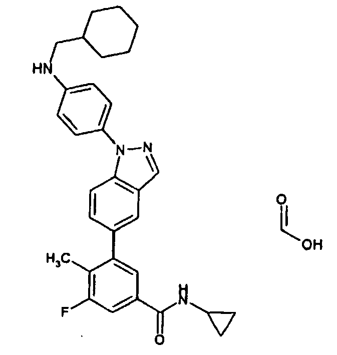

The title compound was prepared by General Method 2 using cyclohexylmethylamine (12mg) to give a brown oil (7mg).
LC-MS: Rt 4.16min, MH+ 497.

### Example 31: N-Cyclopropyl-3-[1-(4-{[2-(dimethylamino)ethyl]amino}phenyl)-1H-indazol-5-yl]-5-fluoro-4-methylbenzamide, diformate salt

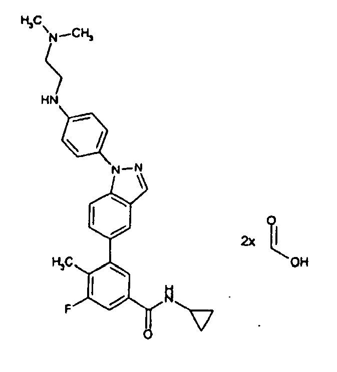

The title compound was prepared by General Method 2 using N,N-dimethylethylenediamine (0.014ml) to give a white solid (20mg).
LC-MS: Rt 2.64min, MH+ 472.

### Example 32: N-Cyclopropyl-3-fluoro-4-methyl-5-{1-[4-(tetrahydro-2H-pyran-4-ylamino)phenyl]-1H-indazol-5-yl}benzamide, formate salt

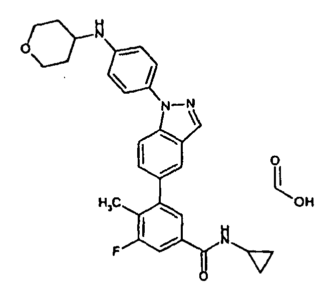

The title compound was prepared by General Method 2 using tetrahydro-2*H*-pyran-4-ylamine (20mg) to give a brown solid (18mg).
LC-MS: Rt 3.40min, MH+ 485.

### Example 33: N-Cyclopropyl-3-fluoro-4-methyl-5-(1-{4-[(terahydro-2-furanylmethyl)amino]phenyl}-1H-indazol-5-yl)benzamide, formate salt

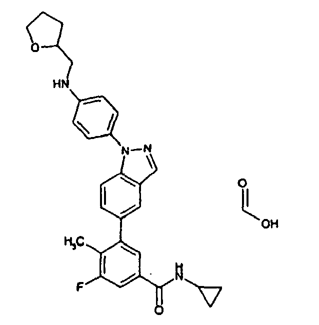

The title compound was prepared by General Method 2 using (tetrahydro-2-furanylmethyl)amine (0.014ml) to give a brown glass (12mg).
LC-MS: Rt 3.56min, MH+ 485.

### Example 34: N-Cyclopropyl-3-fluoro-4-methyl-5-[1-(4-{[2 (methyloxy)ethyl]amino}phenyl)-1H-indazol-5-yl]benzamide, formate salt

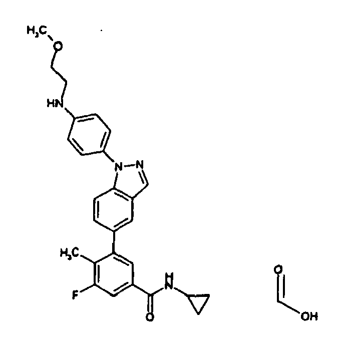

The title compound was prepared by General Method 2 using 2-methoxyethylamine (0.011ml) to give a brown glass (15mg).
LC-MS: Rt 3.40min, MH+ 459.

### Example 35: N-Cyclopropyl-3-[1-(4-{[3-(dimethylamino)propyl]amino}phenyl)-1H-indazol-5-yl]-5-fluoro-4-methylbenzamide, diformate salt

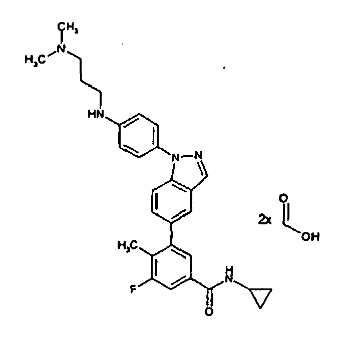

The title compound was prepared by General Method 2 using N,N-dimethyl-1,3-propanediamine(0.016ml) to give a brown glass (10mg).
LC-MS: Rt 2.67min, MH+ 486.

### Example 36: (±)-N-Cyclopropyl-3-(1-{4-[(2,3-dihydroxypropyl)amino]phenyl}-1H-indazol-5-yl)-5-fluoro-4-methylbenzamide

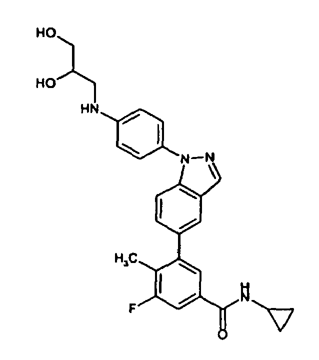

The title compound was prepared by General Method 2 using [(2,2-dimethyl-1,3-dioxolan-4-yl)methyl]amine (0.025ml) to give an off-white solid (14.6mg).
LC-MS: Rt 3.03min, MH+ 475.

### Example 37: N-Cyclopropyl-3-[1-(2,6-dimethyl-4-pyrimidinyl)-1H-indazol-5-yl]-5-fluoro-4-methylbenzamide

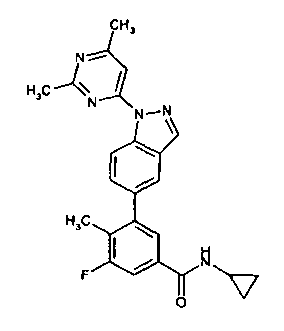

*N*-Cyclopropyl-4',5-difluoro-3'-formyl-6-methyl-3-biphenylcarboxamide (Intermediate 16, 37mg) and 4-hydrazino-2,6-dimethylpyrimidine (20mg,) were stirred together in acetonitrile (5ml) for 2h and then at 50°C for 42h. The solvent was evaporated and the residue was dissolved in DMSO (5ml). Cesium carbonate (41.5mg) was added and the mixture was heated at 180°C in a microwave oven for 20min. The solution was diluted with ethyl acetate (20ml) then extracted with hydrochloric acid (0.5M, 3x20ml). The combined acid fractions were basified to pH8 and extracted with chloroform (3x20ml). The chloroform extracts were reduced to dryness under vacuum and the residue was purified by preparative HPLC to give the title compound as a yellow glass (3.7g).
LC-MS: Rt 2.42min, MH+ 416.

### Example 38: N-Cyclopropyl-3-fluoro-4-methyl-5-[1-(6-oxo-1,6-dihydro-4-pyrimidinyl)-1H-indazol-5-yl]benzamide

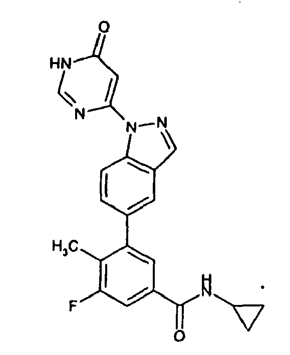

*N*-Cyclopropyl-4',5-difluoro-3'-formyl-6-methyl-3-biphenylcarboxamide (Intermediate 16, 40mg) and 6-hydrazino-4(1*H*)-pyrimidinone (24mg,) were mixed in acetonitrile (5ml) and stirred at 60°C for 18h. The solvent was evaporated and the residue was dissolved in DMSO (5ml). Cesium carbonate (49.5mg) was added and the mixture was heated at 180°C in a microwave oven for 15min. The reaction mixture was diluted with chloroform/ethyl acetate (1:1, 10ml), washed with water (2x10ml) and brine (1x10ml) then concentrated under vacuum to give the title compound as a yellow solid (32mg).
LC-MS: Rt 3.05min, MH+ 404.

### Example 39: N-Cyclopropyl-3-fluoro-4-methyl-5-{3-[4-(methyloxy)phenyl]-1,2-benzisoxazol-6-yl}benzamide

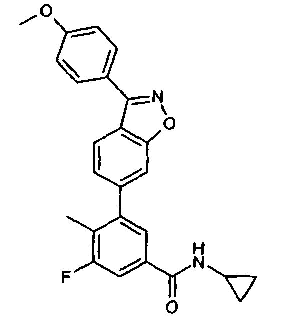

A suspension of 6-bromo-3-[4-(methyloxy)phenyl]-1,2-benzisoxazole (30mg), {5-[(cyclopropylamino)carbonyl]-3-fluoro-2-methylphenyl}boronic acid (28mg), tetrakis(triphenylphosphine)palladium(0) (1mg) and saturated aqueous sodium hydrogen carbonate (0.25ml) in isopropanol (1ml) was stirred at reflux under nitrogen for 6h. The isopropanol was removed under vacuum and the residue was partitioned between dichloromethane (3ml) and water (3ml). The organic layer was separated using a hydrophobic filter tube, the solvent was evaporated and the residue was purified on an SPE cartridge (silica, 1g) eluting with cyclohexane/ethyl acetate (100:0 to 80:20) to give the title compound as a crunchy yellow foam (26mg).
LC-MS: Rt 3.68min, MH+ 417.

### Example 40: N-Cyclopropyl-3-fluoro-5-[3-(4-hydroxyphenyl)-1,2-benzisoxazol-6-yl]-4-methylbenzamide

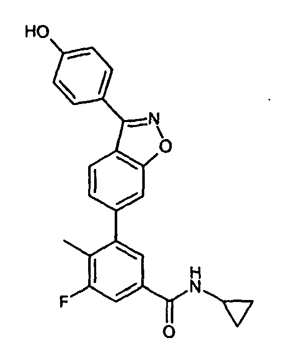

The procedure for Example 39 was followed using 4-(6-bromo-1,2-benzisoxazol-3-yl)phenol (60mg), {5-[(cyclopropylamino)carbonyl]-3-fluoro-2-methylphenyl}boronic acid (52mg), tetrakis(triphenylphosphine)palladium(0) (2mg) and saturated aqueous sodium hydrogen carbonate (0.5ml) in isopropanol (2ml). Elution of the SPE cartridge (silica, 2g) with dichloromethane/methanol (100:0 to 98:2) gave an impure white solid (67mg) which was recrystallised from isopropanol to give the title compound as a crystalline white solid (35mg).
LC-MS: Rt 3.52min, MH+ 403.

### Example 41: N-Cyclopropyl-3-fluoro-4-methyl-5-[1-(3-pyridinylmethyl)-1H-indazol-5-yl]benzamide

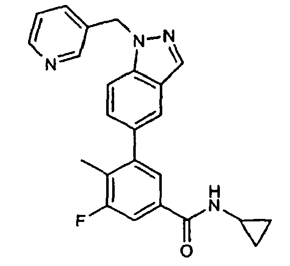

The procedure for Example 4 was followed using *N*-cyclopropyl-3-fluoro-5-(1*H-*indazol-5-yl)-4-methylbenzamide (Intermediate 5, 70mg), sodium hydride (60% dispersion in mineral oil, 22mg), 3-(bromomethyl)pyridine hydrobromide (63mg) and DMF (5ml) to give the title compound as a colourless glass (12mg).
LC-MS: Rt 2.80min, MH+ 401.

### Example 42: N-Cyclopropyl-3-fluoro-4-methyl-5-[1-(4-pyridinylmethyl)-1H-indazol-5-yl]benzamide

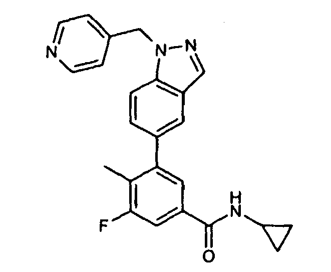

The procedure for Example 4 was followed using *N*-cyclopropyl-3-fluoro-5-(1*H-*indazol-5-yl)-4-methylbenzamide (Intermediate 5, 70mg), sodium hydride (60% dispersion in mineral oil, 22mg), 4-(bromomethyl)pyridine hydrobromide (63mg) and DMF (5ml) to give the title compound as a colourless glass (11mg).
LC-MS: Rt 2.92min, MH+ 401.

### Example 43: N-Cyclopropyl-3-fluoro-4-methyl-5-{1-[(1-oxido-2-pyridinyl)methyl]-1H-indazol-5-yl}benzamide

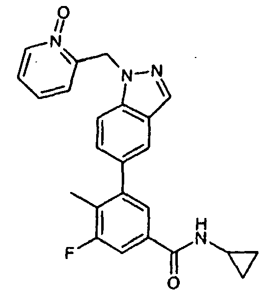

A solution of *N*-cyclopropyl-3-fluoro-4-methyl-5-[1-(2-pyridinylmethyl)-1*H*-indazol-5-yl]benzamide (Example 8, 7mg) in chloroform (1.6ml) was treated with m-CPBA (5.2mg) then stirred at 60°C for 1 h. Methanol (5ml) was added and the solution was applied to an Isolute amino cartridge. Elution with methanol gave the title compound as a white solid (8mg).
LC-MS: Rt 2.77min, MH+ 417.

### Example 44: N-Cyclopropyl-3-fluoro-4-methyl-5-{1-[(1-oxido-3-pyridinyl)methyl]-1H-indazol-5-yl}benzamide

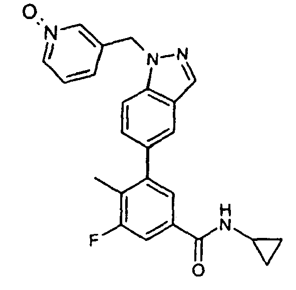

The procedure for Example 43 was followed using *N*-cyclopropyl-3-fluoro-4-methyl-5-[1-(3-pyridinylmethyl)-1*H*-indazol-5-yl]benzamide (Example 41, 7mg) m-CPBA (5.2mg) and chloroform (3ml) to give the title compound as a white solid (7mg).
LC-MS: Rt 2.67min, MH+ 417.

### Example 45: N-Cyclopropyl-3-fluoro-4-methyl-5-{1-[(1-oxido-4-pyridinyl)methyl]-1H-indazol-5-yl}benzamide

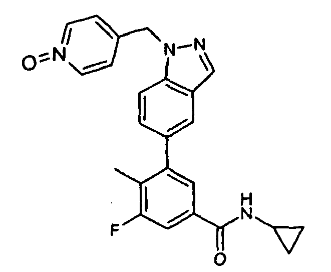

The procedure for Example 43 was followed using *N*-cyclopropyl-3-fluoro-4-methyl-5-[1-(3-pyridinylmethyl)-1*H*-indazol-5-yl]benzamide (Example 42, 8mg) m-CPBA (5.9mg) and chloroform (2ml) to give the title compound as a white solid (8mg).
LC-MS: Rt 2.66min, MH+ 417.

### General Method 3

A mixture of 3-fluoro-4-methyl-5-(1-phenyl-1*H*-indazol-5-yl)benzoic acid (Intermediate 9, 224mg) in thionyl chloride (7.5ml) was stirred at reflux for 3h. Excess thionyl chloride was removed under vacuum and the residue was a zeotroped with toluene to remove residual thionyl chloride. The crude acid chloride (187mg) was dissolved in chloroform (10m) and an aliquot (1.0ml) was added to a solution of the appropriate amine (100µmol) in a mixture (0.5ml) prepared by the addition of diisopropylethylamine (69µl), and dimethylaminopyridine (6mg) to DMF (20ml). The resulting mixture was left at room temperature for 16h, diisopropylethylamine (12µl) was added and the mixture was heated at 50°C for 24h under nitrogen The resulting gums were purified by preparative HPLC.

### Example 46: 3-Fluoro-4-methyl-5-(1-phenyl-1H-indazol-5-yl)-N-2-pyrimidinylbenzamide

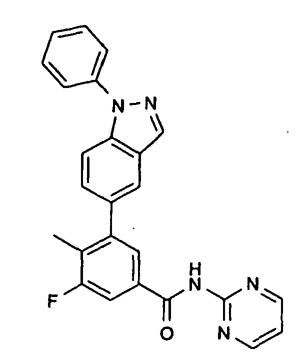

The title compound (1.3mg) was prepared by General Method 3 using 2-aminopyrimidine.
LC-MS: Rt 3.40min, MH+ 424.

### Example 47: 3-Fluoro-N-(4-fluorophenyl)-4-methyl-5-(1-phenyl-1H-indazol-5-yl)benzamide

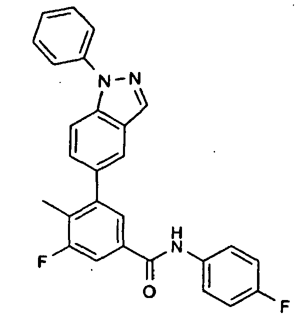

The title compound (4.1mg) was prepared by General Method 3 using 4-fluoroaniline.
LC-MS: Rt 4.0min, MH+ 440.

### Example 48: 3-Fluoro-4-methyl-5-(1-phenyl-1H-indazol-5-yl)-N-3-pyridazinylbenzamide

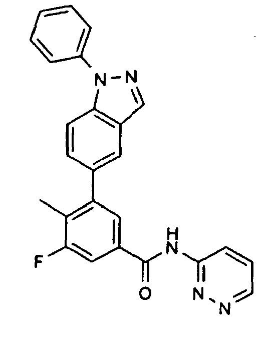

The title compound (3.1 mg) was prepared by General Method 3 using 2-aminopyridazine.
LC-MS: Rt 3.58min, MH+ 424.

### Example 49: 3-Fluoro-4-methyl-5-(1-phenyl-1H-indazol-5-yl)-N-1H-pyrazol-3-ylbenzamide

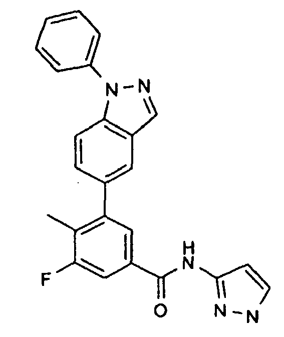

The title compound (2.9mg) was prepared by General Method 3 using 3-aminopyrazole.
LC-MC: 4.10min, MH+ 412.

### Example 50: N-(4-Fluorophenyl)-4-methyl-3-(1-phenyl-1H-indazol-5-yl)benzamide

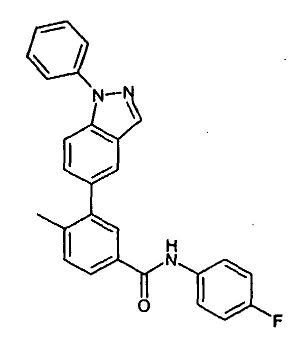

A mixture of 5-bromo-1-phenyl-1*H*-indazole (Intermediate 1 , 39mg), *N*-cyclobutyl-4-methyl-3-(4,4,5,5-tetramethyl-1,3,2-dioxaborolan-2-yl)benzamide (Intermediate 23, 50mg), tetrakis(triphenylphoshine)palladium (0) (3mg) and 2M aqueous sodium bicarbonate in isopropanol (1 ml) was heated at 150°C for 10min in a microwave oven. The reaction mixture was applied to an SPE cartridge (silica, 50g) and eluted with a cyclohexane/ethyl acetate gradient (100:0 to 0:100) to give the title compound as a white solid (40mg).
LC-MS: Rt 3.80min, MH+ 422.

### Example 51: N-Ethyl-3-[3-(4-fluorophenyl)-1N-indazol-6-yl]-4-methylbenzamide

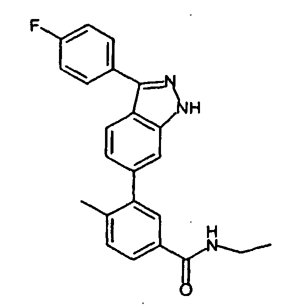

A mixture of 6-bromo-3-(4-fluorophenyl)-1*H*-indazole (Intermediate 20, 27mg), *N-*ethyl-4-methyl-3-(4,4,5,5-tetramethyl-1,3,2-dioxaborolan-2-yl)benzamide (Intermediate 18, 29mg), tetrakis(triphenylphosphine)palladium(0) (2mg) and aqueous sodium hydrogen carbonate (1M, 1ml) in isopropanol (2ml) was stirred in a microwave oven at 150°C for 15min. Water and chloroform were added and the organic layer was separated using a hydrophobic filter tube. The solvent was evaporated and the residue was purified on an SPE cartridge (silica, 2g) eluting with cyclohexane/ethyl acetate (100:0 to 50:50) to give the title compound (21 mg).
LC-MS: Rt 3.40min, MH+ 374.

### Example 52: N-Cyclopropyl-3-[3-(4-fluorophenyl)-1H-indazol-6-yl]-4-methylbenzamide

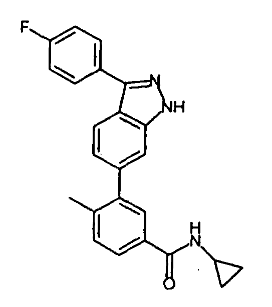

The procedure for Example 51 was followed using 6-bromo-3-(4-fluorophenyl)-1*H-*indazole (Intermediate 20, 27mg), *N*-cyclopropyl-4-methyl-3-(4,4,5,5-tetramethyl-1,3,2-dioxaborolan-2-yl)benzamide (30mg), tetrakis(triphenylphosphine)palladium(0) (2mg) and aqueous sodium hydrogen carbonate (1M, 1ml) in isopropanol (2ml) to give the title compound (20mg).
LC-MS: Rt 3.3min, MH+ 386.

### Example 53: N-Ethyl-4-methyl-3-{3-[4-(methyloxy)phenyl]-1H-indazol-6-yl}benzamide

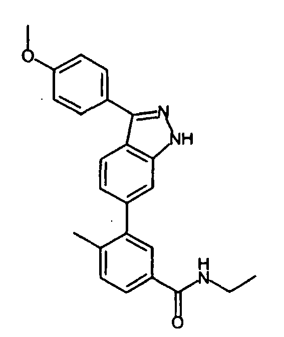

The procedure for Example 51 was followed using 6-bromo-3-(4-methoxyphenyl)-1*H-*indazole (Intermediate 22, 23mg), *N*-ethyl-4-methyl-3-(4,4,5,5-tetramethyl-1,3,2-dioxaborolan-2-yl)benzamide (Intermediate 18, 30mg), tetrakis(triphenylphosphine)palladium(0) (2mg) and aqueous sodium hydrogen carbonate (1M, 1ml) in isopropanol (2ml) to give the title compound (18mg).
LC-MS: Rt 3.41 min, MH+ 386.

### Example 54: N-Cyclopropyl-4-methyl-3-{3-[4-(methyloxy)phenyl]-1H-indazol-6-yl}benzamide

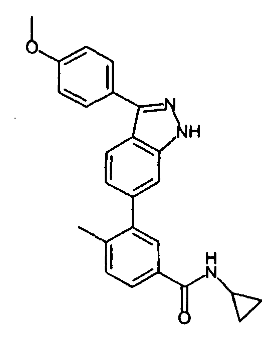

The procedure for Example 51 was followed using 6-bromo-3-(4-methoxyphenyl)-1*H-*indazole (Intermediate 22, 23mg), *N*-cyclopropyl-4-methyl-3-(4,4,5,5-tetramethyl-1,3,2-dioxaborolan-2-yl)benzamide (30mg), tetrakis(triphenylphosphine)palladium(0) (2mg) and aqueous sodium hydrogen carbonate (1M, 1ml) in isopropanol (2ml) to give the title compound (22mg).
LC-MS: Rt 3.32min, MH+ 398.

### Example 55: N-Ethyl-3-[3-(6-fluoro-3-pyridinyl)-1H-indazol-6-yl]-4-methylbenzamide

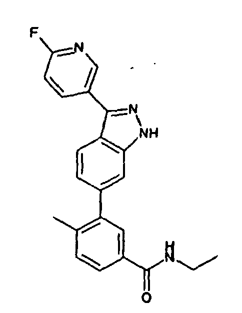

A stirred mixture of N-ethyl-3-(3-iodo-1H-indazol-6-yl)-4-methylbenzamide (Intermediate 24, 30mg), (6-fluoro-3-pyridinyl)boronic acid (130mg), tetrakis(triphenylphosphine)palladium(0) (5mg) and aqueous sodium hydrogen carbonate (1M, 0.3ml) in isopropanol (1.5ml) was heated at 150°C in a microwave oven for 15 min. The solvent was removed under vacuum and the residue was purified by preparative HPLC to give the title compound as a pale brown solid (12mg).
LC-MS: Rt 3.29min, MH⁺375.

### Example 56: N-Ethyl-3-{3-[4-(ethyloxy)phenyl]-1H-indazol-6-yl}-4-ethylbenzamide

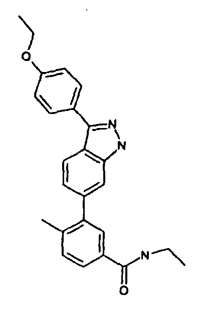

The procedure for Example 55 was followed using N-ethyl-3-(3-iodo-1H-indazol-6-yl)-4-methylbenzamide (Intermediate 24, 33mg), (4-ethoxyphenyl)boronic acid (16mg), tetrakis(triphenylphosphine)palladium(0) (2mg) and aqueous sodium hydrogen carbonate (1M, 0.2ml) in isopropanol (1ml) to give the title compound (16mg).
LC-MS: 3.59min, MH⁺400.

### Example 57: N-Ethyl-4-methyl-3-{3-[4-(methylsulfonyl)phenyl]-1H-indazol-6-yl}benzamide

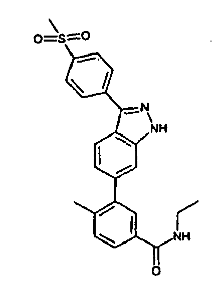

The procedure for Example 55 was followed using N-ethyl-3-(3-iodo-1H-indazol-6-yl)-4-methylbenzamide (Intermediate 24, 33mg) [4-(methylsulfonyl)phenyl]-boronic acid (19mg) tetrakis(triphenylphosphine)palladium(0) (2mg) and aqueous sodium hydrogen carbonate (1M, 0.2ml) in isopropanol (1ml) to give the title compound (16mg).
LC-MS: 3.07min, MH⁺434.

### Example 58: N-Ethyl-3-fluoro-4-methyl-5-[3-(4-methylphenyl)-1H-indazol-6-yl]benzamide

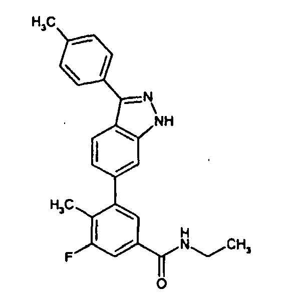

A mixture of *N*-ethyl-3-fluoro-5-(3-iodo-1*H*-indazol-6-yl)-4-methylbenzamide (Intermediate 28, 0.192g), tetrakis(triphenylphosphine)palladium (16mg) and aqueous sodium hydrogen carbonate (1M, 0.1ml) was suspended in isopropanol (1.8ml). An aliquot (0.2ml) was added to (4-methylphenyl)boronic acid (8.5mg) and the mixture was heated by microwave in a sealed vessel at 150°C for 10min. The solvent was evaporated and the residue was purified by preparative HPLC to give the title compound as a colourless glass (10mg).
LCMS: Rt 3.59min, MH+ 388.

### Example 59: N-Ethyl-3-fluoro-4-methyl-5-{3-[4-(trifluoromethy)phenyl]-1H-indazol-6-yl}benzamide

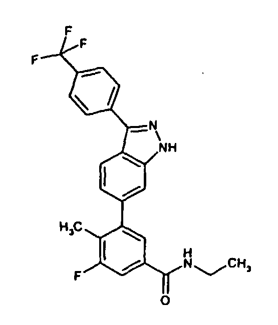

The procedure for Example 58 was followed using an aliquot (0.2ml) of the N-ethyl-3-fluoro-5-(3-iodo-1*H*-indazol-6-yl)-4-methylbenzamide (Intermediate 28) and tetrakis(triphenylphosphine)palladium in propan-2-ol mixture, [4-(trifluoromethyl)phenyl]boronic acid (12mg) and sodium hydrogen carbonate solution (1M, 0.1ml) to give the title compound as a colourless glass (7.1mg).
LCMS: Rt 3.79min, MH+ 442.

### Example 60: 3-[3-(4-Chlorophenyl)-1H-indazol-6-yl]-N-ethyl-5-fluoro-4-methylbenzamide

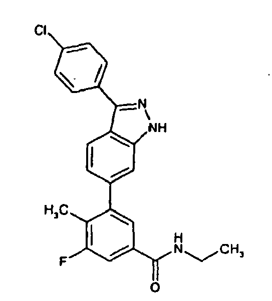

The procedure for Example 58 was followed using an aliquot (0.2ml) of the N-ethyl-3-fluoro-5-(3-iodo-1*H*-indazol-6-yl)-4-methylbenzamide (Intermediate 28) and tetrakis(triphenylphosphine)palladium in propan-2-ol mixture, [4(4-chlorophenyl)boronic acid (12mg) and sodium hydrogen carbonate solution (1M, 0.1ml) to give the title compound as a colourless glass 8.4mg).
LCMS: Rt 3.72min, MH+ 408.

### Example 61: N-Ethyl-3-fluoro-4-methyl-5-{3-[6-(methyloxy)-3-pyridinyl]-1H-indazol-6-yl}benzamide

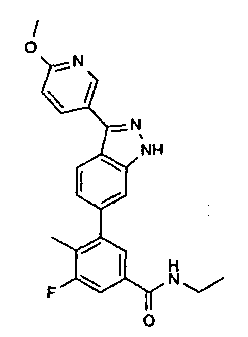

A mixture of 6-bromo-3-[6-(methyloxy)-3-pyridinyl]-1*H*-indazole (Intermediate 30, 59mg), *N*-ethyl-3-fluoro-4-methyl-5-(4,4,5,5-tetramethyl-1,3,2-dioxaborolan-2-yl)benzamide (Intermedaite 31, 71mg), tetrakis(triphenylphosphine)palladium(0) (10mg) and aqueous sodium hydrogen carbonate (1M, 2.5ml) in isopropanol (2.5ml) in a sealed vessel was heated at 150°C for 15min in a microwave oven. The reaction mixture was partitioned between chloroform and water and the organic layer was separated using a hydrophobic filter tubeThe solvent was evaporated and the residue was and purified by preparative HPLC to give the title compound (32mg).
LC-MS: Rt 3.3min, MH⁺ 405.

### Example 62: N-Ethyl-3-fluoro-4-methyl-5-[3-(2-pyridinyl)-1H-indazol-6-yl]benzamide

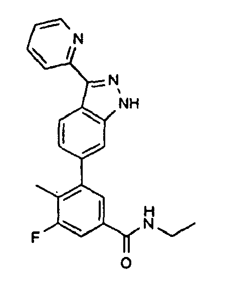

A mixture of 6-bromo-3-(2-pyridinyl)-1*H*-indazole (Intermediate 33, 74mg), N-ethyl-3-fluoro-4-methyl-5-(4,4,5,5-tetramethyl-1,3,2-dioxaborolan-2-yl)benzamide (Intermediate 31, 98mg), tetrakis(triphenylphosphine)palladium(0) (10mg) and aqueous sodium hydrogen carbonate (1M, 2.5ml) in 2-propanol (2.5ml) in a sealed vessel was heated at 150°C for 15min in a microwave oven. The reaction mixture was partitioned between chloroform and water and the organic layer separated using a hydrophobic filter tube. The solvent was evaporated and the residue was purified by preparative HPLC to give the title compound (5mg).
LC-MS: Rt 3.4min, MH⁺ 374.

### Example 63: N-Ethyl-3-fluoro-4-methyl-5-[3-(1-oxido-2-pyridinyl)-1H-indazol-6-yl]benzamide

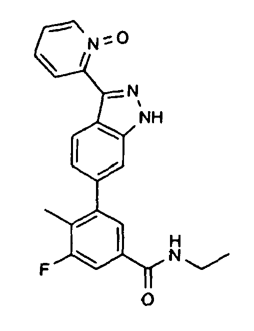

A solution of *N*-ethyl-3-fluoro-4-methyl-5-[3-(2-pyridinyl)-1*H*-indazol-6-yl]benzamide (Example 62, 15mg) in chloroform (1ml) was treated with 3-chloroperoxybenzoic acid (14mg) then stirred for 2h. Methanol was added and the solution was applied to an Isolute amino cartridge, eluting with methanol. The solvent was evaporated and the residue was purified by preparative HPLC to give the title compound (0.6mg).
LC-MS: Rt 2.70min, MH⁺ 391.

### Example 64: N-(1-Ethyl-1H-pyrazol-5-yl)-3-fluoro-5-[3-(4-fluorophenyl)-1H-indazol-6-yl]-4-methylbenzamide

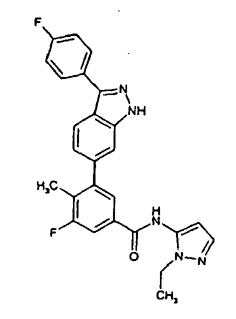

A mixture of N-(1-ethyl-1H-pyrazol-5-yl)-3-fluoro-4-methyl-5-(4,4,5,5-tetramethyl-1,3,2-dioxaborolan-2-yl)benzamide (48mg), 6-bromo-3-(4-fluorophenyl)-1H-indazole (43mg) aqueous sodium hydrogen carbonate (1M, 0.3ml) and tetrakis(triphenylphosphine)palladium (4.8mg) in isopropanol (1.5ml) in a sealed vessel was heated at 150°C for 15min in a microwave oven. The solvent was evaporated and the residue was purified by preparative HPLC to give the title compound as a colourless glass (22mg).
LC-MS: Rt 3.60min, MH⁺ 458.

### Example 65: 3-[3-(1,3-Dimethyl-1H-pyrazol-5-yl)-1H-indazol-6-yl]-N-ethyl-4-methylbenzamide

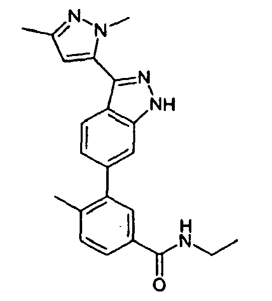

A mixture of 6-bromo-3-(1,3-dimethyl-1H-pyrazol-5-yl)-1H-indazole (Intermediate 38, 34mg), N-ethyl-4-methyl-5-(4,4,5,5-tetramethyl-1,3,2-dioxaborolan-2-yl)benzamide (Intermediate 18, 34mg), aqueous sodium hydrogen carbonate (1M, 234µL) and tetrakis(triphenylphosphine)palladium(0) (3mg) in propan-2-ol (2.5ml) was stirred in a sealed vial at 150°C for 20 min in a microwave oven. The solvent was removed under vacuum and the residue was purified by preparative HPLC to give the title compound as a white solid (8mg).
LC-MS: Rt 3.1 min, MH+ 374.

### Example 66: 3-Fluoro-5-[3-(4-fluorophenyl)-1H-indazol-6-yl]-4-methyl-N-(1-methyl-1H-pyrazol-5-yl)benzamide

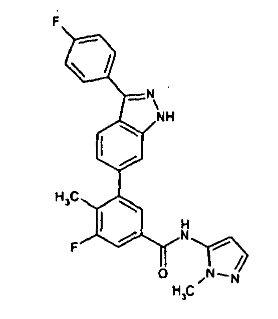

A mixture of 3-fluoro-4-methyl-N-(1-methyl-1H-pyrazol-5-yl)-5-(4,4,5,5-tetramethyl-1,3,2-dioxaborolan-2-yl)benzamide (Intermediate 40, 50mg), 6-bromo-3-(4-fluorophenyl)-1H-indazole (Intermediate 20, 41mg), aqueous sodium hydrogen carbonate (1M, 0.28ml) and tetrakis(triphenylphosphine)palladium (8mg) in isopropanol (1ml) in a sealed vial was heated at 150°C for 15min in a microwave oven. The solvent was evaporated and the residue was purified by preparative HPLC to give the title compound as a white solid (25mg).
LCMS: Rt 3.48min, MH+ 444.

### Example 67: 3-Fluoro-5-[3-(4-fluorophenyl)-1H-indazol-6-yl]-4-methyl-N-1H-pyrazol-3-ylbenzamide

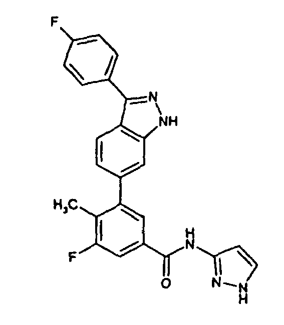

A mixture of 3-fluoro-4-methyl-N-1H-pyrazol-5-yl-5-(4,4,5,5-tetramethyl-1,3,2-dioxaborolan-2-yl)benzamide (Intermediate 42, 47mg), 6-bromo-3-(4-fluorophenyl)-1H-indazole (Intermediate 20, 41 mg), aqueous sodium hydrogen carbonate (1M, 0.28ml) and tetrakis(triphenylphosphine)palladium (8mg) in isopropanol (1.5ml) in a sealed vial was heated at 150°C for 15min in a microwave oven. The solvent was evaporated and the residue was purified by preparative HPLC to give the title compound as a white solid (25mg).
LCMS: Rt 3.56min, MH+ 430.

### Example 68: N-Ethyl-4-methyl-3-{3-[6-(4-morpholinyl)-3-pyridinyl]-1H-indazol-6-yl}benzamide

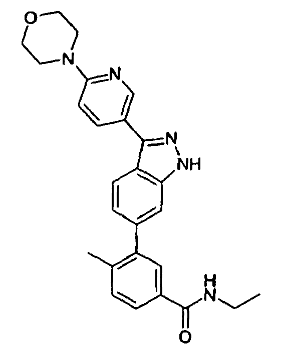

A mixture of 6-bromo-3-[6-(4-morpholinyl)-3-pyridinyl]-1H-indazole (Intermediate 45, 70mg), N-ethyl-4-methyl-5-(4,4,5,5-tetramethyl-1,3,2-dioxaborolan-2-yl)benzamide (Intermediate 18, 56mg), aqueous sodium hydrogen carbonate (1M, 390µL) and tetrakis(triphenylphosphine)palladium(0) (4.5mg) in isopropanol (2.5ml) in a sealed vial was stirred at 150°C for 20 min in a microwave oven. The solvent was removed under vacuum and the residue was purified by preparative HPLC to give the title compound as a yellow solid (5mg).
LC-MS: Rt 2.95min, MS+ 442.

### Example 69: N-Ethyl-3-fluoro-4-methyl-5-[3-(2-pyrimidinyl)-1H-indazol-6-yl]benzamide

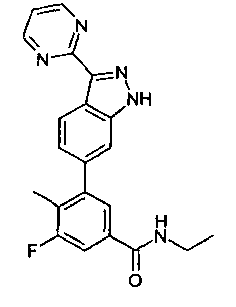

The procedure for Example 68 was followed using 6-bromo-3-(2-pyrimidinyl)-1H-indazole (Intermediate 48, 17mg), N-ethyl-3-fluoro-4-methyl-5-(4,4,5,5-tetramethyl-1,3,2-dioxaborolan-2-yl)benzamide (Intermediate 31, 19mg), sodium hydrogen carbonate (1M, 124µL) and tetrakis(triphenylphosphine)palladium(0) (1.4mg) in isopropanol (2.5ml) to give the title compound (1.7mg).
LC-MS: Rt 3.05min, MS+ 376.

### Example 70: N-Ethyl-4-methyl-5-[3-(5-pyrimidlnyl)-1H-indazol-6-yl]benzamide

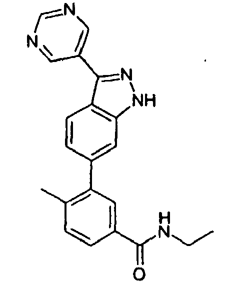

A mixture of 6-bromo-3-(5-pyrimidinyl)-1H-indazole (Intermediate 51, 60mg), N-ethyl-4-methyl-5-(4,4,5,5-tetramethyl-1,3,2-dioxaborolan-2-yl)benzamide (Intermediate 18, 63mg), aqueous sodium hydrogen carbonate (1M, 436µL) and tetrakis(triphenylphosphine)palladium(0) (5mg) in isopropanol (4ml) in a sealed vessel was stirred at 150°C for 20 min in a microwave oven. The solvent was removed under vacuum and the residue was purified by preparative HPLC to give the title compound (22mg).
LC-MS: Rt 2.70min, MS+ 358.

### Example 71: N-Ethyl-4-methyl-3-[3-(2-pyrazinyl)-1H-indazol-6-yl]benzamide

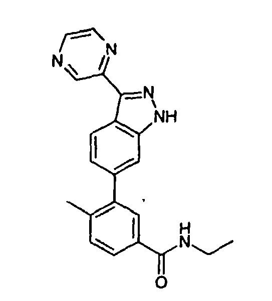

A mixture of 6-bromo-3-(2-pyrazinyl)-1H-indazole (Intermediate 54, 65mg), N-ethyl-4-methyl-5-(4,4,5,5-tetramethyl-1,3,2-dioxaborolan-2-yl)benzamide (Intermediate 18, 68mg), aqueous sodium hydrogen carbonate (1M, 472µL) and PdCl₂(dppf) (10mg) in isopropanol (5ml) in a sealed vial was stirred at 150°C for 25 min in a microwave oven. More PdCl₂(dppf) (10mg) was added and the reaction mixture was heated for a further 25 min at 150°C in the microwave oven. The solvent was removed under vacuum and the residue was purified by preparative HPLC to give the title compound as a brown solid (6mg).
LC-MS: Rt 2.99min, MS+ 358.

### Example 72: N-Ethyl-3-fluoro-5-{3-[4-fluoro-2-(methyloxy)phenyl]-1H-indazol-6-yl}-4-methylbenzamide

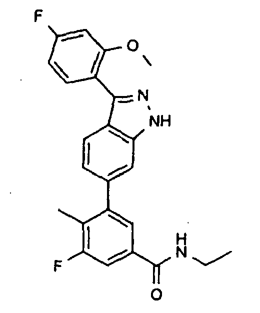

A mixture of 3-(3-bromo-1*H*-indazol-6-yl)-*N*-ethyl-5-fluoro-4-methylbenzamide (Intermediate 55, 50mg), [4-fluoro-2-(methyloxy)phenyl]boronic acid (27mg), tetrakis(triphenylphosphine)palladium(0) (2mg) and aqueous sodium hydrogen carbonate (0.125ml) in isopropanol (0.5ml) in a sealed vial was stirred at 150°C for 20min in a microwave oven. Water (2ml) and dichloromethane (2ml) were added, the phases were separated using a hydrophobic filter tube and the aqueous layer was washed with more dichloromethane (4ml). The combined organics were concentrated under vacuum and the residue was purified by preparative HPLC to give the title compound (29mg).
LC-MS: Rt 3.5min, MH+ 422.

### Example 73: N-Ethyl-3-fluoro-4-methyl-5-{3-[2-(methyloxy)-3-pyridinyl]-1H-indazol-6-yl}benzamide

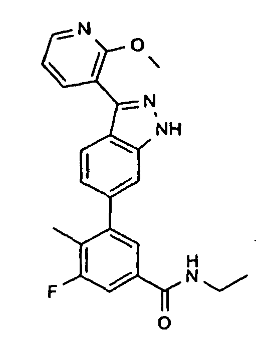

The procedure for Example 72 was followed using 3-(3-bromo-1*H*-indazol-6-yl)-*N-*ethyl-5-fluoro-4-methylbenzamide (Intermediate 55, 50mg) [2-(methyloxy)-3-pyridinyl]boronic acid (24mg) tetrakis(triphenylphosphine)palladium(0) (2mg) and aqueous sodium hydrogen carbonate (0.125ml) in isopropanol (0.5ml) to give the title compound (22mg).
LC-MS: Rt 3.1 min, MH+ 405.

### Example 74: N-Ethyl-3-fluoro-5-[3-(4-fluoro-2-methylphenyl)-1H-indazol-6-yl]-4-methylbenzamide

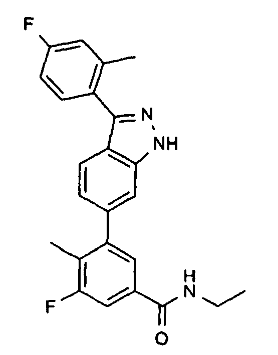

The procedure for Example 72 was followed using 3-(3-bromo-1*H*-indazol-6-yl)-*N-*ethyl-5-fluoro-4-methylbenzamide (Intermediate 55, 50mg) (4-fluoro-2-methylphenyl)boronic acid (31mg) tetrakis(triphenylphosphine)palladium(0) (2mg) and aqueous sodium hydrogen carbonate (0.125ml) in isopropanol (0.5ml) to give the title compound (23mg).
LC-MS: Rt 3.5min, MH+ 406.

### Example 75: N-Ethyl-3-fluoro-4-methyl-5-[3-(3-pyridinyl)-1H-indazol-6-yl]benzamide

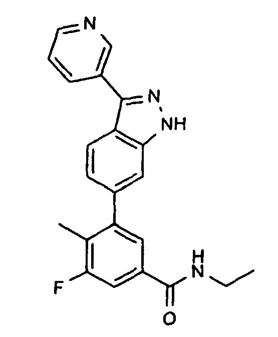

The procedure for Example 72 was followed using 3-(3-bromo-1*H*-indazol-6-yl)-*N-*ethyl-5-fluoro-4-methylbenzamide (Intermediate 55, 50mg) 3-pyridinylboronic acid (20mg) tetrakis(triphenylphosphine)palladium(0) (2mg) and aqueous sodium hydrogen carbonate (0.125ml) in isopropanol (0.5ml). More tetrakis(triphenylphosphine)palladium(0) (2mg) was added and heating was continued for a further 20min. A similar work-up and purification by preparative HPLC gave the title compound (7.8mg).
LC-MS: Rt 3.0min, MH+ 375.

### Example 76: 3-[3-(3,5-Dimethyl-4-isoxazolyl)-1H-indazol-6-yl]-N-ethyl-5-fluoro-4-methylbenzamide

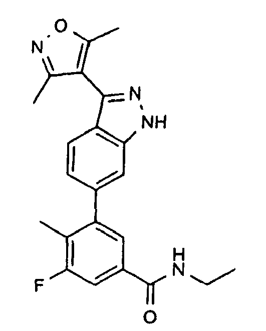

The procedure for Example 72 was followed using 3-(3-bromo-1*H*-indazol-6-yl)-*N-*ethyl-5-fluoro-4-methylbenzamide (Intermediate 55, 50mg), (3,5-dimethyl-4-isoxazolyl)boronic acid (22.5mg), tetrakis(triphenylphosphine)palladium(0) (2mg) and aqueous sodium hydrogen carbonate (0.125ml) in isopropanol (0.5ml). Heating was continued for a further 30min and a similar work-up and purification by preparative HPLC gave the title compound (6mg).
LC-MS: Rt 3.2min, MH+ 393.

### Example 77: N-Ethyl-6-(6-{5-[(ethylamion)carbonyl]-2-methylphenyl}-1H-indazol-3-yl)-3-pyridinecarboxamide

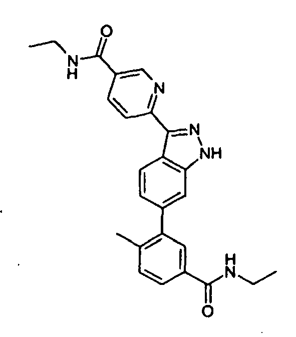

A mixture of impure 1,1-dimethylethyl 1,1-dimethylethyl 2-({5'-[(ethylamino)carbonyl]-3-fluoro-2'-methyl-4-biphenylyl}{5-[(ethylamino)carbonyl]-2-pyridinyl}methylidene)hydrazinecarboxylate (Intermediate 59, 198mg) and 1,8-diazabicyclo[5.4.0]undec-7-ene (54uL) in tetrahydrofuran (3mL) in a sealed vial was heated at 150°C for 30mins in a microwave oven. The solvent was removed under vacuum and the residue purified by chromatography on a silica column eluting with a cyclohexane:ethyl acetate gradient. The resulting product was further purified by preparative HPLC to give the title compound (3mg).
LC-MS: Rt 2.91 min, MS+ 428.

### Example 78: N-(1,4-Dimethyl-1H-pyrazol-5-yl)-3-fluoro-5-[3-(4-fluoroahenyl)-1H-indazol-6-yl]-4-methylbenzamide

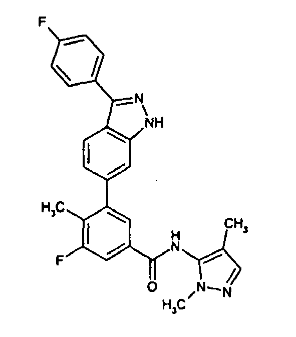

3-Fluoro-5-[3-(4-fluorophenyl)-1*H*-indazol-6-yl]-4-methylbenzoic acid (Intermediate 60, 18mg) in DMF (1ml), 1-hydroxy-7-azabenzotriazole (1mg) and 1-[3-(dimethylamino)propyl]-3-ethylcarbodiimide, hydrochloride (18.2mg) were added to a solution of 1,4-dimethyl-1*H*-pyrazol-5-amine (12.2mg) i n DMF ( 1ml) and the mixture was stirred at room temperature overnight. The DMF was removed under a stream of nitrogen and the residue was purified by preparative HPLC to give the title compound as a white solid (5.4mg).
LCMS: Rt 3.56min, MH+ 458.

### Example 79: N-(1,4-Dimethyl-1H-pyrazol-5-yl)-3-[3-(4-fluorophenyl)-1H-indazol-6-yl]-4-methylbenzamide

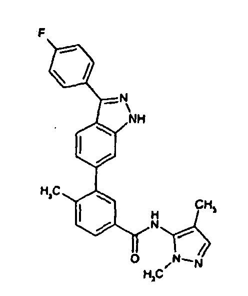

A mixture of *N*-(1,4-dimethyl-1*H*-pyrazol-5-yl)-4-methyl-3-(4,4,5,5-tetramethyl-1,3,2-dioxaborolan-2-yl)benzamide (Intermediate 61, 42mg), 6-bromo-3-(4-fluorophenyl)-1*H-*indazole (Intermediate 20, 33mg) aqueous sodium hydrogen carbonate (1M, 0.25ml) and tetrakis(triphenylphosphine)palladium (2.5mg) in isopropanol (1.5ml) in a sealed vessel was heated in a microwave oven at 150°C for 10min. The solvent was evaporated and the residue was purified by preparative HPLC to give the title compound as a white foam (22.5mg).
LCMS: Rt 3.50min, MH+ 440.

### Example 80: N-(3,5-Dimethyl-4-isoxazolyl)-3-[3-(4-fluorophenyl)-1H-indazol-6-yl]-4-methylbenzamide

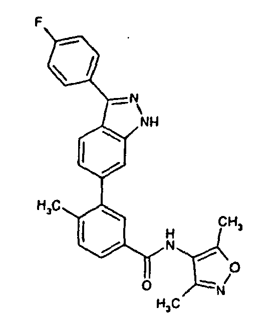

A mixture of *N*-(3,5-dimethyl-4-isoxazolyl)-4-methyl-3-(4,4,5,5-tetramethyl-1,3,2-dioxaborolan-2-yl)benzamide (Intermediate 62, 34mg), 6-bromo-3-(4-fluorophenyl)-1*H-*indazole (Intermediate 20, 29mg), aqueous sodium hydrogen carbonate (1M, 0.2ml) and tetrakis(triphenylphosphine)palladium (2mg) in isopropanol (1.5ml) in a sealed vessel was heated in a microwave oven at 150°C for 10min. The solvent was evaporated and the residue was purified by preparative HPLC to give the title compound as an off- white solid (22.5mg).
LCMS: Rt 3.43min, MH+ 441.

### Example 81: 3-[3-(1,2-Dimethyl-1H-imidazol-5-yl)-1H-indazol-6-yl]-N-ethyl-5-fluoro-4-methylbenzamide

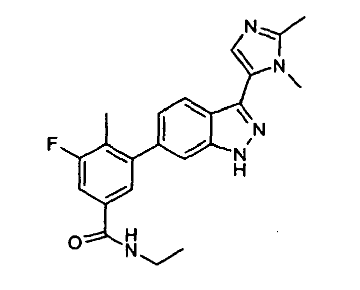

A solution of 1,1-dimethylethyl 3-[3-(1,2-dimethyl-1H-imidazol-5-yl)-1-({[2-(trimethylsilyl)ethyl]oxy}methyl)-1H-indazol-6-yl]-5-fluoro-4-methylbenzoate (Intermediate 68, 90mg), hydrochloric acid (5M, 7ml) and acetonitrile (5ml) was heated at 85° for 1 h. The solvent was evaporated and the residue was triturated with ether (2x20ml). The residual solid was treated with thionyl chloride (4ml) and toluene (5ml) then heated at reflux for 40min. The solvent was removed under vacuum and the residue was suspended in THF (20ml) and treated with a solution of ethylamine in THF ( 2M, 5ml). The solvent was removed under vacuum and the residue was purified by preparative HPLC to give the title compound as a pale brown solid (29mg).
LC-MS: Rt 2.26min, MH+ 392.

### Example 82: N-Ethyl-3-fluoro-4-methyl-5-[3-(2-methyl-4-pyridinyl)-1H-indazol-6-yl]benzamide, formate salt

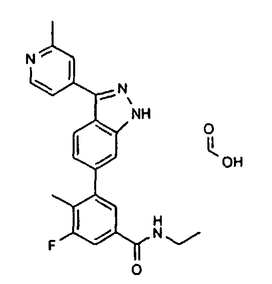

A mixture of (2-methyl-4-pyridinyl)boronic acid hydrochloride (20mg), 3-(3-bromo-1*H-*indazol-6-yl)-*N*-ethyl-5-fluoro-4-methylbenzamide (Intermediate 55, 38mg), sodium hydrogen carbonate (1.5ml) and tetrakis(triphenylphosphine) palladium(0) (5mg) in isopropanol (1ml) in a sealed vessel was heated at 150°C in a microwave oven for 15min. The crude mixture was applied to an SCX cartridge (silica) and eluted with 10% aqueous ammonia (0.88) in methanol. The solvent was evaporated and the residue was purified by preparative HPLC to give the title compound (10.1mg).
LC-MS: Rt 2.6min MH+ 389.

### Abbreviations

- AcOH: Acetic acid
- Ar: Aryl
- Boc: t-Butoxycarbonyl
- DBU: 1,8-Diazabicyclo[5.4.0]undec-7-ene
- DCM: Dichloromethane
- DIPEA: N,N-Diisopropylethylamine
- DME: 1,2-Dimethoxyethane
- DMF: Dimethylformamide
- DMSO: Dimethylsulfoxide
- EtOH: Ethanol
- h: Hours
- Hal: Halogen
- HATU: O-(7-Azabenzotriazol-1-yl)-N,N,N',N'-tetramethyluronium hexafluorophosphate
- Het: Heteroaryl
- m-CPBA: 3-Chloroperbenzoic acid
- MeCN: Acetonitrile
- MeOH: Methanol
- min: Minutes
- Ms: Mesyl
- NBS: N-Bromosuccinimide

- PdCl₂(dppf): [1,1'-bis(Diphenylphosphino)ferrocene]dichloropalladium (II) complex with dichloromethane (1:1)
- Rt: Retention Time
- SPE: Solid phase extraction
- THF: Tetrahydrofuran

### BIOLOGICAL EXAMPLES

The activity of compounds of formula (I) as p38 inhibitors may be determined by the following *in vitro* assays:

### Fluorescence anisotropy kinase binding assay 1

The kinase enzyme, fluorescent ligand and a variable concentration of test compound are incubated together to reach thermodynamic equilibrium under conditions such that in the absence of test compound the fluorescent ligand is significantly (>50%) enzyme bound and in the presence of a sufficient concentration (>10x Kᵢ) of a potent inhibitor the anisotropy of the unbound fluorescent ligand is measurably different from the bound value.

The concentration of kinase enzyme should preferably be ≥ 1 x K_{f}. The concentration of fluorescent ligand required will depend on the instrumentation used, and the fluorescent and physicochemical properties. The concentration used must be lower than the concentration of kinase enzyme, and preferably less than half the kinase enzyme concentration. A typical protocol is:

All components dissolved in Buffer of final composition 62.5 mM HEPES, pH 7.5, 1.25 mM CHAPS, 1.25 mM DTT, 12.5 mM MgCl₂ 3.3% DMSO.
p38 Enzyme concentration: 12 nM
Fluorescent ligand concentration: 5 nM
Test compound concentration: 0.1 nM - 100 µM

Components incubated in 30 µl final volume in NUNC 384 well black microtitre plate until equilibrium reached (5-30 mins)

Fluorescence anisotropy read in LJL Acquest.
Definitions:
Kᵢ = dissociation constant for inhibitor binding
K_{f} = dissociation constant for fluorescent ligand binding

The fluorescent ligand is the following compound: 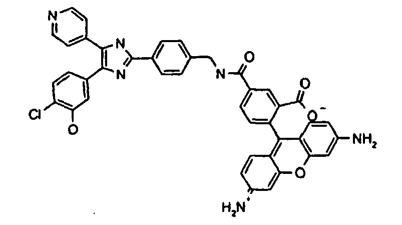 which is derived from 5-[2-(4-aminomethylphenyl)-5-pyridin-4-yl-1H-imidazol-4-yl]-2-chlorophenol and rhodamine green.

### Fluorescence anisotropy kinase binding assay 2 (macro volume assay)

The kinase enzyme, fluorescent ligand and a variable concentration of test compound are incubated together to reach thermodynamic equilibrium under conditions such that in the absence of test compound the fluorescent ligand is significantly (>50%) enzyme bound and in the presence of a sufficient concentration (>10 x *K*i) of a potent inhibitor the anisotropy of the unbound fluorescent ligand is measurably different from the bound value.

The concentration of kinase enzyme should preferably be 2 x Kf. The concentration of fluorescent ligand required will depend on the instrumentation used, and the fluorescent and physicochemical properties. The concentration used must be lower than the concentration of kinase enzyme, and preferably less than half the kinase enzyme concentration.

The fluorescent ligand is the following compound: 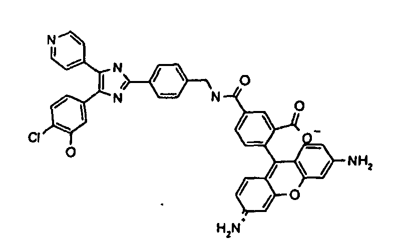 which is derived from 5-[2-(4-aminomethylphenyl)-5-pyridin-4-yl-1H-imidazol-4-yl]-2-chlorophenol and rhodamine green.

Recombinant human p38α was expressed as a GST-tagged protein. To activate this protein, 3.5 µM unactivated p38α was incubated in 50 mM Tris-HCl pH 7.5, 0.1 mM EGTA, 0.1% 2-mercaptoethanol, 0.1mM sodium vanadate, 10mM MgAc, 0.1mM ATP with 200nM MBP-MKK6 DD at 30 degrees for 30 mins. Following activation p38α was re-purified and the activity assessed using a standard filter-binding assay.

Protocol: All components are dissolved in buffer of composition 62.5 mM HEPES, pH 7.5, 1.25 mM CHAPS, 1 mM DTT, 12.5 mM MgCl₂ with final concentrations of 12nM p38α and 5nM fluorescent ligand. 30µl of this reaction mixture is added to wells containing 1 µl of various concentrations of test compound (0.28 nM - 16.6 µM final) or DMSO vehicle (3% final) in NUNC 384 well black microtitre plate and equilibrated for 30-60 mins at room temperature. Fluorescence anisotropy is read in Molecular Devices Acquest (excitation 485nm/emission 535nm).
Definitions:
*K*i = dissociation constant for inhibitor binding
Kf = dissociation constant for fluorescent ligand binding

### Fluorescence anisotropy kinase binding assay 3 (micro volume assay)

The kinase enzyme, fluorescent ligand and a variable concentration of test compound are incubated together to reach thermodynamic equilibrium under conditions such that in the absence of test compound the fluorescent ligand is significantly (>50%) enzyme bound and in the presence of a sufficient concentration (>10 x *K*i) of a potent inhibitor the anisotropy of the unbound fluorescent ligand is measurably different from the bound value.

The concentration of kinase enzyme should preferably be 2 x Kf. The concentration of fluorescent ligand required will depend on the instrumentation used, and the fluorescent and physicochemical properties. The concentration used must be lower than the concentration of kinase enzyme, and preferably less than half the kinase enzyme concentration.

The fluorescent ligand is the following compound: 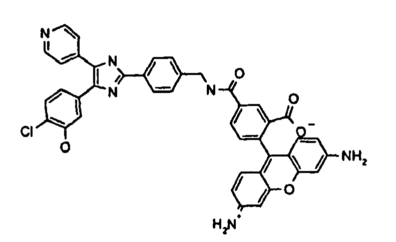 which is derived from 5-[2-(4-aminomethylphenyl)-5-pyridin-4-yl-1H-imidazol-4-yl]-2-chlorophenol and rhodamine green.

Recombinant human p38α was expressed as a GST-tagged protein. To activate this protein, 3.5 µM unactivated p38α was incubated in 50 mM Tris-HCl pH 7.5, 0.1 mM EGTA, 0. 1 % 2-mercaptoethanol, 0.1mM sodium vanadate, 10mM MgAc, 0.1mM ATP with 200nM MBP-MKK6 DD at 30 degrees for 30 mins. Following activation p38α was re-purified and the activity assessed using a standard filter-binding assay.

Protocol: All components are dissolved in buffer of composition 62.5 mM HEPES, pH 7.5, 1.25 mM CHAPS, 1 mM DTT, 12.5 mM MgCl₂ with final concentrations of 12nM p38α and 5nM fluorescent ligand. 6µl of this reaction mixture is added to wells containing 0.2 µl of various concentrations of test compound (0.28 nM - 16.6 µM final) or DMSO vehicle (3% final) in Greiner 384 well black low volume microtitre plate and equilibrated for 30-60 mins at room temperature. Fluorescence anisotropy is read in Molecular Devices Acquest (excitation 485nm/emission 535nm).
Definitions:
*K*i = dissociation constant for inhibitor binding
Kf = dissociation constant for fluorescent ligand binding

### Results

The compounds described in the Examples were tested in at least one of the assays described above and had either IC₅₀ values of <10 µM or pKᵢ values of >6.

## Claims

1. A compound of formula (I): 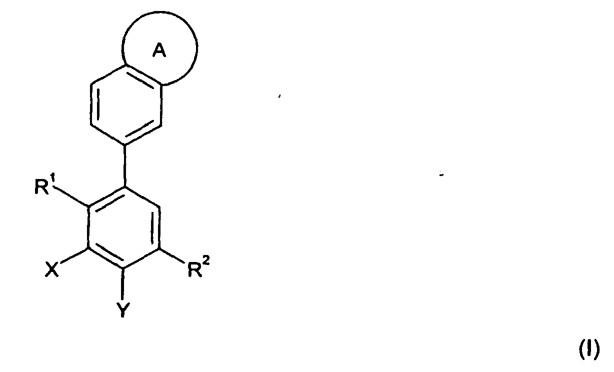 wherein
A is a fused 5-membered heteroaryl ring substituted by -(CH₂)ₘaryl or - (CH₂)ₘheteroaryl wherein the aryl or heteroaryl is optionally substituted by one or more substituents independently selected from oxo, C₁₋₆alkyl, halogen, -CN, trifluoromethyl, - OR³, -(CH₂)ₙCO₂R³, -NR³R⁴, -(CH₂)ₙCONR³R⁴, -NHCOR³, -SO₂NR³R⁴, -NHSO₂R³ and -S(O)ₚR³, and
A is optionally further substituted by one substituent selected from -OR⁵, halogen, trifluoromethyl, -CN, -CO₂R⁵ and C₁₋₆alkyl optionally substituted by hydroxy;
R¹ is selected from methyl and chloro;
R² is selected from -NH-CO-R⁶ and -CO-NH-(CH₂)_{q}-R⁷;
R³ is selected from hydrogen, -(CH₂)ᵣ-C₃₋₇cycloalkyl, -(CH₂)ᵣheterocyclyl, - (CH₂)ᵣaryl, and C₁₋₆alkyl optionally substituted by up to two substituents independently selected from -OR⁸ and -NR⁸R⁹,
R⁴ is selected from hydrogen and C₁₋₆alkyl, or
R³ and R⁴, together with the nitrogen atom to which they are bound, form a 5- or 6- membered heterocyclic ring optionally containing one additional heteroatom selected from oxygen, sulfur and N-R¹⁰;
R⁵ is selected from hydrogen and C₁₋₆alkyl;
R⁶ is selected from hydrogen, C₁₋₆alkyl, -(CH₂)_{q}-C₃₋₇cycloalkyl, trifluoromethyl, - (CH₂)ₛheteroaryl optionally substituted by R¹¹ and/or R¹², and -(CH₂)ₛphenyl optionally substituted by R¹¹ and/or R¹²;
R⁷ is selected from hydrogen, C₁₋₆alkyl, C₃₋₇cycloalkyl, -CONHR¹³, phenyl optionally substituted by R¹¹ and/or R¹², and heteroaryl optionally substituted by R¹¹ and/or R¹²;
R⁸ and R⁹ are each independently selected from hydrogen and C₁₋₆alkyl;
R¹⁰ is selected from hydrogen and methyl;
R¹¹ is selected from C₁₋₆alkyl, C₁₋₆alkoxy, -(CH₂)_{q}-C₃₋₇cycloalkyl, -CONR¹³R¹⁴, - NHCOR¹⁴, halogen, -CN, -(CH₂)ₜNR¹⁵R¹⁶, trifluoromethyl, phenyl optionally substituted by one or more R¹² groups, and heteroaryl optionally substituted by one or more R¹² groups;
R¹² is selected from C₁₋₆alkyl, C₁₋₆alkoxy, halogen, trifluoromethyl, and - (CH₂)ₜNR¹⁵R¹⁶;
R¹³ and R¹⁴ are each independently selected from hydrogen and C₁₋₆alkyl, or
R13 and R¹⁴, together with the nitrogen atom to which they are bound, form a 5- or 6-membered heterocyclic ring optionally containing one additional heteroatom selected from oxygen, sulfur and N-R¹⁰, wherein the ring may be substituted by up to two C₁₋₆alkyl groups;
R¹⁵ is selected from hydrogen, C₁₋₆alkyl and -(CH₂)_{q}-C₃₋₇cycloalkyl optionally substituted by C₁₋₆alkyl,
R¹⁶ is selected from hydrogen and C₁₋₆alkyl, or
R¹⁵ and R¹⁶, together with the nitrogen atom to which they are bound, form a 5- or 6-membered heterocyclic ring optionally containing one additional heteroatom selected from oxygen, sulfur and N-R¹⁰;
X and Y are each independently selected from hydrogen, methyl and halogen;
m, n, p and q are each independently selected from 0, 1 and 2;
r and s are each independently selected from 0 and 1; and
t is selected from 0, 1, 2 and 3;
with the proviso that when A is substituted by -(CH₂)ₘheteroaryl and m is 0, the - (CH₂)ₘheteroaryl group is not a 5-membered heteroaryl ring optionally substituted by C₁₋₂alkyl;
or a pharmaceutically acceptable derivative thereof.

2. A compound according to claim 1 wherein A is a fused 5-membered heteroaryl ring containing up to two heteroatoms independently selected from oxygen and nitrogen.

3. A compound according to claim 1 or claim 2 wherein R¹ is methyl.

4. A compound according to any one of the preceding claims wherein R² is -CO-NH-(CH₂)_{q}-R⁷.

5. A compound according to any one of the preceding claims wherein A is substituted by -(CH₂)ₘheteroaryl wherein the heteroaryl is a 5- or 6-membered heteroaryl ring containing up to two heteroatoms independently selected from oxygen and nitrogen.

6. A compound according to claim 5 wherein the the heteroaryl is optionally substituted by one or two substituents independently selected from oxo, C₁₋₆alkyl, halogen, -OR³, - NR³R⁴ and -(CH₂)ₙCONR³R⁴.

7. A compound according to claim 6 wherein the heteroaryl is substituted by one or two substituents independently selected from oxo and C₁₋₆alkyl.

8. A compound according to any one of claims 1 to 4 wherein A is substituted by - (CH₂)ₘaryl wherein the aryl is phenyl.

9. A compound according to claim 8 wherein the aryl is substituted by one or two substituents independently selected from C₁₋₆alkyl, halogen, -CN, trifluoromethyl, -OR³, - NR³R⁴, -(CH₂)ₙCONR³R⁴ and -S(O)ₚR³.

10. A compound according to any one of the preceding claims wherein X is hydrogen or fluorine.

11. A compound selected from:
*N*-cyclopropyl-3-fluoro-4-methyl-5-(1-phenyl-1*H*-indazol-5-yl)benzamide;
*N*-cyclopropyl-3-fluoro-5-[1-(4-fluorophenyl)-1*H*-indazol-5-yl]-4-methylbenzamide;
*N*-cyclopropyl-3-fluoro-5-[1-(4-fluoro-2-methylphenyl)-1*H*-indazol-5-yl]-4-methylbenzamide;
*N*-cyclopropyl-3-fluoro-4-metho-5-{1-[4-(4-morpholinyl)phenyl]-1*H*-indazol-5-yl}benzamide;
*N*-ethyl-3-fluoro-4-methyl-5-(1-phenyl-1*H*-indazol-5-yl)benzamide;
*N*-(cyclopropylmethyl)-3-fluoro-4-methyl-5-(1-phenyl-1*H*-indazol-5-yl)benzamide;
*N*-cyclopropyl-3-fluoro-4-methyl-5-{1-[4-(methylsulfonyl)phenyl]-1*H*-indazol-5-yl}benzamide;
*N*-cyclopropyl-3-fluoro-4-methyl-5-(1-{4-[2-(methylamino)-2-oxoethyl]phenyl}-1*H*-indazol-5-yl)benzamide;
*N*-cyelopropyl-3-[1-(4-{[2-(dimethylamino)ethyl]amino}phenyl)-1*H*-indazol-5-yl]-5-fluoro-4-methylbenzamide;
*N*-cyclopropyl-3-fluoro-4-methyl-5-{1-[4-(tetrahydro-2*H*-pyran-4-ylamino)phenyl]-1*H*-indazol-5-yl}benzamide;
*N*-cyclopropyl-3-fluoro-4-methyl-5-(1-{4-[(tetrahydro-2-furanylmethyl)amino]phenyl}-1*H-*indazol-5-yl)benzamide;
*N*-cyclopropyl-3-(1-{4-[(2,3-dihydroxypropyl)amino]phenyl}-1*H*-indazol-5-yl)-5-fluoro-4-methylbenzamide;
*N*-cyclopropyl-3-fluoro-4-methy-5-{3-[4-(methyloxy)phenyl]-1,2-benzisoxazol-6-yl}benzamide;
*N*-cyclopropyl-3-fluoro-5-[3-(4-hydroxyphenyl)-1,2-benzisoxazol-6-yl]-4-methylbenzamide;
*N*-cyclopropyl-3-fluoro-4-methyl-5-{1-[(1-oxido-2-pyridinyl)methyl]-1*H*-indazol-5-yl}benzamide;
*N*-ethyl-3-[3-(4-fluorophenyl)-1*H*-indazol-6-yl]-4-methylbenzamide;
*N*-cyclopropyl-3-[3-(4-fluorophenyl)-1*H*-indazol-6-yl]-4-methylbenzamide;
*N*-ethyl-4-methyl-3-{3-[4-(methyloxy)phenyl]-1*H*-indazol-6-yl}benzamide;
*N*-cyclopropyl-4-methyl-3-{3-[4-(methyloxy)phenyl]-1*H*-indazol-6-yl}benzamide;
N-(1-ethyl-1H-pyrazol-5-yl)-3-fluoro-5-[3-(4-fluorophenyl)-1H-indazol-6-yl]-4-methylbenzamide;
3-fluoro-5-[3-(4-fluorophenyl)-1H-indazol-6-yl]-4-methyl-N-(1-methyl-1H-pyrazol-5-yl)benzamide;
N-ethyl-3-fluoro-5-{3-[4-fluoro-2-(methyloxy)phenyl]-1H-indazol-6-yl}-4-methylbenzamide;
*N*-(1,4-dimethyl-1*H*-pyrazol-5-yl)-3-fluoro-5-[3-(4-fluorophenyl)-1*H*-indazol-6-yl]-4-methylbenzamide; and
*N*-(1,4-dimethyl-1*H*-pyrazol-5-yl)-3-[3-(4-fluorophenyl)-1*H*-indazol-6-yl]-4-methylbenzamide;
or a pharmaceutically acceptable derivative thereof.

12. A pharmaceutical composition comprising at least one compound as claimed in any one of claims 1 to 11, or a pharmaceutically acceptable derivative thereof, in association with one or more pharmaceutically acceptable excipients, diluents and/or carriers.

13. A compound according to any one of claims 1 to 11, or a pharmaceutically acceptable derivative thereof, for use in therapy.

14. A compound as claimed in any one of claims 1 to 11, or a pharmaceutically acceptable derivative thereof, for use in the treatment or prophylaxis of a condition or disease state mediated by p38 kinase activity or mediated by cytokines produced by the activity of p38 kinase.

15. Use of a compound as claimed in any one of claims 1 to 11, or a pharmaceutically acceptable derivative thereof, in the manufacture of a medicament for use in the treatment of a condition or disease state mediated by p38 kinase activity or mediated by cytokines produced by the activity of p38 kinase.

16. A process for preparing a compound of formula (I) as claimed in any one of claims 1 to 11, or a pharmaceutically acceptable derivative thereof, which comprises
(a) reacting a compound of formula (II) 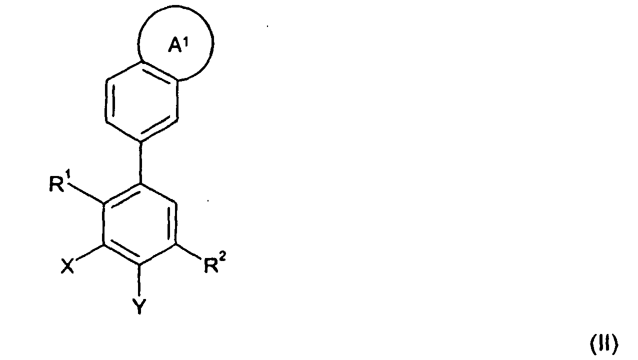 in which R¹, R², X and Y are as defined in claim 1 and A¹ is an unsubstituted fused 5-membered heteroaryl ring with a halide derivative of formula (IIIA) or (IIIB)
Z-(CH₂)ₘaryl (IIIA)
Z-(CH₂)ₘheteroaryl (IIIB)
in which -(CH₂)ₘaryl and -(CH₂)ₘheteroaryl are as defined in claim 1 and Z is halogen,
in the presence of a base,
or, when A is substituted by -(CH₂)ₘaryl wherein m is 0, reacting the compound of formula (II) with a boronic acid compound of formula (IV)
(HO)₂B-(CH₂)ₘaryl (IV)
in which -(CH₂)ₘaryl is as defined in claim 1,
(b) reacting a compound of formula (V) 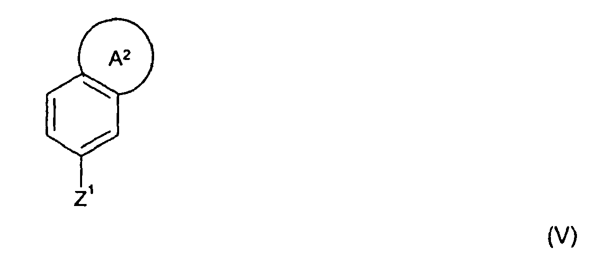 in which A² is A as defined in claim 1 and Z¹ is halogen,
with a compound of formula (VIA) or (VIB) 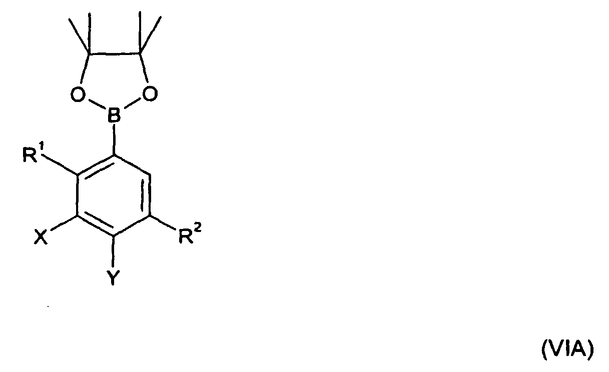 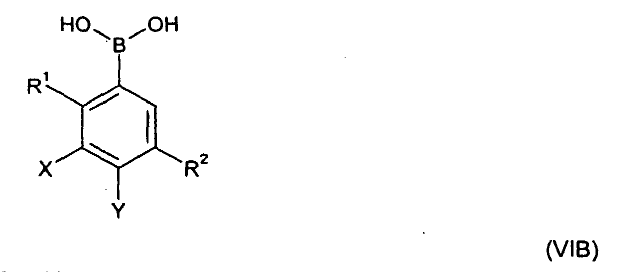 in which R¹, R², X and Y are as defined in claim 1,
in the presence of a catalyst;
(c) reacting a compound of formula (XVI) 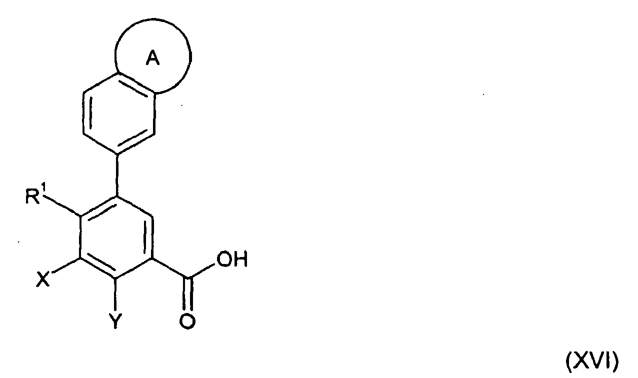 in which A, R¹, X and Y are as defined in claim 1,
with an amine compound of formula (XV)
R⁷-(CH₂)_{q}-NH₂ (XV)
in which R⁷ and q are as defined in claim 1,
under amide forming conditions;
d) when A is a fused pyrazolyl, reacting a compound of formula (XVII) 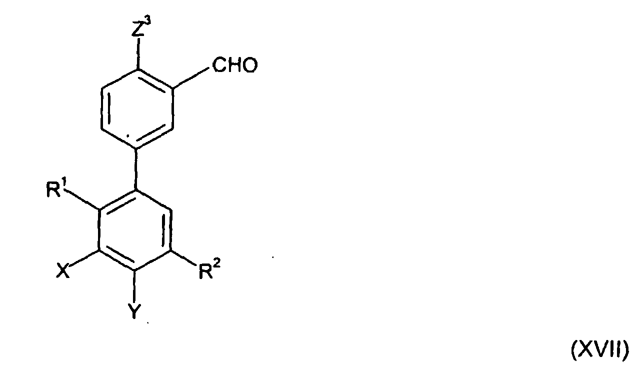 in which R¹, R², X and Y are as defined in claim 1 and Z³ is halogen,
with a hydrazine derivative of formula (VIIIA) or (VIIIB)
H₂NNH-(CH₂)ₘaryl (VIIIA)
H₂NNH-(CH₂)ₘheteroaryl (VIIIB)
in which -(CH₂)ₘaryl and -(CH₂)ₘheteroaryl are as defined in claim 1;
(e) reacting a compound of formula (XVIII) 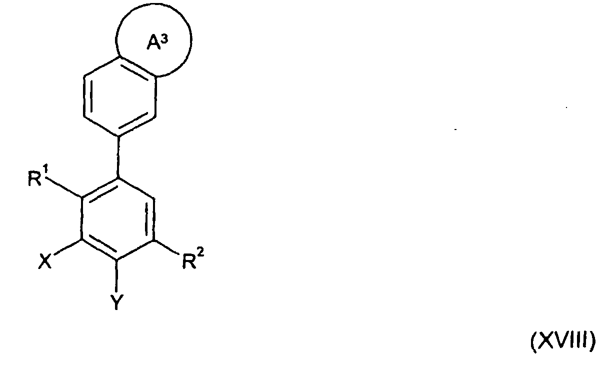 in which R¹, R², X and Y are as defined in claim 1 and A³ is a fused 5-membered heteroaryl ring substituted by halogen, with a suitable boronic acid derivative; or
(f) final stage modification of one compound of formula (I) as defined in claim 1 to give another compound of formula (I) as defined in claim 1.

## Patentansprüche

1. Verbindung der Formel (I): 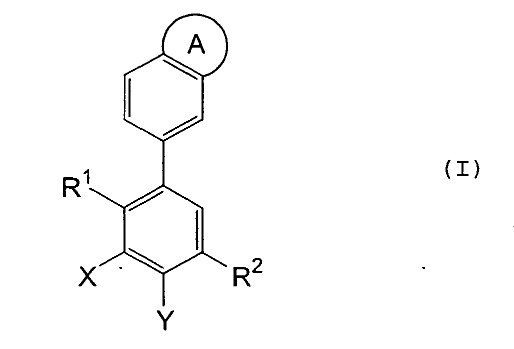 worin
A ein kondensierter 5-gliedriger Heteroarylring ist, der mit -(CH₂)ₘAryl oder -(CH₂)ₘHeteroaryl substituiert ist, worin das Aryl oder Heteroaryl gegebenenfalls mit einem oder mehreren Substituenten substituiert ist, die unabhängig ausgewählt sind aus Oxo, C₁₋₆-Alkyl, Halogen, -CN, Trifluormethyl, -OR³, -(CH₂)ₙCO₂R³, -NR³R⁴, -(CH₂)ₙCONR³R⁴, -NHCOR³, -SO₂NR³R⁴, -NHSO₂R³ und -S(O)ₚR³, und
A gegebenenfalls ferner mit einem Substituenten substituiert ist, der ausgewählt ist aus -OR⁵, Halogen, Trifluormethyl, -CN, -CO₂R⁵ und C₁₋₆-Alkyl, gegebenenfalls substituiert mit Hydroxy;
R¹ ausgewählt ist aus Methyl und Chlor;
R² ausgewählt ist aus -NH-CO-R⁶ und -CO-NH-(CH₂)_{q}-R⁷;
R³ ausgewählt ist aus Wasserstoff, -(CH₂)ᵣ-C₃₋₇-Cycloalkyl, -(CH₂)ᵣHeterocyclyl, -(CH₂)ᵣAryl und C₁₋₆-Alkyl, gegebenenfalls substituiert mit bis zu zwei Substituenten, die unabhängig ausgewählt sind aus -OR⁸ und -NR⁸R⁹
R⁴ ausgewählt ist aus Wasserstoff und C₁₋₆-Alkyl, oder
R³ und R⁴ zusammen mit dem Stickstoffatom, an das sie gebunden sind, einen 5- oder 6-gliedrigen Heterocyclylring bilden, der gegebenenfalls ein zusätzliches Heteroatom enthält, das ausgewählt ist aus Sauerstoff, Schwefel und N-R¹⁰;
R⁵ ausgewählt ist aus Wasserstoff und C₁₋₆-Alkyl;
R⁶ ausgewählt ist aus Wasserstoff, C₁₋₆-Alkyl, -(CH₂)_{q}-C₃₋₇-Cycloalkyl, Trifluormethyl, -(CH₂)ₛHeteroaryl, gegebenenfalls mit R¹¹ und/oder R¹² substituiert, und -(CH₂)ₛPhenyl, gegebenenfalls mit R¹¹ und/oder R¹² substituiert;
R⁷ ausgewählt ist aus Wasserstoff, C₁₋₆-Alkyl, C₃₋₇-Cycloalkyl, -CONHR¹³, Phenyl, gegebenenfalls mit R¹¹ und/oder R¹² substituiert, und Heteroaryl, gegebenenfalls mit R¹¹ und/oder R¹² substituiert;
R⁸ und R⁹ jeweils unabhängig ausgewählt sind aus Wasserstoff und C₁₋₆-Alkyl;
R¹⁰ ausgewählt ist aus Wasserstoff und Methyl;
R¹¹ ausgewählt ist aus C₁₋₆-Alkyl, C₁₋₆-Alkoxy, -(CH₂)_{q}-C₃₋₇-Cycloalkyl, -CONR¹³R¹⁴, -NHCOR¹⁴, Halogen, -CN, -(CH₂)ₜNR¹⁵R¹⁶, Trifluormethyl, Phenyl, gegebenenfalls mit einer oder mehreren R¹²-Gruppen substituiert, und Heteroaryl, gegebenenfalls mit einer oder mehreren R¹²-Gruppen substituiert;
R¹² ausgewählt ist aus C₁₋₆-Alkyl, C₁₋₆-alkoxy, Halogen, Trifluormethyl und -(CH₂)ₜNR¹⁵R¹⁶;
R¹³ und R¹⁴ jeweils unabhängig ausgewählt sind aus Wasserstoff und C₁₋₆-Alkyl, oder
R¹³ und R¹⁴ zusammen mit dem Stickstoffatom, an das sie gebunden sind, einen 5- oder 6-gliedrigen Heterocyclylring bilden, der gegebenenfalls ein zusätzliches Heteroatom enthält, das ausgewählt ist aus Sauerstoff, Schwefel und N-R¹⁰, worin der Ring mit bis zu zwei C₁₋₆-Alkylgruppen substituiert sein kann;
R¹⁵ ausgewählt ist aus Wasserstoff, C₁₋₆-Alkyl und -(CH₂)_{q}-C₃₋₇-Cycloalkyl, gegebenenfalls mit C₁₋₆-Alkyl substituiert,
R¹⁶ ausgewählt ist aus Wasserstoff und C₁₋₆-Alkyl, oder
R¹⁵ und R¹⁶ zusammen mit dem Stickstoffatom, an das sie gebunden sind, einen 5- oder 6-gliedrigen Heterocyclylring bilden, der gegebenenfalls ein zusätzliches Heteroatom enthält, das ausgewählt ist aus Sauerstoff, Schwefel und N-R¹⁰;
X und Y jeweils unabhängig ausgewählt sind aus Wasserstoff, Methyl und Halogen;
m, n, p und q jeweils unabhängig ausgewählt sind aus 0, 1 und 2;
r und s jeweils unabhängig ausgewählt sind aus 0 und 1; und
t ausgewählt ist aus 0, 1, 2 und 3;
mit der Maßgabe, daß, wenn A mit -(CH₂)ₘHeteroaryl substituiert ist und m 0 ist, dann ist die -(CH₂)ₘHeteroarylgruppe nicht ein 5-gliedriger Heteroarylring, der gegebenenfalls mit C₁₋₂-Alkyl substituiert ist;
oder ein pharmazeutisch annehmbares Derivat davon.

2. Verbindung gemäß Anspruch 1, worin A ein kondensierter 5-gliedriger Heteroarylring ist, der bis zu zwei Heteroatome enthält, die unabhängig ausgewählt sind aus Sauerstoff und Stickstoff.

3. Verbindung gemäß Anspruch 1 oder Anspruch 2, worin R¹ Methyl ist.

4. Verbindung gemäß einem der vorangegangenen Ansprüche, worin R² -CO-NH-(CH₂)_{q}-R⁷ ist.

5. Verbindung gemäß einem der vorangegangenen Ansprüche, worin A mit -(CH₂)ₘHeteroaryl substituiert ist, worin das Heteroaryl ein 5- oder 6-gliedriger Heteroarylring ist, der bis zu zwei Heteroatome enthält, die unabhängig ausgewählt sind aus Sauerstoff und Stickstoff.

6. Verbindung gemäß Anspruch 5, worin das Heteroaryl gegebenenfalls mit einem oder zwei Substituenten substituiert ist, die unabhängig ausgewählt sind aus Oxo, C₁₋₆-Alkyl, Halogen, -OR³, -NR³R⁴ und -(CH₂)ₙCONR³R⁴_{.}

7. Verbindung gemäß Anspruch 6, worin das Heteroaryl mit einem oder zwei Substituenten substituiert ist, die unabhängig ausgewählt sind aus Oxo und C₁₋₆-Alkyl.

8. Verbindung gemäß einem der Ansprüche 1 bis 4, worin A mit -(CH₂)ₘAryl substituiert ist, worin das Aryl Phenyl ist.

9. Verbindung gemäß Anspruch 8, worin das Aryl mit einem oder zwei Substituenten substituiert ist, die unabhängig ausgewählt sind aus C₁₋₆-Alkyl, Halogen, -CN, Trifluormethyl, -OR³, -NR³R⁴, -(CH₂)ₙCONR³R⁴ und -S(O)ₚR³.

10. Verbindung gemäß einem der vorangegangenen Ansprüche,
worin X Wasserstoff oder Fluor ist.

11. Verbindung ausgewählt aus:
N-Cyclopropyl-3-fluor-4-methyl-5-(1-phenyl-1H-indazol-5-yl)benzamid,
N-Cyclopropyl-3-fluor-5-[1-(4-fluorphenyl)-1H-indazol-5-yl]-4-methylbenzamid,
N-Cyclopropyl-3-fluor-5-[1-(4-fluor-2-methylphenyl)-1H-indazol-5-yl]-4-methylbenzamid,
N-Cyclopropyl-3-fluor-4-methyl-5-{1-[4-(4-morphonlinyl)phenyl]-1H-indazol-5-yl}benzamid,
N-Ethyl-3-fluor-4-methyl-5-(1-phenyl-1H-indazol-5-yl)benzamid,
N-(Cyclopropylmethyl)-3-fluor-4-methyl-5-(1-phenyl-1H-indazol-5-yl)benzamid,
N-Cyclopropyl-3-fluor-4-methyl-5-{1-(4-(methylsulfonyl)phenyl]-1H-indazol-5-yl}benzamid,
N-Cyclopropyl-3-fluor-4-methyl-5-(1-{4-[2-(methylamino)-2-oxoethyl]phenyl}-1H-indazol-5-yl)benzamid,
N-Cyclopropyl-3-[1-(4-{[2-(dimethylamino)ethyl]amino}phenyl)-1H-indazol-5-yl]-5-fluor-4-methylbenzamid,
N-Cyclopropyl-3-fluor-4-methyl-5-{1-[4-(tetrahydro-2H-pyran-4-ylamino)phenyl]-1H-indazol-5-yl}benzamid,
N-Cyclopropyl-3-fluor-4-methyl-5-(1-{4-[(tetrahydro-2-furanylmethyl)amino]phenyl}-1H-indazol-5-yl)benzamid,
N-Cyclopropyl-3-(1-{4-[(2,3-dihydroxypropyl)amino]phenyl}-1H-indazol-5-yl)-5-fluor-4-methylbenzamid,
N-Cyclopropyl-3-fluor-4-methyl-5-{3-(4-(methyloxy)phenyl]-1,2-benzisoxazol-6-yl}benzamid,
N-Cyclopropyl-3-fluor-5-[3-(4-hydroxyphenyl)-1,2-benzisoxazol-6-yl]-4-methylbenzamid,
N-Cyclopropyl-3-fluor-4-methyl-5-{1-[(1-oxido-2-pyridinyl)methyl]-1H-indazol-5-yl}benzamid,
N-Ethyl-3-[3-(4-fluorphenyl)-1H-indazol-6-yl]-4-methylbenzamid,
N-Cyclopropyl-3-[3-(4-fluorphenyl)-1H-indazol-6-yl]-4-methylbenzamid,
N-Ethyl-4-methyl-3-{3-[4-(methyloxy)phenyl]-1H-indazol-6-yl}benzamid,
N-Cyclopropyl-4-methyl-3-{3-[4-(methyloxy)phenyl]-1H-indazol-6-yl}benzamid,
N-(1-Ethyl-1H-pyrazol-5-yl)-3-fluor-5-[3-(4-fluorphenyl)-1H-indazol-6-yl]-4-methylbenzamid,
3-Fluor-5-[3-(4-fluorphenyl)-1H-indazol-6-yl]-4-methyl-N-(1-methyl-1H-pyrazol-5-yl)benzamid,
N-Ethyl-3-fluor-5-{3-[4-fluor-2-(methyloxy)phenyl]-1H-indazol-6-yl}-4-methylbenzamid,
N-(1,4-Dimethyl-1H-pyrazol-5-yl)-3-fluor-5-[3-(4-fluorphenyl)-1H-indazol-6-yl]-4-methylbenzamid und
N-(1,4-Dimethyl-1H-pyrazol-5-yl)-3-[3-(4-fluorphenyl)-1H-indazol-6-yl]-4-methylbenzamid
oder ein pharmazeutisch annehmbares Derivat davon.

12. Pharmazeutische Zusammensetzung, die wenigstens eine Verbindung gemäß einem der Ansprüche 1 bis 11 oder ein pharmazeutisch annehmbares Derivat davon zusammen mit einem oder mehreren pharmazeutisch annehmbaren Exzipienten, Verdünnungsstoffen und/oder Trägern umfaßt.

13. Verbindung gemäß einem der Ansprüche 1 bis 11 oder ein pharmazeutisch annehmbares Derivat davon zur Verwendung in der Therapie.

14. Verbindung gemäß einem der Ansprüche 1 bis 11 oder ein pharmazeutisch annehmbares Derivat davon zur Verwendung in der Behandlung oder Prophylaxe eines Zustands oder eines Krankheitszustands, der durch p38-Kinaseaktivität oder durch Cytokine vermittelt wird, die durch die Aktivität der p38-Kinase produziert werden.

15. Verwendung einer Verbindung gemäß einem der Ansprüche 1 bis 11 oder eines pharmazeutisch annehmbaren Derivats davon in der Herstellung eines Medikaments zur Verwendung in der Behandlung eines Zustands oder Krankheitszustands, der durch p38-Kinaseaktivität oder durch Cytokine vermittelt wird, die durch die Aktivität der p38-Kinase produziert werden.

16. Verfahren zur Herstellung einer Verbindung der Formel (I) gemäß einem der Ansprüche 1 bis 11 oder eines pharmazeutisch annehmbaren Derivats davon, das umfaßt:
(a) Umsetzen einer Verbindung der Formel (II): 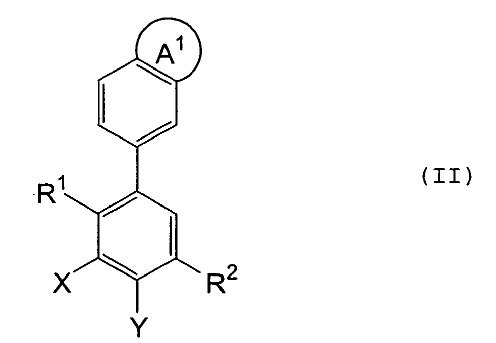 worin R¹, R², X und Y wie in Anspruch 1 definiert sind und A¹ ein unsubstituierter kondensierter 5-gliedriger Heteroarylring ist, mit einem Halogenidderivat der Formel (IIIA) oder (IIIB):
Z-(CH₂)ₘAryl (IIIA)
Z-(CH₂)ₘHeteroaryl (IIIB)
worin -(CH₂)ₘAryl und -(CH₂)ₘHeteroaryl wie in Anspruch 1 definiert sind und Z Halogen ist, in Gegenwart einer Base,
oder, wenn A mit -(CH₂)ₘAryl substituiert ist, worin m 0 ist, Umsetzen der Verbindung der Formel (II) mit einer Boronsäureverbindung der Formel (IV):
(HO)₂B-(CH₂)ₘAryl (IV)
worin -(CH₂)ₘAryl wie in Anspruch 1 definiert ist,
(b) Umsetzen einer Verbindung der Formel (V): 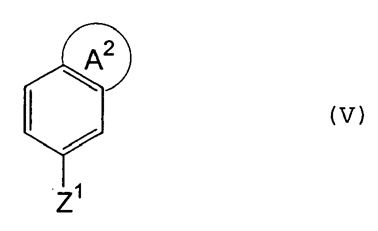 worin A² A wie in Anspruch 1 definiert ist und Z¹ Halogen ist,
mit einer Verbindung der Formel (VIA) oder (VIB): 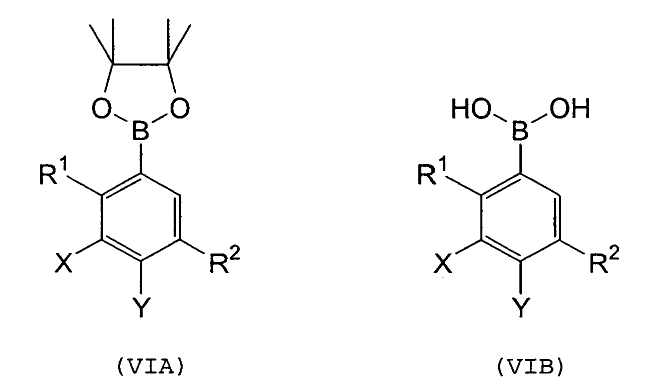 worin R¹, R², X und Y wie in Anspruch 1 definiert sind, in Gegenart eines Katalysators;
(c) Umsetzen einer Verbindung der Formel (XVI): 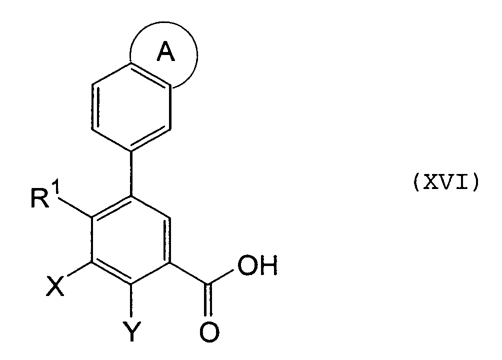 worin A, R¹, X und Y wie in Anspruch 1 definiert sind, mit einer Aminverbindung der Formel (XV):
R⁷-(CH₂)_{q}-NH2 (XV)
worin R⁷ und q wie in Anspruch 1 definiert sind, unter Amid-bildenden Bedingungen;
(d) wenn A ein kondensiertes Pyrazolyl ist, Umsetzen einer Verbindung der Formel (XVII): 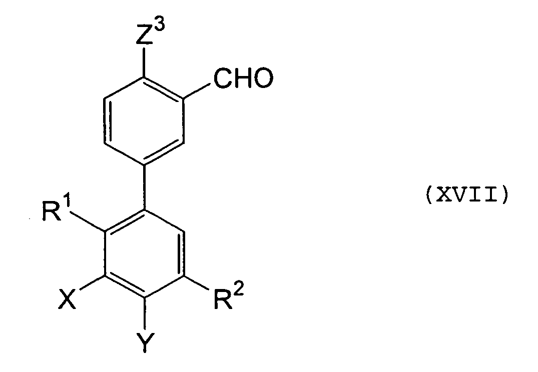 worin R¹, R², X und Y wie in Anspruch 1 definiert sind und Z³ Halogen ist,
mit einem Hydrazinderivat der Formel (VIIIA) oder (VIIIB) :
H₂NNH-(CH₂)ₘAryl (VIIIA)
H₂NNH-(CH₂)ₘHeteroaryl (VIIIB)
worin -(CH₂)ₘAryl und -(CH₂)ₘHeteroaryl wie in Anspruch 1 definiert sind;
(e) Umsetzen einer Verbindung der Formel (XVIII): 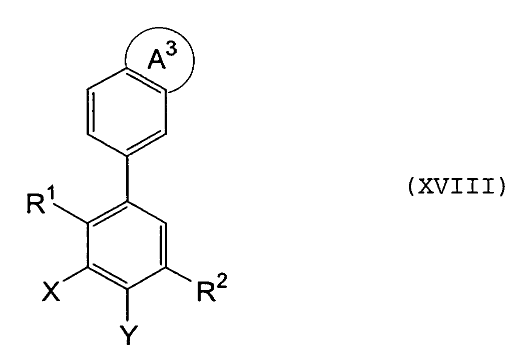 worin R¹, R², X und Y wie in Anspruch 1 definiert sind und A³ ein fusionierter 5-gliedriger Heteroarylring ist, der mit Halogen substituiert ist, mit einem geeigneten Boronsäurederivat; oder
(f) Endstufenmodifikation einer Verbindung der Formel (I) gemäß Anspruch 1, um eine andere Verbindung der Formel (I) gemäß Anspruch 1 zu ergeben.

## Revendications

1. Composé de formule (I) : 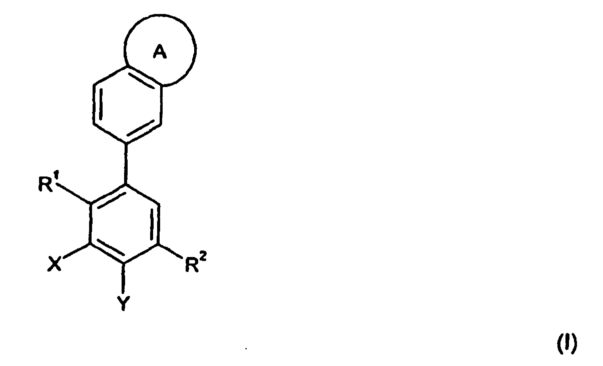 dans laquelle
A est un cycle hétéroaryle condensé de 5 éléments substitué par un groupe -(CH₂)ₘaryle ou -(CH₂)ₘhétéroaryle, dans lequel le groupe aryle ou hétéroaryle est éventuellement substitué par un ou plusieurs substituants indépendamment choisis parmi les groupes oxo, alkyle en C₁-C₆, halogéno, -CN, trifluorométhyle, -OR³, - (CH₂)ₙCO₂R³, -NR³R⁴, -(CH₂)ₙCONR³R⁴, -NHCOR³, -SO₂NR³R⁴, -NHSO₂R³ et -S(O)ₚR³, et
A est éventuellement substitué par un substituant choisi parmi les groupes -OR⁵, halogéno, trifluorométhyle, -CN, -CO₂R⁵ et alkyle en C₁-C₆ éventuellement substitué par un groupe hydroxy ;
R¹ est choisi parmi les groupes méthyle et chloro ;
R² est choisi parmi les groupes -NH-CO-R⁶ et -CO-NH-(CH₂)_{q}-R⁷ ;
R³ est choisi parmi un atome d'hydrogène, les groupes - (CH₂)ᵣ-cycloalkyle en C₃-C₇, -(CH₂)ᵣhétérocyclyle, -(CH₂)ᵣaryle, et alkyle en C₁-C₆ éventuellement substitué par jusqu'à deux substituants indépendamment choisis parmi -OR⁸ et -NR⁸R⁹,
R⁴ est choisi parmi un atome d'hydrogène et un groupe alkyle en C₁-C₆, ou
R³ et R⁴ ensemble avec l'atome d'azote auquel ils sont attachés, forment un cycle hétérocyclique de 5 ou 6 éléments contenant éventuellement un autre hétéroatome choisi parmi un atome d'oxygène, un atome de soufre et N-R¹⁰ ;
R⁵ est choisi parmi un atome d'hydrogène et un groupe alkyle en C₁-C₆ ;
R⁶ est choisi parmi un atome d'hydrogène, les groupes alkyle en C₁-C₆, -(CH₂)_{q}-cycloalkyle en C₃-C₇, trifluorométhyle, -(CH₂)ₛhétéroaryle éventuellement substitué par R¹¹ et/ou R¹², et -(CH₂)ₛphényle éventuellement substitué par R¹¹ et/ou R¹² ;
R⁷ est choisi parmi un atome d'hydrogène, les groupes alkyle en C₁-C₆, cycloalkyle en C₃-C₇, -CONHR¹³, phényle éventuellement substitué par R¹¹ et/ou R¹², et hétéroaryle éventuellement substitué par R¹¹ et/ou R¹² ;
R⁸ et R⁹ sont chacun indépendamment choisis parmi un atome d'hydrogène et un groupe alkyle en C₁-C₆ ;
R¹⁰ est choisi parmi un atome d'hydrogène et un groupe méthyle ;
R¹¹ est choisi parmi les groupes alkyle en C₁-C₆, alcoxy en C₁-C₆, -(CH₂)_{q}-cycloalkyle en C₃-C₇, -CONR¹³R¹⁴, -NHCOR¹⁴, halogéno, -CN, -(CH₂)ₜNR¹⁵R¹⁶, trifluorométhyle, phényle éventuellement substitué par un ou plusieurs groupes R¹² et hétéroaryle éventuellement substitué par un ou plusieurs groupes R¹² ;
R¹² est choisi parmi les groupes alkyle en C₁-C₆, alcoxy en C₁-C₆, halogéno, trifluorométhyle, et -(CH₂)ₜNR¹⁵R¹⁶;
R¹³ et R¹⁴ sont chacun indépendamment choisis parmi un atome d'hydrogène et un groupe alkyle en C₁-C₆, ou
R¹³ et R¹⁴ ensemble avec l'atome d'azote auquel ils sont attachés, forment un cycle hétérocyclique de 5 ou 6 éléments contenant éventuellement un autre hétéroatome choisi parmi un atome d'oxygène, un atome de soufre et N-R¹⁰, où le cycle peut être substitué par jusqu'à deux groupes alkyles en C₁-C₆ ;
R¹⁵ est choisi parmi un atome d'hydrogène, les groupes alkyle en C₁-C₆ et -(CH₂)_{q}-cycloalkyle en C₃-C₇ éventuellement substitué par groupe alkyle en C₁-C₆,
R¹⁶ est choisi parmi un atome d'hydrogène et un groupe alkyle en C₁-C₆, ou
R¹⁵ et R¹⁶ ensemble avec l'atome d'azote auquel ils sont attachés, forment un cycle hétérocyclique de 5 ou 6 éléments contenant éventuellement un autre hétéroatome choisi parmi un atome d'oxygène, un atome de soufre et N-R¹⁰ ;
X et Y sont chacun indépendamment choisis parmi un atome d'hydrogène, un groupe méthyle et un atome d'halogène ;
m, n, p et q sont chacun indépendamment choisis parmi 0, 1 et 2 ;
r et s sont chacun indépendamment choisis parmi 0 et 1 ; et
t est choisi parmi 0, 1, 2 et 3 ;
à condition que lorsque A est substitué par un groupe -(CH₂)ₘ-hétéroaryle et m vaut 0, le groupe -(CH₂)ₘ-hétéroaryle n'est pas un groupe hétéroaryle de 5 élément éventuellement substitué par un groupe alkyle en C₁-C₂ ;
ou un dérivé pharmaceutiquement acceptable de celui-ci.

2. Composé selon la revendication 1, dans lequel A est un cycle hétéroaryle condensé de 5 éléments contenant jusqu'à deux hétéroatomes indépendamment choisis parmi un atome d'oxygène et un atome d'azote.

3. Composé selon la revendication 1 ou 2, dans lequel R¹ est un groupe méthyle.

4. Composé selon l'une quelconque des revendications précédentes, dans lequel R² est -CO-NH-(CH₂)_{q}-R⁷.

5. Composé selon l'une quelconque des revendications précédentes, dans lequel A est substitué par un groupe (CH₂)ₘ- hétéroaryle dans lequel le groupe hétéroaryle est un cycle hétéroaryle de 5 ou 6 éléments contenant jusqu'à deux hétéroatomes indépendamment choisis parmi un atome d'oxygène et un atome d'azote.

6. Composé selon la revendication 5, dans lequel le groupe hétéroaryle est éventuellement substitué par un ou deux substituants indépendamment choisis parmi les groupes oxo, alkyle en C₁-C₆, halogène, -OR³, -NR³R⁴, et -(CH₂)ₙCONR³R⁴.

7. Composé selon la revendication 6, dans lequel le groupe hétéroaryle est éventuellement substitué par un ou deux substituants indépendamment choisis parmi les groupes oxo et alkyle en C₁-C₆.

8. Composé selon l'une quelconque des revendications 1 à 4, dans lequel A est substitué par un groupe -(CH₂)ₘ-aryle dans lequel le groupe aryle est un groupe phényle.

9. Composé selon la revendication 8, dans lequel le groupe aryle est éventuellement substitué par un ou deux substituants indépendamment choisis parmi les groupes alkyle en C₁-C₆, halogéno, -CN, trifluorométhyle, -OR³, -NR³R⁴, -(CH₂₎ₙCONR³R⁴, et -S (O)ₚR³.

10. Composé selon l'une quelconque des revendications précédentes, dans lequel X est un atome d'hydrogène ou un atome de fluor.

11. Composé choisi parmi :
le N-cyclopropyl-3-fluoro-4-méthyl-5-(1-phényl-1H-indazol-5-yl)benzamide ;
le N-cyclopropyl-3-fluoro-5-[1-(4-fluorophényl)-1H-indazol-5-yl]-4-méthylbenzamide ;
le N-cyclopropyl-3-fluoro-5-[1-(4-fluoro-2-méthylphényl)-1H-indazol-5-yl]-4-méthylbenzamide ;
le N-cyclopropyl-3-fluoro-4-méthyl-5-{1-[4-(4-morpholinyl)phényl]-1H-indazol-5-yl}benzamide ;
le N-éthyl-3-fluoro-4-méthyl-5-(1-phényl-1H-indazol-5-yl)benzamide ;
le N-(cyclopropylméthyl)-3-fluoro-4-méthyl-5-(1-phényl-1H-indazol-5-yl)benzamide ;
le N-cyclopropyl-3-fluroo-4-méthyl-5-{1-[4-(méthyl-sulfonyl)phényl]-1H-indazol-5-yl}benzamide ;
le N-cyclopropyl-3-fluoro-4-méthyl-5-(1-{4-[2-(méthyl-amino)-2-oxoéthyl]phényl}-1H-indazol-5-yl)benzamide ;
le N-cyclopropyl-3-[1-(4-{[2-(diméthylamino)éthyl]amino}-phényl)-1H-indazol-5-yl]-5-fluoro-4-méthylbenzamide ;
le N-cyclopropyl-3-fluoro-4-méthyl-5-{1-[4-(tétrahydro-2H-pyran-4-ylamino)phényl]-1H-indazol-5-yl}benzamide ;
le N-cyclopropyl-3-fluoro-4-méthyl-5-(1-{4-[(tétrahydro-2-furanylméthyl)amino]phényl}-1H-indazol-5-yl)benzamide ;
le N-cyclopropyl-3-(1-{4-[(2,3-dihydroxypropyl)amino]-phényl}-1H-indazol-5-yl)-5-fluoro-4-méthylbenzamide ;
le N-cyclopropyl-3-fluoro-4-méthyl-5-{3-[4-(méthyloxy)-phényl]-1,2-benzisoxazol-6-yl}benzamide ;
le N-cyclopropyl-3-fluoro-5-[3-(4-hydroxyphényl)-1,2-benzisoxazol-6-yl]-4-méthylbenzamide ;
le N-cyclopropyl-3-fluoro-4-méthyl-5-{1-[(1-oxydo-2-pyridinyl)méthyl]-1H-indazol-5-yl}benzamide ;
le N-éthyl-3-[3-(4-fluorophényl)-1H-indazol-6-yl]-4-méthylbenzamide ;
le N-cyclopropyl-3-[3-(4-fluorophényl)-1H-indazol-6-yl]-4-méthylbenzamide ;
le N-éthyl-4-méthyl-3-{3-[4-(méthyloxy)phényl]-1H-indazol-6-yl}benzamide ;
le N-cyclopropyl-4-méthyl-3-{3-[4-(méthyloxy)phényl]-1H-indazol-6-yl}benzamide ;
le N-(1-éthyl-1H-pyrazol-5-yl)-3-fluoro-5-[3-(4-fluoro-phényl)-1H-indazol-6-yl]-4-méthylbenzamide ;
le 3-fluoro-5-[3-(4-fluorophényl)-1H-indazol-6-yl]-4-méthyl-N-(1-méthyl-1H-pyrazol-5-yl)benzamide ;
le N-éthyl-3-fluoro-5-{3-[4-fluoro-2-(méthyloxy)phényl]-1H-indazol-6-yl}-4-méthylbenzamide ;
le N-(1,4-diméthyl-1H-pyrazol-5-yl)-3-fluoro-5-[3-(4-fluorophényl)-1H-indazol-6-yl]-4-méthylbenzamide ; et
le N-(1,4-diméthyl-1H-pyrazol-5-yl)-3-[3-(4-fluoro-phényl)-1H-indazol-6-yl]-4-méthylbenzamide ;
ou un dérivé pharmaceutiquement acceptable de ceux-ci.

12. Composition pharmaceutique comprenant au moins un composé selon l'une quelconque des revendications 1 à 11, ou un dérivé pharmaceutiquement acceptable de celui-ci, en association avec un ou plusieurs excipients, diluants et/ou supports pharmaceutiquement acceptables.

13. Composé selon l'une quelconque des revendications 1 à 11, ou un dérivé pharmaceutiquement acceptable de celui-ci, pour une utilisation en thérapie.

14. Composé selon l'une quelconque des revendications 1 à 11, ou un dérivé pharmaceutiquement acceptable de celui-ci, pour une utilisation dans le traitement ou la prophylaxie d'une pathologie ou d'une maladie médiée par l'activité de la kinase p38 ou médiée par des cytokines produites par l'activité de la kinase p38.

15. Utilisation d'un composé selon l'une quelconque des revendications 1 à 11, ou d'un dérivé pharmaceutiquement acceptable de celui-ci, pour la fabrication d'un médicament destiné à une utilisation dans le traitement ou la prophylaxie d'une pathologie ou d'une maladie médiée par l'activité de la kinase p38 ou médiée par des cytokines produites par l'activité de la kinase p38.

16. Procédé de préparation d'un composé de formule (I) selon l'une quelconque des revendications 1 à 11, ou d'un dérivé pharmaceutiquement acceptable de celui-ci, comprenant
(a) faire réagir un composé de formule (II) 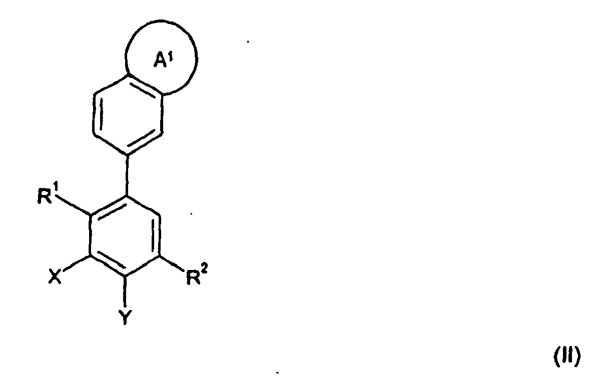 où R¹, R², X et Y sont tels que définis dans la revendication 1 et A¹ est un cycle hétéroaryle condensé de 5 éléments non substitué, avec un dérivé halogéné de formule (IIIA) ou (IIIB)
Z-(CH₂)ₘ-aryle (IIIA)
Z-(CH₂)ₘ-hétéroaryle (IIIB)
où les groupes -(CH₂)ₘ-aryle et -(CH₂)ₘ-hétéroaryle sont tels que définis dans la revendication 1 et Z est un atome d'halogène,
en présence d'une base,
ou, lorsque A est substitué par un groupe -(CH₂)ₘ-aryle dans lequel m vaut 0, faire réagir le composé de formule (II) avec un acide boronique de formule (IV)
(HO)₂B-(CH₂)ₘ-aryle (IV)
où le groupe -(CH₂)ₘ-aryle est tel que défini dans la revendication 1,
(b) faire réagir un composé de formule (V) 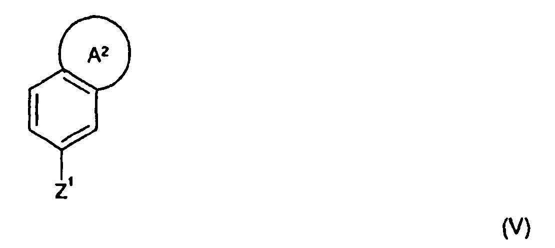 où A² est A comme défini dans la revendication 1 et Z¹ est un atome d'halogène,
avec un composé de formule (VIA) ou (VIB) 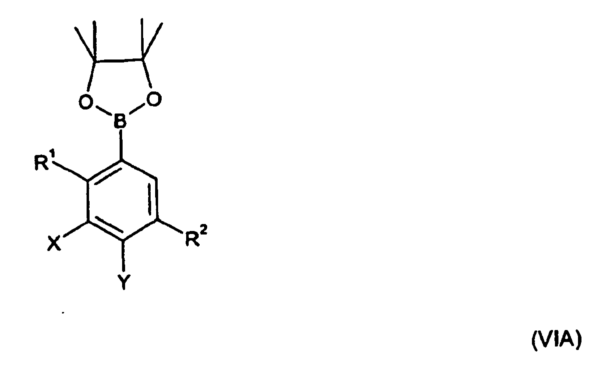 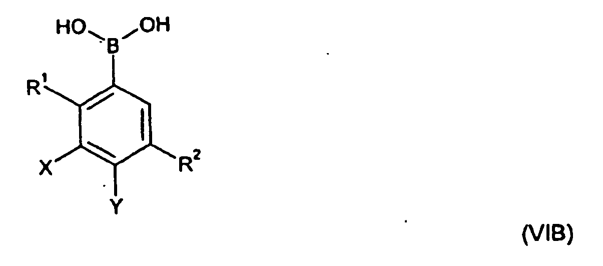 où R¹, R², X et Y sont tels que définis dans la revendication 1,
en présence d'un catalyseur ;
(c) faire réagir un composé de formule (XVI) 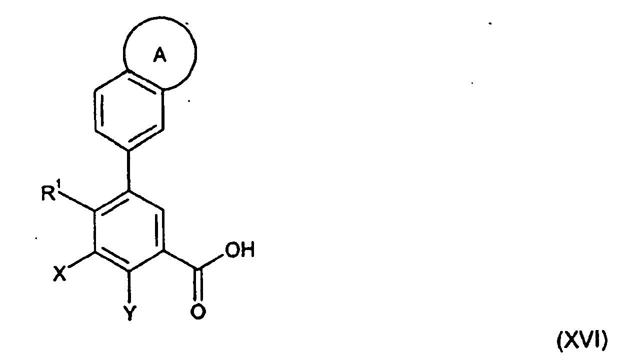 où A, R¹, X et Y sont tels que définis dans la revendication 1,
avec une amine de formule (XV)
R⁷-(CH₂)_{q}-NH₂ (XV)
où R⁷ et q sont tels que définis dans la revendication 1, dans des conditions de formation d'amide ;
(d) lorsque A est un groupe pyrazolyle condensé, faire réagir un composé de formule (XVII) 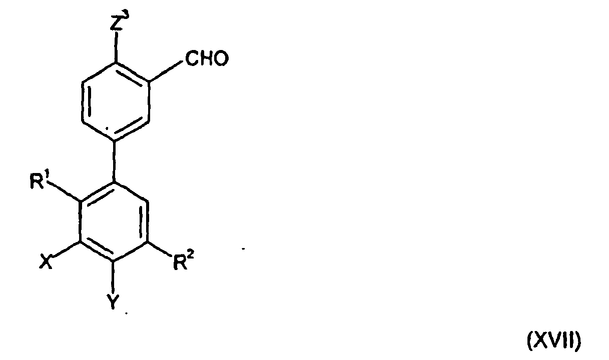 où R¹, R², X et Y sont tels que définis dans la revendication 1 et Z³ est un atome d'halogène,
avec une hydrazine de formule (VIIIA) ou (VIIIB)
H₂NNH-(CH₂)ₘ-aryle (VIIIA)
H₂NNH-(CH₂)ₘ-hétéroaryle (VIIIB)
où les groupes -(CH₂)ₘ-aryle et -(CH₂)ₘ-hétéroaryle sont tels que définis dans la revendication 1 ;
(e) faire réagir un composé de formule (XVIII) 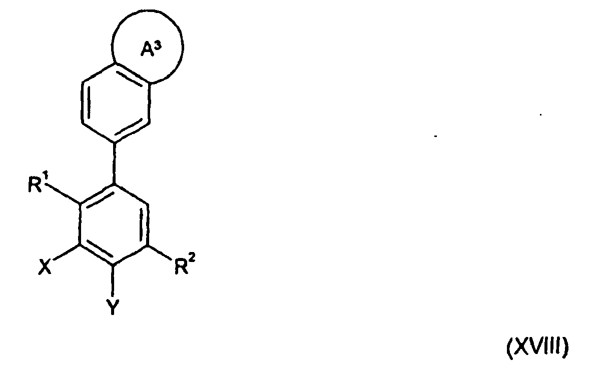 où R¹, R², X et Y sont tels que définis dans la revendication 1 et A³ est un cycle hétéroaryle condensé de 5 éléments substitué par un atome d'halogène,
avec un dérivé d'acide boronique approprié ; ou
f) modifier l'étape finale d'un composé de formule (I) tel que défini dans la revendication 1 pour donner un autre composé de formule (I) tel que défini dans la revendication 1.
